# EUROPÄISCHE PATENTSCHRIFT

(11) **EP 1 227 848 B1**
(45) Veröffentlichungstag und Bekanntmachung des Hinweises auf die Patenterteilung: **24.05.2006**
(21) Anmeldenummer: 00984960.5
(22) Anmeldetag: 03.11.2000
(51) Int. Cl.: A61K 47/48, C07K 19/00, C12N 15/62, C07K 1/107, C07K 14/005, C07K 14/755

(54) **VERFAHREN ZUR VERBINDUNG VON MOLEKULAREN SUBSTANZEN**
METHOD FOR LINKING MOLECULAR SUBSTANCES
METHODE POUR LIER DES SUBSTANCES MOLECULAIRES

(30) Priorität: 03.11.1999 DE 19952956
(43) Veröffentlichungstag der Anmeldung: 07.08.2002
(73) Patentinhaber: ACGT Progenomics AG, 06120 Halle (Saale) (DE)
(72) Erfinder: BÖHM, Gerald, 06114 Halle (Saale) (DE); SCHMIDT, Ulrich, West Leederville, WA 6007 (AU); PARTHIER, Christoph, 06108 Halle (Saale) (DE); GÜNTHER, Constanze, 06114 Halle (DE)
(74) Vertreter: Reinhard - Skuhra - Weise & Partner
(86) Internationale Anmeldenummer: PCT/EP2000/010873
(87) Internationale Veröffentlichungsnummer: WO 2001/032684

(56) Entgegenhaltungen:
- DATABASE CHEMABS [Online] CHEMICAL ABSTRACTS SERVICE, COLUMBUS, OHIO, US; SCHMIDT, ULI ET AL: "Stringent purification of recombinant proteins using WW/polyproline affinity chromatography" retrieved from STN Database accession no. 135:16206 CA XP002171196 & INT. J. BIO-CHROMATOGR. (2001), 6(1), 79-85 , 2001,
- DATABASE CHEMABS [Online] CHEMICAL ABSTRACTS SERVICE, COLUMBUS, OHIO, US; HOFFMULLER, ULRICH ET AL: "Mapping and characterization of epitopes recognized by WW domains using cellulose-bound peptide libraries" retrieved from STN Database accession no. 131:239439 CA XP002171197 & PEPT. PROC. AM. PEPT. SYMP., 15TH (1999), MEETING DATE 1997, 551-552. EDITOR(S): TAM, JAMES P.;KAUMAYA, PRAVIN T. P. PUBLISHER: KLUWER, DORDRECHT, NETH. , 1999,
- DATABASE CHEMABS [Online] CHEMICAL ABSTRACTS SERVICE, COLUMBUS, OHIO, US; LINN, HILLARY ET AL: "Using molecular repertoires to identify high-affinity peptide ligands of the WW domain of human and mouse YAP" retrieved from STN Database accession no. 127:259156 CA XP002171198 & BIOL. CHEM. (1997), 378(6), 531-537 , 1997,
- EINBOND, AARON ET AL: "Towards prediction of cognate complexes between the WW domain and proline-rich ligands" FEBS LETT. ( 1996 ), 384(1), 1-8 , 1996, XP002171194
- NGUYEN, JACK T. ET AL: "Exploiting the basis of proline recognition by SH3 and WW domains: design of N-substituted inhibitors" SCIENCE (WASHINGTON, D. C.) (1998), 282(5396), 2088-2092 , 1998, XP002171195

## Beschreibung

Die vorliegende Erfindung betrifft ein Verfahren zur Verbindung von zwei oder mehr molekularen Substanzen über Adaptersegmente, die eine gerichtete Wechselwirkung bewirken, basierend auf der Affinität von prolinreichen Aminosäure-Sequenzen und Proteindomänen vom Typ WW.

### Gebiet der Erfindung und Stand der Technik

Die Wechselwirkung von zwei oder mehr molekularen Substanzen ist ein häufiges Problem im Rahmen der biotechnologischen und pharmazeutisch-medizinischen Forschung, Entwicklung und Anwendung. Insbesondere werden dabei meist die Wechselwirkungen von zwei oder mehr Proteinen oder Peptiden als molekulare Substanzen betrachtet. Derartige Wechselwirkungen werden oft im Rahmen biochemischer und zellbiologischer Forschung untersucht, beispielsweise zur intra- und interzellulären Kommunikation, der Signaltransduktion auf molekularer Ebene oder bei Analysen von Protein-Protein-Wechselwirkungen (unter anderem bei Verwendung der *two-hybrid*-Systeme und davon abgeleiteten Verfahren). Zum anderen ist die Assoziation von Biomolekülen, insbesondere von zwei oder mehr Proteinen, zur in vitro-Synthese eines Fusionsproteins für viele biotechnologische Prozesse von hoher Bedeutung. Solchermaßen generierte Fusionsproteine können beispielsweise heterobifunktionelle (bivalente) Antikörper sein (sogenannte *diabodys;* vgl. O. Perisic, P.A. Webb, P. Holliger, G. Winter & R.L. Williams, Crystal structure of a diabody, a bivalent antibody fragment, *Structure* 2, S. 1217-1226, 1994), die aus den Bindungsdomänen (Fab / Fv / scFv - Fragmente) zweier verschiedener Antikörper bestehen. Sind dabei die beiden Valenzen beispielsweise jeweils gegen Tumorzellen bzw. natürliche Killerzellen gerichtet, dann kann das bivalente, hybride Fusionsprotein demzufolge eine Anheftung von Killerzellen an Tumorzellen vermitteln. Im Falle von Immunotoxinen werden Antikörper mit toxischen Substanzen gekoppelt und die Zellgifte durch die spezifische Antigen-Antikörper-Wechselwirkung in vordefinierte Zelltypen dirigiert (vgl. M.A. Ghetie & E.S. Vitetta, Recent developments in immunotoxin therapy, *Curr. Opin. Immunol.* 6, S. 707-714, 1994).

Mit Hilfe von Fusionskonstrukten und Assoziaten von verschiedenen Proteinen lassen sich grundsätzlich beliebige Effektoren miteinander kombinieren, und durch entsprechende Wechselwirkungen mit Antigenen oder anderen biologischen Effekten zwei Funktionen oder Eigenschaften in einem hybriden Molekül realisieren. Eine Reihe von Beispielen hierzu sind publiziert (J.P. McGrath, X. Cao, A. Schutz, P. Lynch, T. Ebendal, M.J. Coloma, S.L. Morrison & S.D. Putney, Bifunctional fusion between nerve growth factor and a transferrin receptor antibody, *J. Neurosci. Res.* 47, S. 123-133, 1997; J.M. Betton, J.P. Jacob, M. Hofnung, J.K. Broome-Smith, Creating a bifunctional protein by insertion of beta-lactamase into the maltodextrin-binding protein, *Nat. Biotechnol.* 15, S. 1276-1279, 1997; Y. Maeda, H. Ueda, T. Hara, J. Kazami, G. Kawano, E. Suzuki & T. Nagamune, Expression of a bifunctional chimeric protein A-*Vargula hilgendorfii* luciferase in mammalian cells, *Biotechniques* 20, S. 116-121, 1996; W. Wels, I.M. Harwerth, M. Zwickl, N. Hardman, B. Groner & N.E. Hynes, Construction, bacterial expression and characterization of a bifunctional single-chain antibody-phosphatase fusion protein targeted to the human erbB-2 receptor, *Biotechnology (N. Y.)* 10, S. 1128-1132, 1992).

Heterobifunktionelle Konstrukte werden häufig durch die Synthese eines Fusionsproteins auf Genebene erzeugt. Dies setzt in der Regel geeignete Verknüpfungselemente (Linker) zwischen den beiden Partnern, sowie zugängliche Termini der Polypeptidketten voraus. In ungünstigen Fällen kann die Fusion der Partner dazu führen, daß das Fusionsprodukt inaktiv ist, beispielsweise weil das Fusionsprotein eine korrekte dreidimensionale Faltungstopologie nicht ausbilden kann. Es ist daher oft wünschenswert, daß die Zusammenlagerung der beiden Fusionspartner *in vitro,* das heißt, nach der getrennten Synthese und Faltung der beiden Partner, erfolgt. Ein solches Verfahren würde beispielsweise auch die schnelle Herstellung und Analyse verschiedener Kombinationen von Einzel-Bausteinen erlauben, ohne daß jeweils neue genetische Konstrukte erforderlich sind. Zur Fusion dieser Bausteine sind Adaptersegmente vonnöten, durch die der Prozeß der Fusion bzw. der gerichteten Assoziation der beteiligten Partner von deren Herstellung entkoppelt wird. Weiterhin ist es dabei notwendig, daß die Adaptersegmente (Domänen bzw. Peptidsequenzen) an die beteiligten Partner fest angekoppelt sind, ohne deren spezifischen Eigenschaften ansonsten zu verändern.

In anderen Anwendungsfällen ist es erwünscht, daß eine kurzzeitige, aber starke Wechselwirkung zwischen zwei Molekülspezies erfolgt. Dabei spielen Peptide und kleine Proteindomänen eine besonders wichtige Rolle, da sie im Prozeß der rekombinanten Herstellung von Proteinen vergleichsweise einfach an den gewünschten Zielproteinen angebracht werden können. Anwendungsfälle dafür sind beispielsweise die Aufreinigung von rekombinant hergestellten Proteinen über spezifische Bindungssegmente. Hier wird oft ein Poly-Histidin-Peptidabschnitt zur Bindung an Nickel-Chelat-Säulen (vgl. P. Hengen, Purification of His-Tag fusion proteins from *Escherichia coli, Trends Biochem. Sci.* 20, S. 285-286, 1995), oder die Bindung eines als Strep-Tag bekannten Peptidsegmentes an Streptavidin (T.G. Schmidt, J. Koepke, R. Frank & A. Skerra, Molecular interaction between the Strep-tag affinity peptide and its cognate target, streptavidin, *J. Mol. Biol.* 255, S. 753-766, 1996), eingesetzt. Das His-Tag-Verfahren hat jedoch den Nachteil, daß lediglich eine Anbindung des Poly-Histidin-Peptidabschnitts an Nickelionen-enthaltende Strukturen erfolgen kann; die Verbindung beispielsweise von zwei natürlichen Proteinen oder Peptiden ist auf diese Weise nicht möglich. Zur Verbindung von molekularen Substanzen ist das Verfahren daher nicht oder nur in Ausnahmefällen geeignet. Auch findet man in den auf diese Art aufgereinigten Präparationen oft Nickelionen in der Lösung, was das System für medizinisch-therapeutische Anwendung unattraktiv macht. Beim Strep-Tag-Verfahren ist der die Bindung vermittelnde Bereich des Bindungspartners relativ groß, so daß es aus sterischen Gründen für viele Verbindungen nicht geeignet ist. Darüber hinaus besitzen Avidin und Streptavidin jeweils vier Bindungsstellen, so daß eine geregelte Ausbildung von zwei verschiedenen miteinander verbundenen molekularen Substanzen in Lösung sehr schwierig ist.

Neben der Anwendung bei der Reinigung von solchermaßen markierten Proteinen ist auch die Immobilisierung der Proteine an einer festen, inerten Matrix von biotechnologisch hoher Bedeutung, beispielsweise bei der Rückfaltung von Proteinen an einer Matrix zur Verhinderung von Aggregationsprozessen während der Faltung (vgl. G. Stempfer, B. Höll-Neugebauer & R. Rudolph, Improved refolding of an immobilized fusion protein, *Nat. Biotechnol.* 14, S. 329-334, 1996), oder die Immobilisierung eines Enzyms in einem Bioreaktor. Polyionische Sequenzen, die in dem genannten Verfahren bisher eingesetzt werden, besitzen jedoch den Nachteil, daß ihre Wechselwirkung bei Anwesenheit von Polyionen wie beispielsweise DNA in der Lösung, oder auch durch verschiedene Lösungsmitteladditive empfindlich gestört wird.

Aufgabe der vorliegenden Erfindung ist es, ein Verfahren zur Verbindung von molekularen Substanzen bereitzustellen, das die erwähnten Nachteile des Stands der Technik nicht aufweist.

Dies wird erfindungsgemäß durch ein Verfahren nach Anspruch 1 zur Verbindung von zwei oder mehr molekularen Substanzen miteinander über Adaptersegmente erreicht,
dadurch gekennzeichnet, daß
eine der molekularen Substanzen so modifiziert wird, daß sie als Adaptersegment in zumindest einem Bereich eine WW-Domäne oder eine davon abgeleitete Struktur aufweist,
eine andere molekulare Substanz so modifiziert wird, daß sie als Adaptersegment in zumindest einem Bereich eine prolinreiche Sequenz aufweist, die an die WW-Domäne oder eine davon abgeleitete Struktur bindet,
und die molekularen Substanzen durch die Zusammenlagerung von WW-Domäne oder davon abgeleiteten Strukturen und prolinreicher Sequenz miteinander in Wechselwirkung treten, um eine Bindung aneinander zu erreichen.

Vorteilhafte Ausführungsformen ergeben sich aus den Unteransprüchen und der Beschreibung.

### Beschreibung

Die Verknüpfung von zwei oder mehreren verschiedenen molekularen Substanzen (Molekülspezies) zu einem - üblicherweise heterobifunktionellen - Fusionskonstrukt ist ein Prozeß von hohem biotechnologischen und pharmazeutischen Interesse. Üblicherweise werden im Rahmen der erfindungsgemäßen Anwendung Proteine und/oder Peptide als zu verbindende Molekülspezies verwendet, da die Adaptersegmente der vorliegenden Erfindung aus dieser chemischen Klasse stammen. Erfindungsgemäß sind aber auch andere molekulare Substanzen einsetzbar, die mit einem der Adaptersegmente dieser Erfindung versehen sind. Beispielsweise kann erfindungsgemäß eine feste Matrix mit einer molekularen Substanz über die genannten Adaptersegmente verbunden werden. Oftmals müssen die beiden zu verbindenden Substanzen stabil und kovalent miteinander verbunden werden. Andererseits kann es bei einigen Anwendungen auch erwünscht sein, daß die Wechselwirkung zwischen den beiden Molekülspezies nur für eine begrenzte Zeit vorhanden ist und beispielsweise durch äußere Zusätze schnell wieder aufgelöst werden kann. In wiederum anderen Anwendungen muß eine Molekülspezies für eine begrenzte Zeit immobilisiert werden, also mit einer Matrix spezifisch wechselwirken, beispielsweise zur Aufreinigung eines Proteins aus einem Zell-Rohextrakt bei der rekombinanten Herstellung eines Proteins, oder bei einer matrixgestützten Rückfaltung des Proteins. Für derartige Anwendungsfälle ist die vorliegende Erfindung geeignet.

Erfindungsgemäß kann beispielsweise ein Protein zur Verpackung in das Innere einer virusähnlichen Hülle dirigiert werden, oder es können zwei oder mehr verschiedene Proteine zu einem chimären Protein mit neuen Eigenschaften, beispielsweise als bivalente Antikörper, verbunden werden. In analoger Weise kann diese Wechselwirkung zur Immobilisierung einer molekularen Spezies verwendet werden, beispielsweise zur Abtrennung dieser Substanz aus einem Substanzgemisch.

Zusätzlich zur Verbindung über die Adaptersegmente, die auf der Wechselwirkung zwischen WW-Domäne und prolinreicher Sequenz beruht, kann eine kovalente Bindung der molekularen Substanzen miteinander erfolgen. Diese kovalente Bindung kann dabei über eine Disulfidverbrückung durch künstlich eingeführte Cysteine an geeigneter räumlicher Stelle in beiden molekularen Substanzen zu einer dauerhaften Verbindung zwischen den beiden molekularen Substanzen führen. Durch die Disulfidverbrückung können bifunktionelle Fusionsmoleküle generiert werden, die unter physiologischen und allen üblichen Lösungsmittelbedingungen stabil vorliegen und somit auch für medizinische, therapeutische, diagnostische, und biotechnologische Prozesse nutzbar sind.

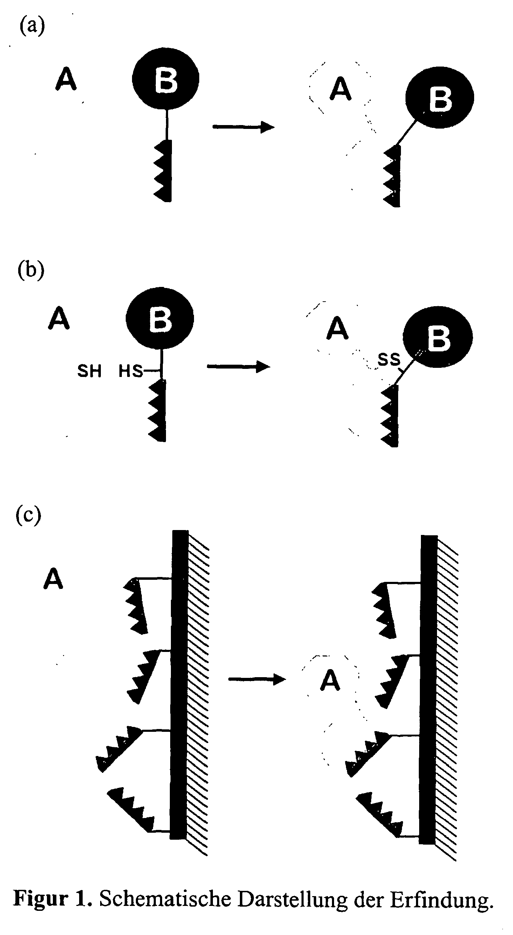

Oben beschriebene mögliche Ausführungsformen der Erfindung sind auch in Figur 1 beispielhaft dargestellt.

Erfindungsgemäß wird zur Verbindung von zwei oder mehr molekularen Substanzen miteinander die hochspezifische Wechselwirkung von Proteinabschnitten, die unter dem Begriff WW-Domänen bekannt sind, mit einer prolinreichen Peptidsequenz (mit einem Prolin-Anteil von mehr als 50 % innerhalb einer kurzen Peptidabfolge von 2 bis 6 Aminosäuren) ausgenutzt. Diese beiden Molekülspezies zeigen eine außerordentlich starke Wechselwirkung zueinander (K_{D} 20 bis 100 nM), wenn sie miteinander inkubiert werden. Die langsame Dissoziation der Partner führt dazu, daß die Interaktion zunächst nur temporär wirksam ist. Ist dies unerwünscht, so kann die Dissoziation durch die Fixierung der Bindungspartner mittels einer Disulfidbrücke verhindert werden. Dazu werden künstlich Cysteine in geeigneter räumlicher Position in die beiden Adaptersegmente oder im Bereich der Adaptersegmente eingeführt. Nach der Assoziation der Partner können die Cysteinpaare durch Wahl geeigneter Redoxbedingungen oxidiert werden und auf diese Weise dauerhaft kovalent miteinander verknüpft werden. Das entstehende hybride Fusionsprotein kann wesentliche Eigenschaften der jeweils zugrundeliegenden Molekülspezies aufweisen.

Die WW-Domäne ist eine kleine, globuläre Proteindomäne, die üblicherweise aus 30 bis 40 Aminosäuren besteht (vgl. M. Sudol, The WW Domain Binds Polyprolines and is Involved in Human Diseases, *Exp. & Mol. Medicine* 28, S. 65-69, 1996), es sind jedoch auch kürzere Varianten bekannt. WW-Domänen weisen eine hohe natürliche Affinität zu prolinreichen Liganden auf, die mit Dissoziationskonstanten von 20 bis 100 nM gebunden werden. Die prolinreichen Liganden besitzen eine für die Bindung notwendige Mindestlänge von 5 bis 15 Aminosäuren bei einem Prolingehalt von mehr als 50 % innerhalb dieses Segments, wobei die direkte Wechselwirkung üblicherweise innerhalb eines lokalen Segments von 2 bis 6 Aminosäuren (mit mehr als 50 % Prolingehalt) auftritt. Natürliche Liganden stellen dabei fast ausschließlich Proteine dar, die in ihrer Aminosäuresequenz prolinreiche Abschnitte enthalten, jedoch sind auch prolinreiche Peptide spezifische Liganden von WW-Domänen.

Die Bezeichnung WW-Domäne leitet sich aus der Beobachtung ab, daß zwei konservierte Tryptophan-Reste (abgekürzt WW) im Abstand von 20 bis 22 Aminosäuren auftreten; das zweite Tryptophan und eine Reihe von zumeist ebenfalls konservierten hydrophoben Aminosäuren bilden dabei die Bindungstasche für den prolinreichen Liganden. Nach dem zweiten Tryptophan ist oft im Abstand von 2 Aminosäuren ein konserviertes Prolin lokalisiert. Es sind eine Reihe von verschiedenen WW-Domänen-Typen bekannt, die in derzeit 4 Klassen eingeordnet werden, und die sich insbesondere bezüglich der präferentiell gebundenen prolinreichen Peptidliganden voneinander unterscheiden. WW-Domänen können prinzipiell mit den (strukturell unverwandten) SH3-Domänen um die Bindung von prolinreichen Liganden konkurrieren, jedoch weisen die Liganden der SH3-Domänen abweichende Konsensussequenzen auf, so daß sich prolinreiche Peptidliganden ableiten lassen, die spezifisch von WW-Domänen gebunden werden. Außerdem ist die Bindung der WW-Domänen an prolinreiche Liganden in der Regel stärker als die von SH3-Domänen. Die nachstehende Tabelle gibt einen Überblick über Vertreter und Ligandenbindungseigenschaften von WW-Domänen-Proteinen.

| WW-Domänen-Typ | Spezifisches Bindungsmotiv des prolinreichen Liganden* | Beispiel / Vertreter |
|---|---|---|
| Typ I | Pro-Pro-(beliebig)-(Tyr) | YAP65, Pin1, Dystrophin |
| Typ II | Pro-Pro-Leu-Pro | FBP11, FE65 |
| Typ III | Pro-Gly-Met | FBP21, PRP40 |
| Typ IV | Phospho-Ser / phospho-Thr | Pin 1, Nedd4 |

| | | |
|---|---|---|
| in unmittelbarer Umgebung einer prolinreichen Sequenz (> 50 % Prolingehalt) | | |

Die Umwandlung einer WW-Domäne vom Typ I in eine des Typs II, verbunden mit der damit einhergehenden Änderung der Spezifität bezüglich des prolinreichen Peptids, kann beispielsweise durch die Aminosäure-Austausche L14W und H16G in der WW-Typ I-Domänensequenz erreicht werden. Die Struktur eines Vertreters aus der Klasse I (Yes-Associated Protein, YAP) zeigt, daß diese WW-Domäne aus drei β-Strängen besteht, die ein β-Faltblatt ausbilden (vgl. M. Macias, M. Hyvonen, E. Baraldi, J. Schultz, M. Sudol, M. Saraste & H. Oschkinat, The Structure of the WW Domain in Complex with a Proline-Rich Peptide, *Nature* 382, S. 646-649, 1996). Die Ligandenbindungstasche wird aus dem zweiten β-Strang des Faltblattes unter Mitwirkung des zweiten konservierten Tryptophans gebildet.

Die wichtigste biologische Rolle von WW-Domänen besteht offensichtlich in der intrazellulären Signalübermittlung. WW-Domänen werden darüber hinaus direkt oder indirekt mit einer Reihe von Erkrankungen in Verbindung gebracht, beispielsweise dem angeborenen Liddle-Syndrom, der Muskeldystrophie, und der Alzheimer-Erkrankung; sie sind daher auch Ziel einer Reihe von therapeutischen Strategien. Schließlich spielen WW-Domänen eine biologische Rolle bei der Embryonalentwicklung der Nieren und im intrazellulären Lebenszyklus von Retroviren.

Im Rahmen dieser Erfindung wurde gefunden, daß überraschenderweise WW-Domänen eine stabile Struktur (Faltungstopologie) unter üblichen Lösungsmittelbedingungen ausbilden können, auch wenn sie aus ihrem ursprünglichen molekularen Kontext heraus isoliert und in beziehungsweise an andere Proteine, wie beispielsweise einem viralen Hüllprotein, gentechnisch fusioniert werden. Dies trifft beispielsweise auf eine WW-Domäne aus der Klasse der forminbindenden Proteine mit einer außergewöhnlich geringen Größe von nur 31 Aminosäuren zu, die unter diesen Bedingungen eine stabile Struktur (Faltungstopologie) ausbildet. Ungewöhnlicherweise stört die Einbringung der WW-Domäne unter günstigen Bedingungen, zusammen mit Linkersegmenten, bestehend aus den Aminosäuren Serin und Glycin, in externe Loops von Proteinen offenbar weder deren Faltung, noch wird die Bindungseigenschaft der WW-Domäne dadurch negativ beeinflußt. Es konnte gezeigt werden, daß dies auch für Varianten der WW-Domäne gilt, bei denen beispielsweise Aminosäuren an spezifischen Positionen gegen Cystein ausgetauscht wurden. Es kann auch für weitere von WW-Domänen abgeleitete Strukturen gelten, wie beispielsweise mehrere konsekutiv aneinandergereihte WW-Domänen, deren Beiträge zur Bindung sich jeweils addieren oder in günstigen Fällen synergistisch sind, verkürzte oder verlängerte WW-Domänen, oder auch WW-Domänen mit ortsgericheten Austauschen von einzelnen Aminosäuren, die - je nach gewünschter Anwendung - beispielsweise stärker oder schwächer als die natürliche Proteindomänen an prolinreiche Sequenzen zu binden vermögen. Solche veränderten Domänen können beispielsweise durch ein Bindungs-Screening mit Hilfe der Phagen-Display-Technologie nach dem Stand der Technik gewonnen werden.

Proteine, die eine inserierte (das heißt, in geeignete Loopbereiche innerhalb der Polypeptidkette des Wirtsproteins eingebrachte) oder anfusionierte (jeweils am N-Terminus und/oder am C-Terminus des Wirtsproteins befindliche) WW-Domäne besitzen, zeigen eine hohe Affinität zu prolinreichen Sequenzen. Diese prolinreichen Sequenzen können dabei wiederum an andere Proteine, Peptide, oder andere molekulare Substanzen fusioniert sein. Damit können zwei beliebige Molekülspezies durch die daran angehängten Interaktionspartner (WW-Domäne und prolinreiche Sequenz) aneinander angelagert werden. Diese Anlagerung beruht zunächst auf einer hydrophoben Interaktion, vermittelt durch WW-Domäne und prolinreichem Liganden. Diese Interaktion kann jedoch in Hinblick auf höhere Spezifität und flexibleren Einsatz durch Hinzuziehen von beispielsweise ionischen Wechselwirkungen oder Einführung von kovalenten Bindungen zwischen WW-Domäne und prolinreichem Ligand angepaßt werden. So kann durch Plazierung von zusätzlichen Aminosäuren, die unterschiedlich geladen sind, oder durch Punktmutationen in den oder in der Nähe der Adaptersegmente die Anlagerung des prolinreichen Liganden an die WW-Domäne verstärkt oder spezifischer gestaltet werden. Eine kovalente Disulfidverbrückung der beiden Komponenten wiederum erlaubt eine dauerhafte und feste Bindung der Adaptersegmente und der damit verbundenen molekularen Substanzen.

Auch eine Verbindung von mehr als zwei molekularen Substanzen miteinander ist erfindungsgemäß möglich.

Die kinetischen Parameter der Wechselwirkung wie Dissoziationskonstante (k_{D}) konnten im Rahmen der Untersuchungen durch Interaktionsmessungen bestimmt werden, die auf Oberflächen-Plasmon-Resonanzmessungen beruhen. Damit kann gezeigt werden, daß die Wechselwirkungen zwischen WW-Domänen und prolinreicher Peptidsequenz für die in der vorliegenden Erfindung beschriebenen Anwendungsfälle grundsätzlich geeignet sind.

Durch die Natur der Wechselwirkung der Adaptersegmente kommt es ausschließlich zur Bildung einer heteromeren Hybrid-Spezies, bestehend aus einem Anteil mit WW-Domäne und einem Anteil mit prolinreicher Sequenz. Die Ausbildung von homofunktionellen Molekülen (Homodimeren) kann ausgeschlossen werden. Gegenüber anderen Systemen mit vergleichbaren Eigenschaften hat die verwendete WW-Domäne den Vorteil, außergewöhnlich klein und kompakt zu sein. Dadurch ist sie bei vielen Anwendungen, beispielsweise den Antigen-Antikörper-Wechselwirkungen, anderen Ligandenbindungsdomänen (beispielsweise Lipocalinen und Anticalinen) deutlich überlegen.

Weiterhin konnte gezeigt werden, daß die Einführung von Cystein-Resten an spezifischen Stellen innerhalb der WW-Domäne und im prolinreichen Substrat dazu benutzt werden kann, über die Wechselwirkung zwischen WW-Domäne und prolinreicher Sequenz hinaus, die kovalente Kopplung der Assoziationspartner zu bewirken und demzufolge die an diese Adaptersegmente anfusionierten Proteinanteile miteinander in stabile Verbindung zu bringen. Eine Dissoziation der Interaktionspartner kann so auch unter ungünstigen Bedingungen wie beispielsweise besonders hohen oder sehr niedrigen Salzkonzentrationen, oder unter physiologisch extremen Temperaturen, nicht erfolgen. Dazu wird beispielsweise an der Stelle Asp8 (Nummerierung folgt der WW-Domäne aus dem forminbindenden Protein FBP11) oder alternativ auch an der Stelle Lys 19 ein Austausch gegen Cystein vorgenommen. Diese Positionen sind nur beispielhaft ausgewählt; die Einbringung von spezifischen Cysteinen kann auch an anderen Stellen der WW-Domäne oder in der Umgebung hiervon, oder in der prolinreichen Sequenz oder in der Umgebung hiervon sinnvoll und erfolgreich sein.

Der besondere Vorteil ist, daß wiederum nur heterobifunktionelle Spezies (Heterodimere) gebildet werden, weil die starke Wechselwirkung von prolinreichem Peptid und WW-Domäne bewirkt, daß sich zunächst nur Assoziate zwischen diesen beiden Adaptersegmenten ausbilden können. Die nachfolgende Disulfidverbrückung unter oxidierenden Bedingungen führt dann zur gerichteten Ausbildung von kovalent verbrückten, heteromeren Spezies. Aufgrund der hohen lokalen Konzentration (Annäherung) der Cysteine in der assoziierten Form kann die Disulfidverbrückung auch unter leicht reduzierenden Bedingungen erfolgreich sein und damit besonders spezifisch erfolgen. Im Gegensatz dazu würden im Falle einer zufälligen Disulfidverbrückung, das heißt, ohne die vorgeschaltete starke Affinität der Adaptersegmente zueinander (also bei nicht erfindungsgemäßer Anwendung), unter oxidierenden Bedingungen auch unerwünschte Homodimere der beiden Interaktionspartner als Nebenprodukte gebildet werden.

Das in der vorliegenden Erfindung beschriebene Verfahren eignet sich dazu, beliebige Interaktionspartner in Lösung *(in vitro)* aneinander zu heften, wobei sowohl eine temporäre als auch eine dauerhafte Bindung der beiden Partner möglich ist. Gleichfalls kann das Verfahren dazu genutzt werden, Proteine oder Peptide oder andere molekulare Substanzen, die mit einem der beiden Adaptertypen (WW-Domäne oder prolinreiche Sequenz) ausgestattet sind, aus einem Substanzgemisch spezifisch abzutrennen. Dies erfolgt durch reversible Bindung an eine Matrix, die den jeweils anderen Wechselwirkungspartner kovalent gebunden hat. Die starke Bindung bewirkt, daß die Moleküle auch unter stringenten Lösungsmittelbedingungen an der Matrix anhaften. Das Verfahren erlaubt dadurch beispielsweise die schnelle und effiziente Aufreinigung von rekombinanten Proteinen aus dem Zellrohextrakt von Bakterien oder eukaryontischen Zellen, unter der Voraussetzung, daß das rekombinante (zu reinigende) Molekül eines der beiden Adaptersegmente (WW-Domäne oder prolinreiche Sequenz) in Fusion oder als Insertion trägt, während das entsprechende Gegenstück zu dem Adaptersegment an der festen Phase immobilisiert wurde.

Gleichermaßen ist dieses Immobilisierungsverfahren dazu geeignet, spezifische Modifikationen oder eine Rückfaltung des immobilisierten Proteins an der Matrix unter Vermeidung von Aggregationsprozessen durchzuführen. Schließlich sind mit der vorliegenden Erfindung auch Anwendungen möglich, bei denen eine einfache und stabile Immobilisierung einer molekularen Substanz eine Schlüsselrolle spielen, beispielsweise bei Biosensoren oder in Bioreaktoren (vgl. R.S. Phadke, Biosensors and enzyme immobilized electrodes, *Biosystems* 27, S. 203-206, 1992; M. Abdul-Mazid, Biocatalysis and immobilized enzyme/cell bioreactors. Promising techniques in bioreactor technology, *Biotechnology (N. Y.)* 11, S. 690-695, 1993).

Neben Proteinen und Peptiden können für das in der vorliegenden Erfindung beschriebene Verfahren auch andere Substanzen verwendet werden. So können Peptidderivate, Peptidantibiotika, Proteine mit modifizierten Seitenketten wie Fluoreszenzmarkierung, Alkylierungen, Acetylierung, gemischte Disulfide mit thiolhaltigen Substanzen, und analoge Veränderungen in gleicher Weise eingesetzt werden. Aber auch Peptid- oder Proteinkonjugate mit Kohlenhydrat-, Nukleinsäure- oder Lipidanteilen können in das Verfahren eingesetzt werden. Ebenfalls lassen sich Nukleinsäuren wie DNA, RNA, Ribozyme, synthetische Nukleinsäuren wie beispielsweise Peptide Nucleic Acids, oder Hybride hiervon mit einem Adaptersegment koppeln, beispielsweise auf chemischem Wege. Sie sind dann ebenfalls dazu geeignet, eine Interaktion mit einem analogen Wechselwirkungspartner einzugehen. Einzige Voraussetzung ist die stabile Anbindung jeweils eines der beiden verwendeten Adaptersegmente.

Als Proteine kommen im Rahmen der erfindungsgemäßgen Anwendung insbesondere Antikörper, antikörperanaloge Substanzen, Enzyme, Strukturproteine, sowie Kapsomere von Viren oder Phagen in Betracht.

Die Einfügung oder Anbindung der prolinreichen Sequenz beziehungsweise der WW-Domäne oder einer davon abgeleiteten Struktur in eine molekulare Substanz kann prinzipiell an jeder Stelle der molekularen Substanz erfolgen, sofern dadurch nicht die Struktur der WW-Domäne wesentlich beeinflußt wird. Gegebenenfalls ist es günstig, die Anbindung oder Einfügung unter Verwendung von geeigneten Linkersegmenten, wie im Beispiel 1 für das Protein PyVPI-WW150 beschrieben, durchzuführen. Im Falle der Einfügung in Proteine ist es zweckmäßig, solche Bereiche aus der Struktur des Proteins herauszusuchen, in denen keine periodischen Sekundärstrukturelemente wie α-Helix oder β-Faltblatt vorliegen. Die Einfügung von WW-Domänen oder prolinreichen Sequenzen in Proteinstrukturen erfolgt am günstigsten dort, wo "Turn"-Bereiche oder "Random Coil"-Bereiche nach der üblichen Definition vorliegen.

Die Bindung der beiden Adaptersegmente aneinander kann unter dem Aspekt verschiedener physikalischer Wechselwirkungen betrachtet werden. So kann ein hydrophober Effekt bei der Stabilisierung der Wechselwirkung dominieren, wie dies in nachfolgendem Beispiel 7 demonstriert wird. Es können aber auch andere Interaktionsformen zur Ausbildung einer Bindung beitragen, wie beispielsweise ionische Wechselwirkungen, Ion-Dipol-Wechselwirkungen, Dipol-Dipol-Wechselwirkungen, Wasserstoffbrückenbindungen, van der Waals-Kräfte, oder Dispersionskräfte. Schließlich kann neben den genannten Beispielen für nichtkovalente Verbindungen auch eine kovalente Verbindung der beiden molekularen Substanzen bewirkt werden. Dabei wird eine chemisch stabile Atombindung zwischen zwei Atomen der Interaktionspartner ausgebildet, vorzugsweise in Form einer Disulfidbrücke von zwei beteiligten Cystein-Seitenketten.

Die Matrix kann zur Immobilisierung eines der Adaptersegmente (WW-Domäne oder prolinreiche Sequenz) beispielsweise über den N-Terminus der prolinreichen Sequenz oder der WW-Domäne (Kopplung über N-Hydroxysuccinimidester der Matrix) oder über eine Thiolgruppe eines in der prolinreichen Sequenz bzw. der WW-Domäne enthaltenen Cysteins (Kopplung über Iodacetamidgruppe der Matrix) beladen werden. Als Matrices kommen beispielsweise Agarose und Agarosederivate, Agarosebeads, Sepharose, Dextrane, Kohlenhydrate, oder ähnliches Polymermaterial nach dem Stand der Technik in Betracht.

Anwendungen dieser Erfindung sind in den nachfolgenden Beispielen aufgezeigt, durch die der Schutzumfang der Erfindung jedoch nicht begrenzt sein soll.

In der Beschreibung und in den Beispielen wird auf die folgenden Figuren Bezug genommen.

**Figur 1** zeigt eine schematische Darstellung der Erfindung. Es werden Adaptersegmente auf der Basis der Wechselwirkung von prolinreichen Substanzen mit WW-Domänen und davon abgeleiteten Formen verwendet. (a) Verknüpfung von zwei molekularen Spezies A und B über Adaptersegmente. (b) Verknüpfung von zwei molekularen Spezies A und B analog zu (a), jedoch mit zusätzlicher Disulfidverbrückung zur kovalenten Verknüpfung der Partner. (c) Matrix-Immobilisierung einer molekularen Substanz über die Adaptersegmente (eine der molekularen stellt die Matrix oder ein Teil der Matrix dar). Die Adaptersegmente können sowohl an den Enden (Termini), als auch in Form von Insertionen den Molekülen angefügt werden.

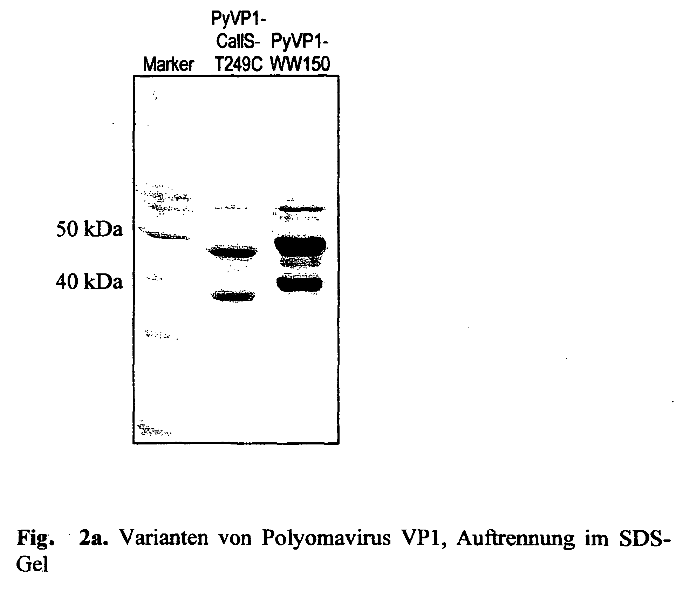

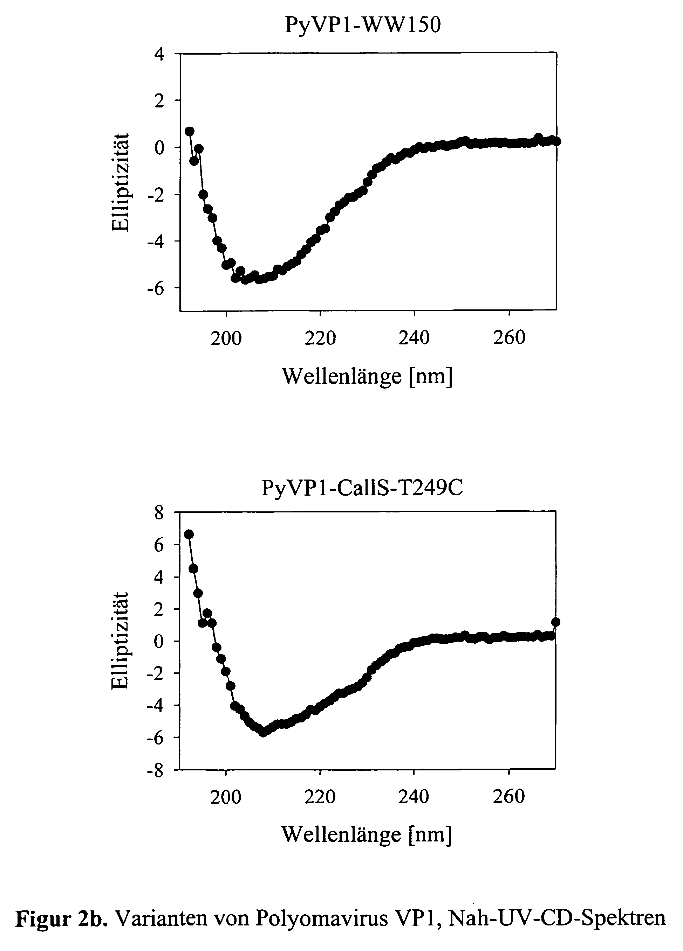

**Figur 2** zeigt in (a) einen Vergleich mittels SDS-PAGE der Proteinmassen und der Reinigungseffizienzen von verschiedenen Varianten des Polyomavirusproteins VP1, die PyVP1-CallS-T249C-Variante (vergleichbar dem Wildtyp des Proteins) und die PyVPI-WW150-Variante, bei der in der Nähe der Aminosäureposition 150 eine WW-Domäne in einen Loop inseriert ist. Herstellung und Reinigung der Varianten ist in Beispiel 1 ausführlich beschrieben. Bei beiden Varianten sind üblicherweise auftretende Abbauprodukte des Proteins zu verzeichnen, die eine kleinere Molekularmasse aufweisen. (b) Circulardichroismus-Spektren (CD) der PyVP1-WW150-Variante und der PyVP1-CallS-T249C-Variante. Die inserierte WW-Domäne an Position 150 zeigt eine native Faltung, wodurch sich der β-Faltblattanteil im CD-Spektrum erhöht.

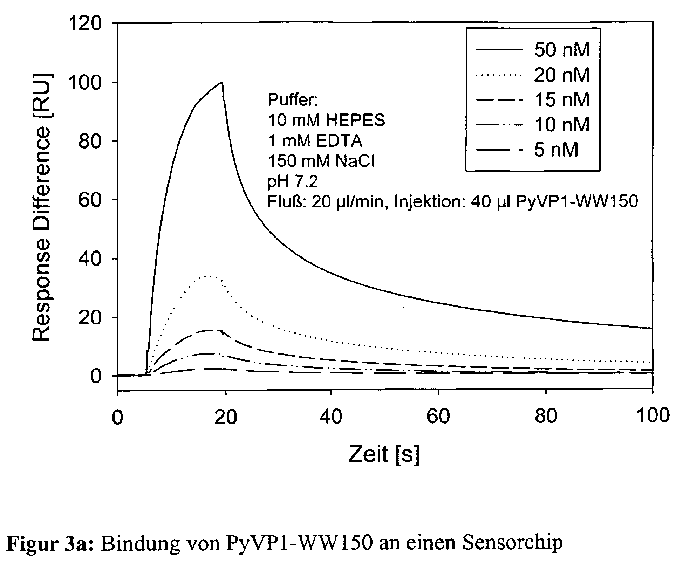

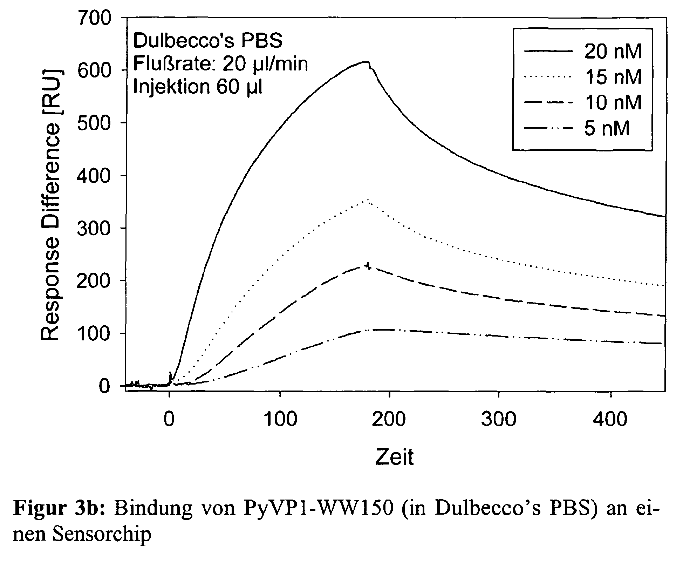

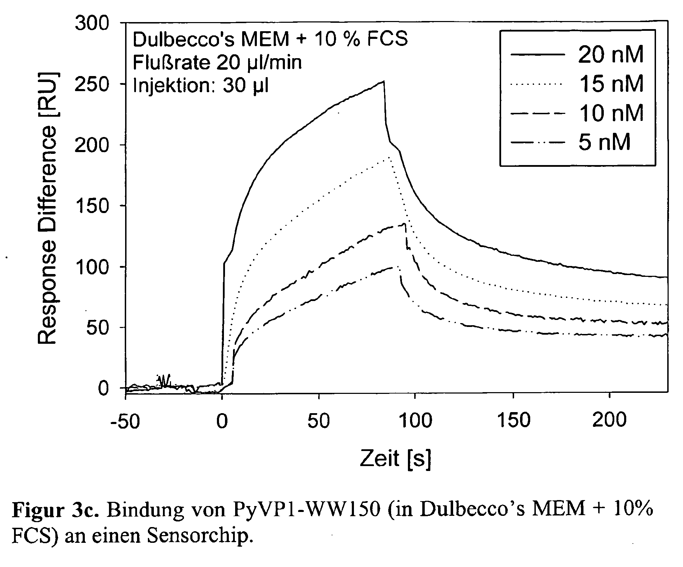

**Figur 3** zeigt die Bindung von PyVP1-WW150 an einen Sensorchip mit immobilisiertem prolinreichen Peptid, gemäß Beispiel 2. Die drei Messungen, basierend auf Oberflächenplasmonresonanz, zeigen, daß die Lösungsmittelzusätze nur geringfügigen Einfluß auf Affinität und Spezifität der Wechselwirkung ausüben, wobei die in (b) und (c) verwendeten Additive jeweils komplexe physiologische Substanzgemische darstellen. (a) Bindung von PyVP1-WW150 an die Sensoroberfläche unter üblichen Lösungsmittelbedingungen. (b) Bindung von PyVP1-WW150 an die Sensoroberfläche unter Verwendung von Dulbecco's PBS als Laufmittel. (c) Bindung von PyVPI-WW150 an die Sensoroberfläche bei Zugabe von Fötalem Kälberserum (FCS) als Modell für ein Gemisch biologisch relevanter Substanzen.

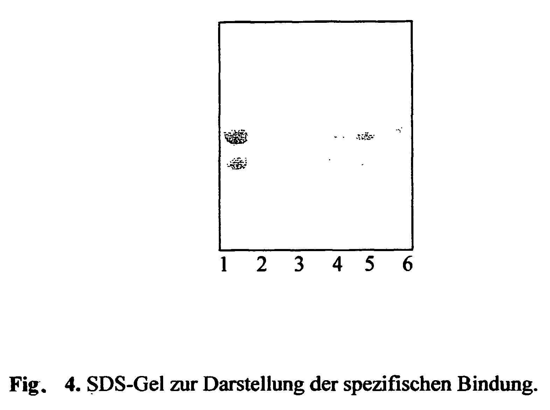

**Figur 4** zeigt ein SDS-Gel zur Darstellung der spezifischen Bindung von PyVPI-WW150 an eine prolinpeptidhaltige Matrix. Bahn 1: Auftrag VP1-WW150 (gereinigt nach Beispiel 1); Bahn 2 und Bahn 3: verschiedene Waschfraktionen; Bahn 4 und Bahn 5: Elutionsfraktionen mit 1 % SDS im Elutionspuffer; Bahn 6: 10 kDa-Molekularmassenstandard. Das Beispiel zeigt, daß WW-Domänen-haltige Proteine an einer Matrix reversibel immobilisiert werden können. Die beobachtete Doppelbande der PyVPI-Variante stellt das native Protein sowie eine proteolytische Abbaubande des Proteins dar, die üblicherweise in allen Präparationen auftritt.

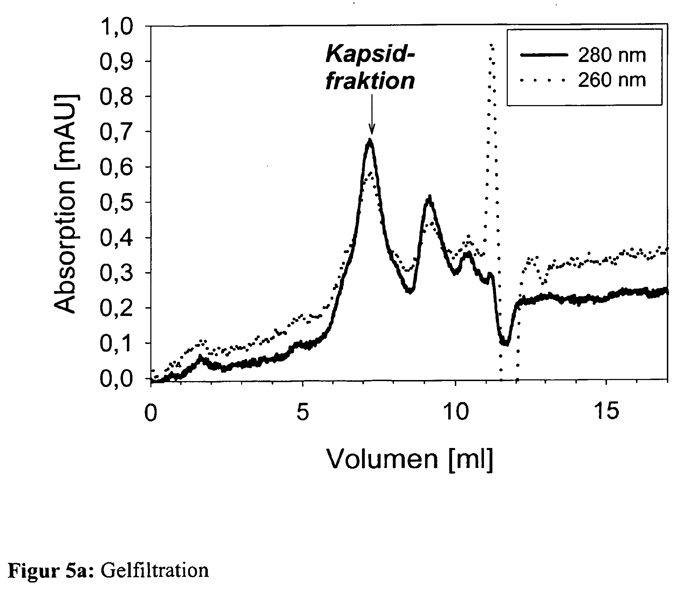

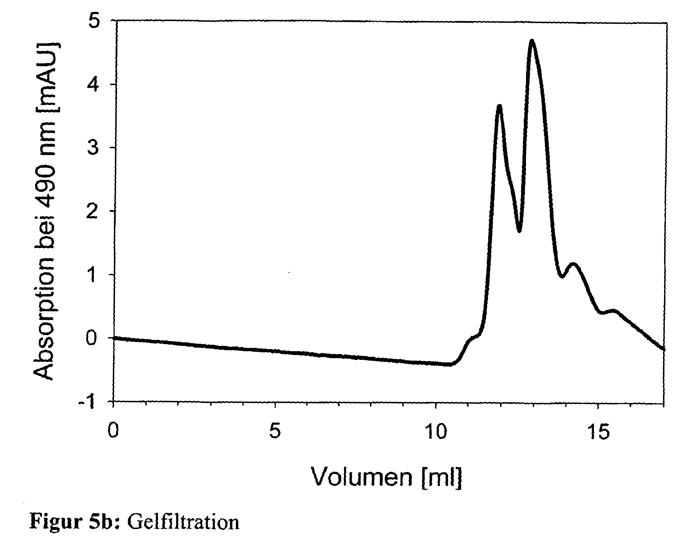

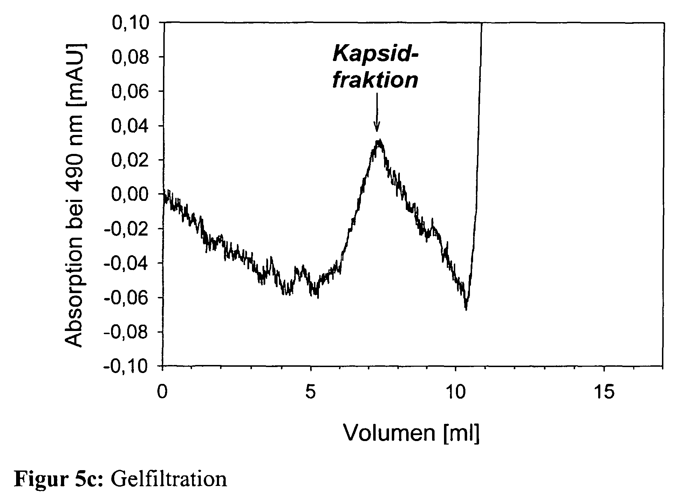

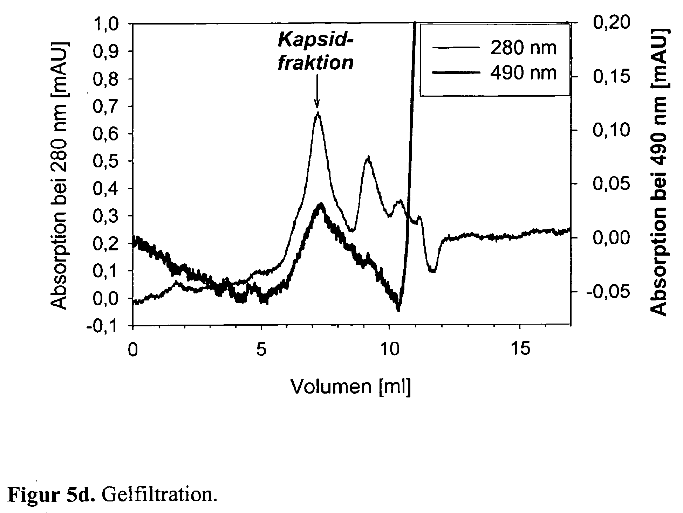

**Figur 5** zeigt eine Gelfiltration (TSKGel G5000PWXL, Fa. TosoHaas) zur Demonstration der Bindung eines prolinreichen Peptides an die Oberfläche eines virusähnlichen Kapsids, wobei sich auf dieser Oberfläche die in das Hüllprotein VP1 inserierte WW-Domänen befinden. Die Assemblierung des PyVP1-WW150-Proteins zum Kapsid erfolgt unter den in Beispiel 4 angegebenen Bedingungen. An die virusähnlichen Kapside kann ein prolinreiches Peptid gebunden werden, das durch die spezifische Absorption eines daran gekoppelten Farbstoffes nachgewiesen wird. (Fig. 5 a): Nachweis der Kapsidbildung durch Absorption bei 260 und 280 nm; die Kapside eluieren bei einem Volumen von 6 bis 8 ml, die nichtassemblierten, freien Pentamere erscheinen bei 9 bis 10 ml. (Fig. 5b): Absorption des fluoreszenzmarkierten Peptids bei 490 nm; die Elution erfolgt parallel zu der Kapsidelution und der Pentamerelution bei 6 bis 10 ml. Oberhalb von 10 ml eluiert überschüssiges, freies Peptid sowie der Fluoreszenzfarbstoff. (Fig. 5c): das freie, ungebundene Peptid zeigt keine Wechselwirkung mit der Matrix und eluiert ausschließlich oberhalb von 10 ml. (Fig. 5d): Überlagerung der Chromatogramme 5a und 5b, zur Darstellung der Koelution des gebundenen Peptides mit der Kapsidfraktion.

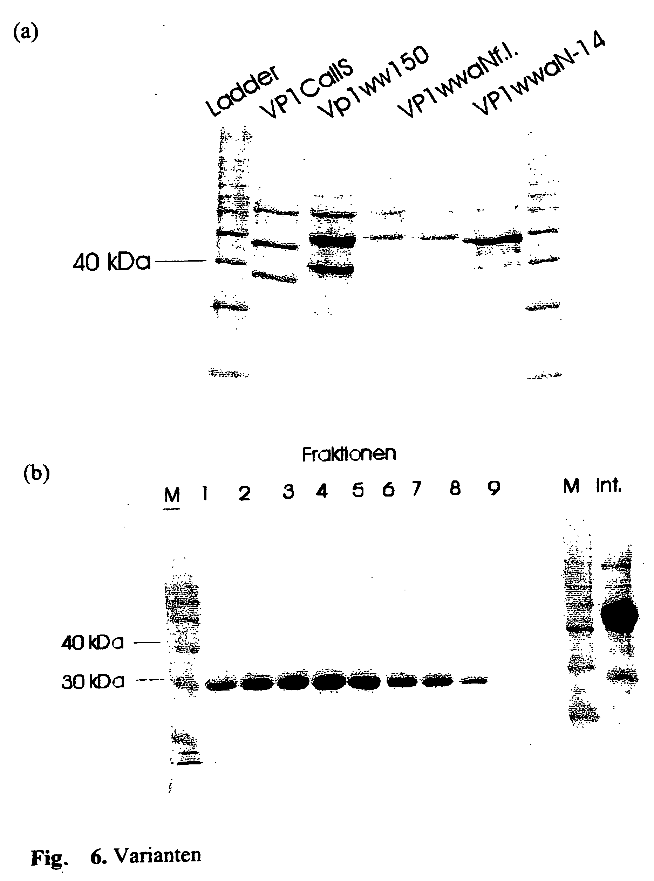

**Figur 6** zeigt in (a) die Reinigung der Varianten PyVP1-3C-WW1 und PyVP1-3C-WW[N-14]. Das SDS-Gel (12 %) zeigt das PyVPI-Protein ohne WW-Domäne (Bahn 2), die PyVP1-WW150-Variante aus Beispiel 1 (Bahn 3), sowie die beiden Varianten aus Beispiel 8 (PyVP1-3C-WW1 auf Bahn 4 und Bahn 5, PyVpl-3C-WW[N-14] auf Bahn 6). Bahn 1 und Bahn 7, Molekularmassenstandard (10 kDa-Leiter). (b) Reinigung der GFP-Variante GFP-PLP, und Darstellung auf einem SDS-Gel (15 %). Bahnen M, Molekularmassenstandard (10 kDa-Leiter); Bahn Int, Waschfraktion von der Intein-Affinitätssäule; Fraktionen 1 bis 9, verschiedene Elutionsfraktionen des GFP-PLP-Proteins.

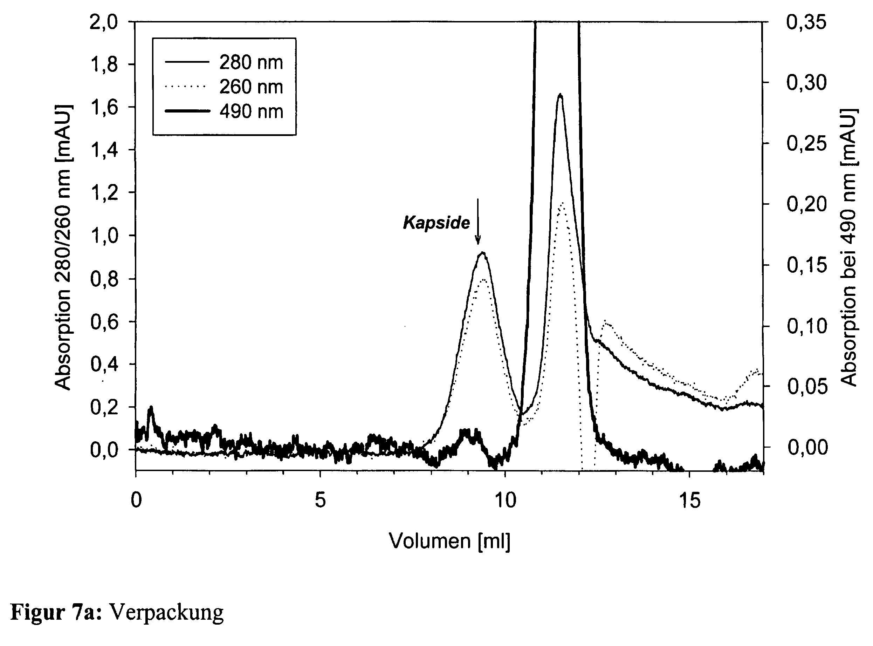

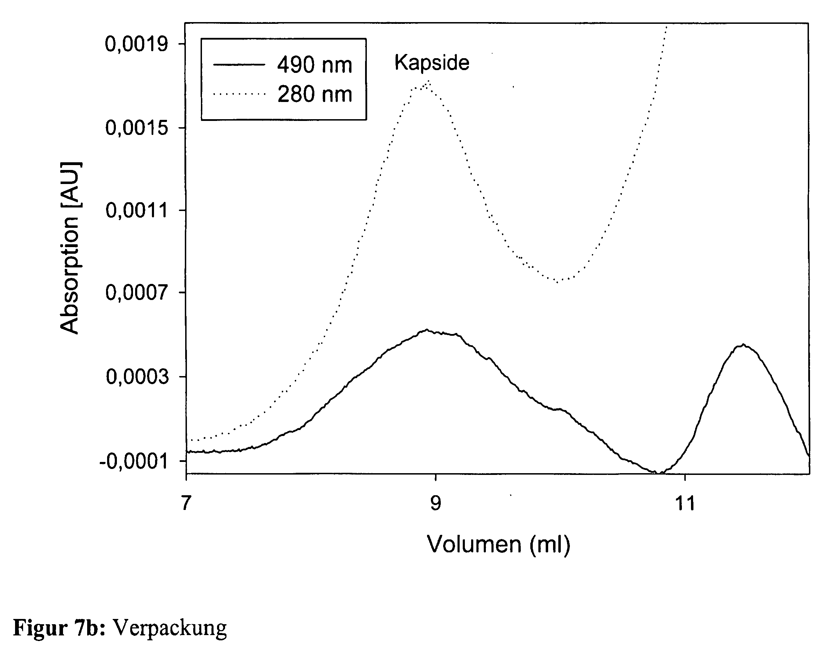

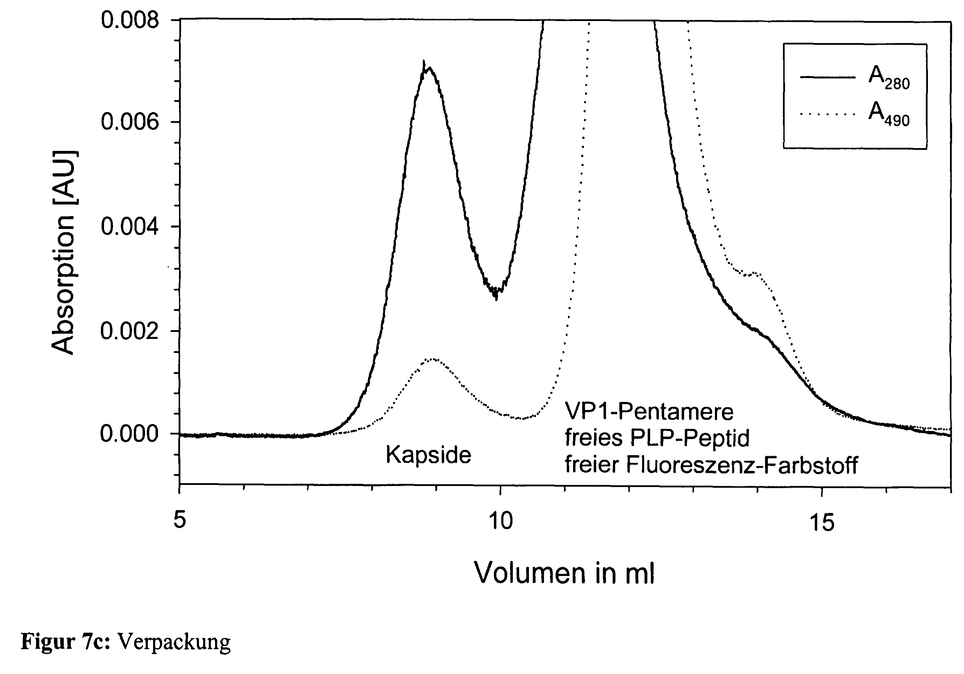

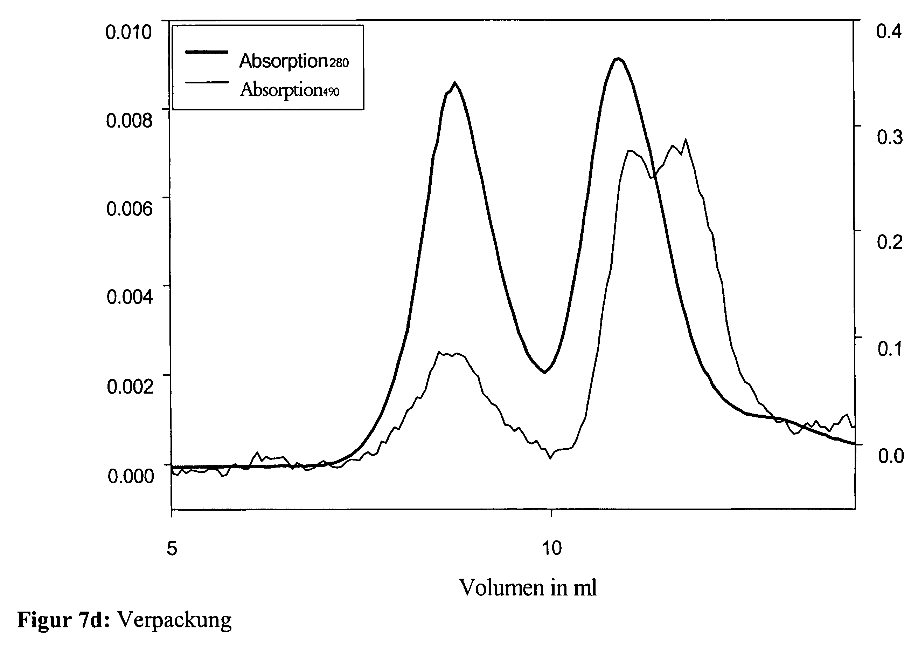

**Figur 7** zeigt die Verpackung von molekularen Substanzen in das Innere von virusähnlichen Hüllen auf der Basis von Polyomavirus VP1-Varianten. (a) Verpackung von GFP-PLP in Hüllen, die PyVP1-3C-WW1 enthalten. GFP-PLP wird vor der Assemblierung unter Standardbedingungen in sechsfachem molaren Überschuß zugesetzt. Gezeigt ist ein Gelfiltrationsexperiment (TSKgel G6000PWXL, Fa. TosoHaas), bei dem Kapsidfraktionen (Elution bei 9 ml) von freien, nichtassemblierten Pentameren der PyVP1-Varianten sowie dem GFP-Protein (11 bis 13 ml) abgetrennt werden. In der Kapsidfraktion ist eine detektierbare Menge an GFP vorhanden, die über die WW-Domänen/Polyprolin-Wechselwirkung in das Innere der Kapside dirigiert wurde. (b) Verpackung von GFP mit WW-Domäne am N-Terminus in das Innere von virusähnlichen Kapsiden, assembliert aus PyVP1-3C-[N-14]-PLP (prolinreiche Sequenz am verkürzten N-Terminus). Analog zu dem Beispiel in (a) wird GFP-WWI mit PyVPI-3C-[N-14]-PLP inkubiert und Kapside durch Assemblierung unter Standardbedingungen hergestellt. Dabei werden die Polyprolin-Peptide über die Affinität zur WW-Domäne in das Innere der Kapside verbracht. (c) Verpackung eines fluoreszenzmarkierten Peptids (prolinreiche Sequenz) in das Innere von virusähnlichen Kapsiden. Analog zu dem Beispiel in (a) wird das Peptid mit PyVP1-3C-WW[N-14] inkubiert und Kapside durch Assemblierung unter Standardbedingungen hergestellt. Dabei werden die Polyprolin-Peptide über die Affinität an die WW-Domäne in das Innere der Kapside verbracht. (d) Verpackung von GFP mit prolinreicher Sequenz am C-Terminus in das Innere von virusähnlichen Kapsiden, die mit PyVP1-3C-WW[N-14] assembliert sind. Analog zu dem Beispiel in (a) wird GFP-PLP mit PyVP1-3C-WW[N-14] inkubiert und Kapside durch Assemblierung unter Standardbedingungen hergestellt. Dabei wird das GFP-PLP über die Affinität zur WW-Domäne in das Innere der Kapside verbracht.

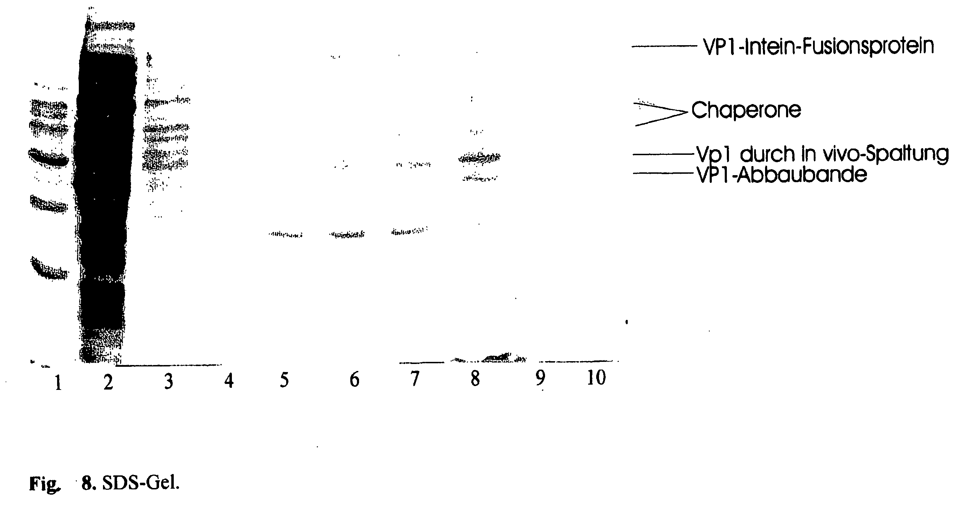

**Figur 8** zeigt ein SDS-Gel zur Darstellung der Reinigung von Proteinen mit WW-Domäne aus einem Substanzgemisch, hier einem Zellextrakt. Bahn 1: 10 kDa-Molekularmassenstandard; Bahn 2: Rohextrakt von (PyVP1-3C-WW1)-Intein-CBD-Fusionsprotein; Bahn 3: Durchlauf; Bahn 4 bis 10: Verschiedene Fraktionen der Elution des Fusionsproteins, mit 2 % SDS im Elutionspuffer. Die Immobilisierung des Fusionsproteins erfolgt dabei über eine Säule mit kovalent gebundenem prolinreichen Peptid. Nach dem Auftrag des Rohextraktes wird die Säule mit insgesamt 10 Säulenvolumina eines Puffers, der 2 M NaCI enthält, gewaschen. Neben dem Fusionsprotein werden Abbauprodukte davon sowie molekulare Chaperone, die bekanntermaßen an PyVP 1 binden, detektiert.

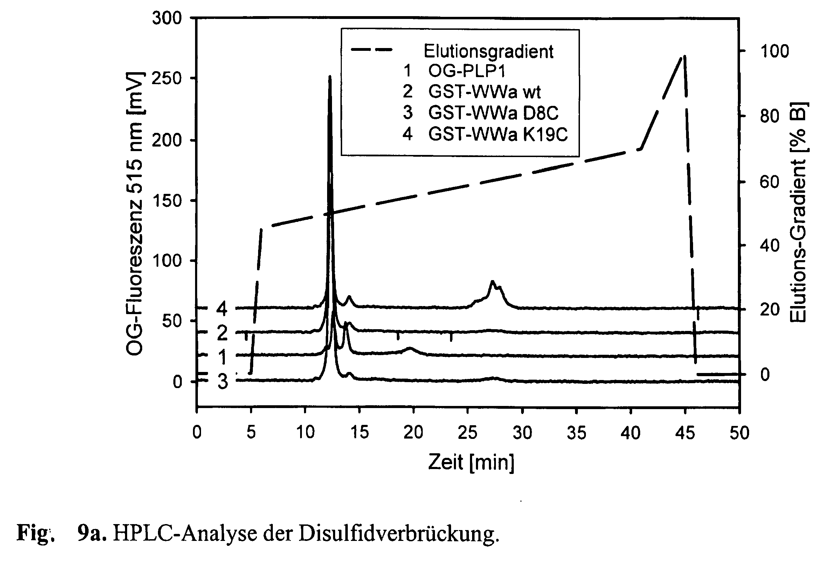

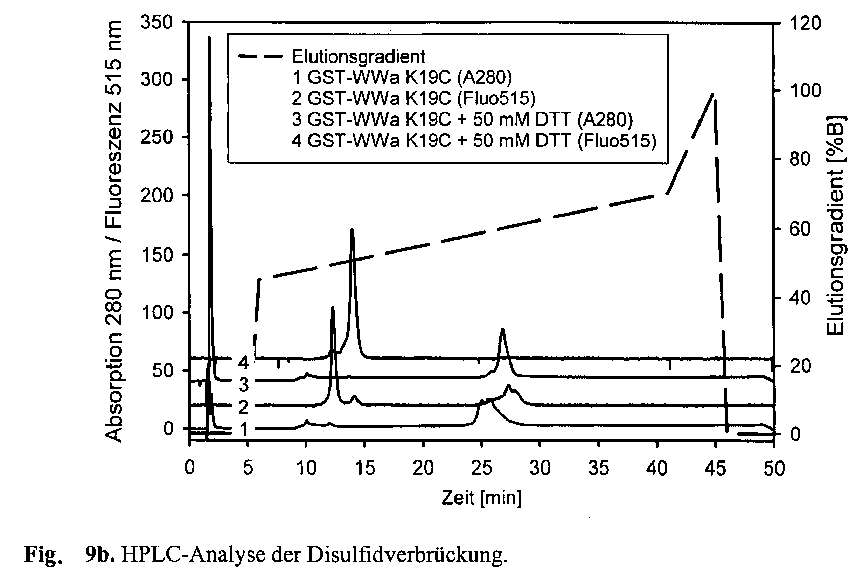

**Figur 9** zeigt eine HPLC-fluorimetrische Darstellung der Disulfidverbrückung einer prolinreichen molekularen Substanz mit einer WW-Domäne, die zum Zweck einer Affinitätsreinigung an Gluthathion-S-Transferase (GST) fusioniert wurde (Fig. 9a) und unter reduzierenden Bedingungen (50 mM DTT, Fig. 9b).

### Beispiel 1

### Insertion einer WW-Domäne in das äuβere Segment einer in vitro assemblierten, virusähnlichen Proteinhiille (PyVP1-WW150)

Im ersten Beispiel wird eine WW-Domäne der Aminosäuresequenz Gly-Ser-Gly-Trp-Thr-Glu-His-Lys-Ser-Pro-Asp-Gly-Arg-Thr-Tyr-Tyr-Tyr-Asn-Thr-Glu-Thr-Lys-Gln-Ser-Thr-Trp-Glu-Lys-Pro-Asp-Asp in einen spezifischen Loop eines viralen Hüllproteins eingesetzt. Dabei wird vor und nach der WW-Domäne zusätzlich ein Linker, bestehend aus alternierenden Gly-Ser-Aminosäuren, inseriert. Das verwendete virale Hüllprotein ist im gegebenen Beispiel das in Lösung pentamere Polyomavirus-VP1-Hüllprotein, das nach dem Stand der Technik *in vitro* zu einer virusähnlichen Hülle assemblierbar ist. Anhand der Kristallstruktur des Proteins läßt sich erkennen, daß eine Loop-Region in der Struktur in der Nähe der Aminosäureposition 150 für die Insertion der WW-Domäne möglicherweise geeignet ist, da sich diese Loop-Region bei Assemblierung des pentameren Proteins zu einer virusähnlichen Hülle auf der Außenseite der Hülle befindet.

Die Expression und Reinigung von PyVPI-WW150 erfolgt als Fusionsprotein mit einer C-terminal fusionierten Inteindomäne und einer sich daran anschließenden Chitin-Bindungsdomäne (CBD). Dazu wird zunächst ein Plasmid hergestellt, das auf dem Vektor pCYB2 des IMPACT-Systems (Fa. New England Biolabs) beruht. Über die multiple Klonierungsstelle des pCYB2 wird mit Hilfe der Restriktionsschnittstellen Ndel - XmaI (Fa. New England Biolabs) ein über PCR-Amplifikation hergestelltes DNA-Fragment nach Standardverfahren einkloniert, das für eine Variante des VP1-Gens von Maus-Polyomavirus codiert.

Als Basis hierfür wird eine Polyomavirus-Variante verwendet, die keinerlei Cysteine in der Sequenz aufweist; die sechs Cysteine des Wildtyp-Proteins wurden zuvor mit Hilfe von üblichen Mutagenisierungsverfahren durch Serin ersetzt. Diese Variante von PyVP1 hat den Vorteil, daß die Redoxbedingungen der Lösung keinen Einfluß auf den Zustand des Proteins haben; sie ist dadurch in vielen Anwendungen einfacher handhabbar. Darüber hinaus kann bei der späteren Einführung eines Cysteins in die inserierte WW-Domäne eine spezifische Disulfidverbrückung von WW-Domäne und prolinreicher Sequenz vorgenommen werden. Als weitere Variante wird eine Modifikation an der Stelle 249 verwendet; das dort im Wildtypprotein vorhandene Threonin wird durch Cystein ersetzt. An dieser Stelle im Protein ist eine Markierung mit Hilfe von Fluoreszenzfarbstoffen nach dem Stand der Technik vorteilhaft möglich. Die geschützte Lokalisation im Pentamer erlaubt die Markierung an dieser Stelle ohne unerwünschte Nebeneffekte. Die verwendete Variante von Polyomavirus VP1 wird korrekt mit PyVPI-CaIIS-T249C benannt, nachfolgend verkürzt als PyVP1 bezeichnet.

Für die PCR werden die folgenden Oligonucleotide als Primer verwendet: vp1NImp (5'-TAT ACA TAT GGC CCC CAA AAG AAA AAG C-3'), und vp1CImp (5'-ATA TCC CGG GAG GAA ATA CAG TCT TTG TTT TTC C-3'). Bei dieser PCR werden gleichzeitig die C-terminalen Aminosäuren des Wildtyp-VP1-Proteins von Gly383-Asn384 in Pro383-Gly384 umgewandelt, da ein C-terminal lokalisiertes Asparagin für das Intein-Spaltsystem sehr ungünstig bezüglich der Spalteigenschaften ist. Die genannten Punktmutationen beeinflussen im Weiteren nicht die wesentlichen Eigenschaften des PyVPI-Proteins. Der *tac*-Promotor des pCYB2-Vektors liefert nur geringe Expressionsmengen des Fusionsproteins, daher wird das Fusionskonstrukt PyVP1-Intein-CBD über eine weitere PCR aus dem pCYB2-Vektor isoliert und in einen hochexprimierenden pET-Vektor mit *T7lac*-Promotor (Plasmid pET21a, Fa. Novagen), über NdeI - EcoRI - Restriktionsschnittstellen, kloniert. Oligonucleotide: vp1-NImp (5'-TAT ACA TAT GGC CCC CAA AAG AAA AAG C-3'), und 5'-ATA TGA ATT CCA GTC ATT GAA GCT GCC ACA AGG-3'.

Die Klonierung der WW-Domäne als Insertion in den externen Loop von PyVP1 zwischen die Aminosäurepositionen 148 und 149 erfolgt in mehreren Schritten. Mit den Oligonukleotiden FBP11-WWaN (5'-ATA CTC TTC AGG CAG CGG CTG GAC AGA ACA TAA ATC ACC TGA TGG-3') und FBP11-WWaC (5'-ATA CTC TTC TAC CAC TAC CAT CAT CCG GCT TTT CCC AGG TAG ACT G-3') wird eine PCR auf ein DNA-Fragment durchgeführt, welches das forminbindende Protein 11 (FBP11) aus dem Organismus *Mus musculus* (Maus) enthält. In dieser Gensequenz ist unter anderem eine WW-Domäne kodiert. Die Oligonukleotide führen gleichzeitig eine kurze Linkersequenz von je 5 Aminosäuren, bestehend aus alternierenden Glycin-Serin-Aminosäuren, ein. Eine zweite PCR auf den vorstehend beschriebenen Vektor amplifiziert das N-terminale Fragment von PyVP1 zwischen den Aminosäuren 1 und 148 mit Hilfe der Oligonukleotide vp1NImp (s. oben) und vp1-150-WWaC (5'-ATA CTC TTC AGG TAG CGG CGT AAA CAC AAA AGG AAT TTC CAC TCC AG-3'). Eine dritte PCR schließlich amplifiziert auch das C-terminale Fragment von PyVP1 zwischen den Aminosäuren 149 und dem C-terminalen Ende des Proteins, mit Hilfe der Oligonukleotide vpl-150-WWaN (5'-ATA CTC TTC AGC CGC TGC CTG TAT CTG TCG GTT TGT TGA ACC CAT G-3') und vp1CImp (s. oben).

Alle drei PCR-Produkte werden anschließend mit dem Typ IIS-Restriktionsenzym EamI 104 I (Fa. Stratagene) verdaut. Das N-terminale und das C-terminale Fragment von PyVP1 (PCR-Produkte 2 und 3, s. oben) werden mit Hilfe von Alkalischer Phosphatase (CIP, Fa. New England Biolabs) dephosphoryliert, um im nachfolgenden Ligationsschritt aus den drei aufbereiteten PCR-Fragmenten eine Gensequenz zu schaffen mit folgender Reihenfolge: (PyVP1-N-Terminus) - WW-Domänen-Fragment - (PyVP1-C-Terminus). Eine anschließende PCR mit den Oligonukleotiden vp1NImp und vp1CImp (s. vorstehend) amplifiziert nun das Ligationsprodukt der drei Fragmente, nachfolgend abgekürzt als PyVP1-WW150 bezeichnet. Das PCR-Produkt kann anschließend mittels Standardverfahren in den Vektor pCRblunt (Fa. Invitrogen) kloniert werden. Nach Ausschneiden des klonierten Fragmentes PyVP1-WW150 mit Hilfe der Restriktionsenzyme Nde I - Sma I kann im Anschluß die finale Klonierung in das bereits zuvor beschriebene Plasmid pET21a erfolgen.

Der zuletzt entstandene Vektor erlaubt die Expression des Fusionsproteins (PyVP1-WW150)-Intein-CBD mit Hilfe des hochexprimierenden *T7lac*-Promotors in *E. coli* BL21(DE3)-Zellen (Fa. Novagen). Dazu werden transformierte Zellen in 5 1 - Erlenmeyerkolben, die je 2 1 LB-Medium enthalten, bei 37 °C angezogen, bis die OD₆₀₀ der Kultur 2.0 bis 2.5 beträgt. Die Induktion der Proteinexpression erfolgt durch 1 mM IPTG im Medium. Die Kulturen werden danach für weitere 20 Stunden bei 15 °C inkubiert; die niedrige Temperatur minimiert die Abspaltung des Intein-Anteils im Fusionsprotein unter in vivo-Bedingungen. Die Zellen werden durch Zentrifugation geerntet, in 70 ml Resuspensionspuffer (20 mM HEPES, 1 mM EDTA, 100 mM NaCl, 5 % (w/v) Glycerol, pH 8.0) aufgelöst, und durch Hochdruck-Homogenisation aufgeschlossen. Nach Zentrifugation des Rohextraktes für 60 min bei 48 000 g wird ein klarer Zellextrakt erhalten. Dieser Extrakt wird mit einer Flußrate von 0.5 ml/min bei einer Temperatur von 10 °C auf eine 10 ml Chitin-Affinitäts-Säule (New England Biolabs) aufgetragen. Die Säule wird anschließend mit 3 Säulenvolumina des Resuspensionspuffers, 15 Säulenvolumina eines Waschpuffers hoher Ionenstärke (20 mM HEPES, 1 mM EDTA, 2 M NaCl, 5 % (w/v) Glycerol, pH 8.0) und wiederum 3 Säulenvolumina des Resuspensionspuffers gewaschen; dadurch werden alle unerwünschten *E*. *coli-*Wirtsproteine von der Chitin-Matrix entfernt.

Die Abspaltung des PyVPI-WW150-Monomers aus dem Fusionsprotein mittels der selbstspleißenden Inteinaktivität wird durch einen Puls (3 Säulenvolumina) mit jeweils 50 mM Dithiothreitol (DTT), 50 mM Hydroxylamin, oder 30 mM DTT zusammen mit 30 mM Hydroxylamin, im Resuspensionspuffer induziert. Dazu wird die beladene Chitinmatrix mit einer der angegebenen Lösungen für 14 Stunden bei 10 °C inkubiert. Das PyVPI-WW150-Protein wird dabei vollständig freigesetzt und kann mittels säulenchromatographischer Standardverfahren von der Chitinmatrix und den an der Matrix anhaftenden übrigen Bestandteilen des Fusionsproteins abgetrennt werden. Dazu wird geeigneterweise ein linearer Salzgradient mit einer Konzentration zwischen 0.1 und 2.0 M NaCl verwendet. Die Regeneration der Chitinmatrix erfolgt nach den Angaben des Herstellers durch Waschen des Chitin-Materials mit 3 Säulenvolumina eines SDS-haltigen Puffers (1 % SDS (w/v) in Resuspensionspuffer).

Das PyVP1-WW150-Protein wird im beschriebenen Verfahren als lösliches Pentamer exprimiert und ist nativ. Fig. 2a zeigt ein SDS-Gel mit den gereingten Fraktionen von Wildtyp-PyVPI (bzw. der davon abgeleiteten Variante PyVP1-CallS-T249C) und der PyVP1-WW150-Variante, die aufgrund der zusätzlichen inserierten Aminosäuren eine höhere Masse aufweist. Fig. 2b zeigt vergleichende CD-Spektren der hergestellten Proteine in 10 mM HEPES, 150 mM NaCl, pH 7.2, die eine korrekte Faltung der Proteinspezies zeigen. Eine Dekonvolution der beiden CD-Spektren nach dem Stand der Technik zeigt, daß im Falle der PyVPI-WW150-Domäne eine Zunahme an β-Faltblattstruktur gegenüber dem PyVPI-Protein zu verzeichnen ist. Dies weist darauf hin, daß die inserierte WW-Domäne ihre native Struktur als β-Faltblatt beibehalten hat.

Das Beispiel zeigt, daß überraschenderweise die WW-Domäne mit korrekter Faltung unter geeigneten Bedingungen in Loopbereiche von Proteinstrukturen inseriert werden kann, ohne deren native Struktur wesentlich zu stören. Das in Lösung pentamere PyVP 1-WW150-Protein enthält die native WW-Domäne inseriert in die Polypeptidkette und präsentiert diese bei Assemblierung auf der Außenseite der virusähnlichen Hülle (vgl. Beispiel 2).

### Beispiel 2

### Charakterisierung der Eigenschaften von PyVP1-WW150

Anhand des Beispiels 1 kann ein Protein (PyVP1-WW150) hergestellt werden, das artifiziell eine WW-Domäne inseriert hat. Die Bindungseigenschaften von PyVP1-WW150 in Bezug auf prolinreiche Liganden können mit verschiedenen Verfahren charakterisiert werden. Eine dafür günstige Methode bedient sich der Oberflächen-Plasmon-Resonanz; im vorgegebenen Beispiel wird dazu das Gerät Biacore X (Fa. Biacore AB) verwendet. Ein synthetisches Peptid der Sequenz Cys-Ser-Gly-Pro-Pro-Pro-Pro-Pro-Pro-Pro-Pro-Leu-Pro wird nach Angaben des Herstellers über eine Thiol- bzw. Aminokopplung an einen Sensorchip Typ CM5 gekoppelt. Dabei wird zunächst eine Menge von 80 Resonanzeinheiten (RU) des angegebenen Peptids an der Oberfläche immobilisiert. Die nachfolgenden Messungen erfolgen jeweils bei 25 °C und einer Flußrate von 20 µl/min.

Es werden Bindungsstudien von PyVP1-WW150 an den Sensorchip mit immobilisiertem prolinreichen Peptid unter verschiedenen Lösungsmittelbedingungen durchgeführt. Die erste Messung erfolgt unter Standard-Lösungsmittelbedingungen, mit 10 mM HEPES, 1 mM EDTA, 150 mM NaCl, pH 7.2. Die Proteinkonzentration von PyVP1-WW150 wird im Bereich von 5 bis 50 nM variiert. Anhand von Fig. 3a ist ersichtlich, daß PyVP1-WW150 mit hoher Affinität an den Sensorchip bindet. Die gebundene Menge ist, wie erwartet, proportional zur eingesetzten Proteinkonzentration. Die Bindungskonstante K_{D} des PyVPI-WW150-Proteins wird dabei mit einem Wert von 5 nM bestimmt (Fig. 3a). Wie aus der Figur weiterhin ersichtlich ist, ist die Bindung nicht dauerhaft, sondern das Protein dissoziiert nach der Beladung der Sensoroberfläche in einem langsamen Prozeß wieder ab. Dies zeigt, daß die Wechselwirkung der Interaktionspartner reversibel ist.

Zum Test der Bindung unter Bedingungen physiologischer Ionenstärke wird bei der zweiten Messung als Lösungsmittel Dulbecco's PBS verwendet (Fa. Gibco). Die übrigen experimentellen Bedingungen werden analog zum vorstehend beschriebenen ersten Versuch gewählt. Fig. 3b demonstriert, daß die Bindung des PyVPI-WW150 mit Dulbecco's PBS keine signifikanten Unterschiede gegenüber der Bindung unter Standardbedingungen (Fig. 3a) aufweist. Aus dem Experiment können Bindungsparameter für die Assoziation (Kₒₙ =2x10⁵ M⁻¹s⁻¹) und Dissoziation (K_{off} = 1.8×10⁻³ s⁻¹) abgeleitet werden. Das Beispiel zeigt, daß die veränderten Lösungsmittelbedingungen keinen wesentlichen Einfluß auf die Bindung des PyVP1-WW150 an das prolinreiche Peptid besitzen, und legt nahe, daß die Interaktion der beiden Partner auch unter physiologischen Bedingungen stabil erfolgt. Damit ist eine prinzipielle Anwendbarkeit des Systems auch unter klinischen Bedingungen im Rahmen von Diagnostika oder Therapeutika möglich.

Zur Bestimmung der Spezifität der Bindung wird in einer dritten Messung als Laufpuffer Dulbecco's MEM-Medium mit 10 % FCS (Fötales Kälberserum, Fa. Gibco) verwendet. FCS stellt dabei ein Modellsystem dar für ein Gemisch aus verschiedenen Proteinen und anderen Substanzen, die in biologischen Systemen relevant sind. Fig. 3c zeigt, daß auch unter diesen Bedingungen eine signifikante und spezifische Bindung des PyVP1-WW150-Proteins an die Sensoroberfläche zu verzeichnen ist. Wie bei den beiden vorstehend beschriebenen Messungen ist auch hier das Response-Signal an der Sensoroberfläche proportional zu der eingesetzten Konzentration an PyVP1-WW150-Protein. Damit ist gezeigt, daß die Wechselwirkung des PyVP1-WW150 mit dem immobilisierten prolinreichen Peptid unabhängig ist von dem Vorhandensein eines Gemisches anderer Substanzen, wie sie beispielsweise im Serum vorkommen.

Zusammenfassend zeigen diese drei Untersuchungen mit Hilfe der Biacore-Technik und einer Sensoroberfläche mit immobilisiertem prolinreichen Peptid, daß die Bindung zwischen molekularen Substanzen, die eine WW-Domäne enthalten, und prolinreichen Liganden mit hoher Affinität und Spezifität erfolgt. Die Wechselwirkung ist dabei reversibel und nicht wesentlich von den gewählten Lösungsmittelbedingungen abhängig. Die Dissoziation erfolgt vergleichsweise langsam gegenüber der Assoziation; die Dissoziationskonstante liegt bei 20 nM.

### Beispiel 3

### Immobilisierung an einer Matrix

Ein weiteres Verfahren zur Charakterisierung von Bindungseigenschaften ist eine reversible Immobilisierung der WW-Domäne an einer inerten Matrix. Dazu wird ein synthetisches prolinreiches Peptid (Sequenz Cys-Ser-Gly-Pro-Pro-Pro-Pro-Pro-Pro-Pro-Pro-Leu-Pro) über eine Thiolkopplung an Sulfolink-Säulenmaterial (Fa. Pierce) nach Vorschrift des Herstellers gekoppelt. Mit der auf diese Weise modifizierten Matrix wird eine Chromatographie-Säule beladen. Dies erlaubt ein Auftragen der Proben auf die Matrix und Elution von gebundenen Proteinen unter verschiedenen Bedingungen. Das nach Beispiel 1 gereinigte PyVPI-WW150-Protein wird auf die Säule aufgetragen (Lösungsmittel 10 mM HEPES, 1 mM EDTA, 150 mM NaCl, 5 % Glycerin, pH 7.2). Wie aus Figur 4 ersichtlich ist, bindet das Protein an die Matrix und erscheint nur in geringen Mengen in den Waschfraktionen. Eine nachfolgende Elution des Proteins von der Matrix ist durch Zugabe von 1 % SDS oder 300 mM Arginin zum Laufpuffer möglich.

Dieses Experiment zeigt, daß das PyVP1-WW150-Protein in der Lage ist, reversibel an eine Matrix zu binden, die ein prolinreiches Peptid trägt. Dadurch kann eine zeitweise Immobilisierung erfolgen. Ein Ablösen des Proteins von der Matrix ist durch Zugabe von Additiven zum Laufpuffer möglich.

### Beispiel 4

### Bindung eines prolinreichen Peptids an ein Kapsid

In einem weiteren Experiment wird die Bindung eines fluoreszenzmarkierten Peptids mit prolinreicher Sequenz an die Oberfläche (Außenseite) von virusähnlichen Kapsiden untersucht. Die Assemblierung des Proteins erfolgt dabei in Analogie zu bereits beschriebenen Bedingungen nach dem Stand der Technik (vgl. Salunke, Caspar & Garcea, Polymorphism in the assembly of polyomavirus capsid protein VP1, *Biophys. J.* 56, S. 887-900, 1989). Die virusähnlichen Kapside werden nach Dialyse des Proteins gegen 10 mM HEPES, 50 mM NaCl, 0.5 mM CaCl₂, 5 % Glycerin, pH 7.2, erhalten. Das prolinreiche Peptid Cys-Ser-Gly-Pro-Pro-Pro-Pro-Pro-Pro-Pro-Pro-Leu-Pro wird mit einem Fluorescein-Maleimid-Derivat (Fa. Molecular Probes) nach Herstellerangaben spezifisch am N-terminalen Cystein markiert. Nach der Assemblierung der Virusprotein-Varianten zu Kapsiden wird ein zehnfacher molarer Überschuß an fluoreszenzmarkiertem Peptid zugesetzt. Durch Gelfiltration (Säule TSKGel G5000PWXL, Fa. TosoHaas) lassen sich eindeutig virusähnliche Kapsidhüllen nachweisen und von freien, nicht assemblierten Kapsidbausteinen sowie von überschüssigem Peptid und Fluoreszenzfarbstoff abtrennen. Das auf die Oberfläche der Kapside an die dort befindlichen WW-Domänen gebundene Peptid eluiert in den Kapsidfraktionen und kann durch die spezifische Absorption des Fluoreszenzfarbstoffes nachgewiesen werden (Fig. 5).
Dieses Beispiel zeigt, daß die PyVP1-WW150-Variante unter geeigneten Bedingungen Kapsidstrukturen (virusähnliche Hüllen) ausbilden kann. Diese Kapside sind dazu in der Lage, das prolinreiche Peptid zu binden. Damit lassen sich über die spezifische und starke Wechselwirkung von WW-Domäne und prolinreicher Sequenz molekulare Substanzen gerichtet auf die Oberfläche (Außenseite) der virusähnlichen Strukturen aufbringen.

### Beispiel 5

### Verpacken von GFP in das Innere einer virusähnlichen Proteinhülle

In diesem Beispiel wird gezeigt, daß durch günstige Positionierung der Adaptersegmente eine Dirigierung von molekularen Substanzen in das Innere von Virusüllen oder von virusähnlichen Hüllen (Kapsiden) erfolgen kann. Aufgrund der dreidimensionalen Struktur von Polyomavirus VP1 ist nach dem Stand der Technik bekannt, daß der N-Terminus des Proteins nach der Assemblierung zum Kapsid im Innenraum der Hülle lokalisiert ist. Dabei sind möglicherweise die ersten 14 Aminosäuren des Proteins entbehrlich, da sie in der Röntgenstruktur des Kapsids nicht detektiert werden können. Es werden daher zwei verschiedene Varianten des PyVPI-Proteins hergestellt, die eine WW-Domäne am Aminoterminus des nativen Wildtyp-Proteins enthalten (Variante PyVP1-3C-WW1), bzw. die WW-Domäne an einem um 14 Aminosäuren verkürzten N-Terminus tragen (Variante PyVP1-3C-WW[N-14]) und eine Variante des PyVPI-Proteins, das eine prolinreiche Sequenz am N-Terminus trägt (PyVP1-3C-[N-14]-PLP). Basis für diese Varianten ist eine PyVP1-Variante, das über die Cysteine C19 und C114 verfügt und in die zusätzlich ein spezifisches neues Cystein eingeführt ist (analog zur Variante PyVP1-CallS-T249C). Diese Variante wird im folgendenen abgekürzt mit PyVP1-3C.

Zunächst wird eine Amplifikation der WW-Domäne mittels PCR durchgeführt; als Templat dient dabei analog zu Beispiel 1 das FBP11-Gen der Maus. Als Oligonukleotide für die PCR werden dabei 5'-AAT ATA TCA TAT GTC CAT CAT CCG GCT TTT CCC AGG TAG ACT-3' (mit NdeI-Schnittstelle), und 5'-TAT TAA TCA TAT GAG CGG CTG GAC AGA ACA TAA ATC ACC TGA TGG-3' verwendet. Das erhaltene PCR-Produkt wird anschließend über die mittels der Oligonukleotide eingeführten Schnittstellen Nde I - Nde I in den Expressionsvektor pET21a aus Beispiel 1 einkloniert, der das Gen für ein Fusionsprotein PyVP1-Intein-CBD enthält; am 5'-Ende des Gens befindet sich dabei eine singuläre Nde I - Schnittstelle. Das exprimierte Genprodukt dieses Vektors ist das gewünschte Protein PyVP1-3C-WW1. Analog dazu wird die Klonierung mit einem um 14 Aminosäuren verkürzten Fragment von PyVP1-3C durchgeführt (PyVPI-3C-WW[N-14]), nach dem in Beispiel 1 beschriebenen Standardverfahren. Dazu wird eine PCR durchgeführt auf das PyVP1-Genfragment, mit 5'-GCG CGC GCA TAT GAG CAC CAA GGC TAG CCC AAG ACC CG-3' und dem Oligonukleotid vp1CImp (vgl. Beispiel 1). Das dabei entstehende PCR-Produkt wird mit den Restriktionsenzymen Nde I - Sma I verdaut und das Fragment nach Standardverfahren in den Vektor pET21 a aus Beispiel 1 kloniert. Expression und Reinigung der beiden Proteine erfolgt in Übereinstimmung mit Beispiel 1. Die gereinigten Proteine sind den Varianten PyVP1 und PyVP1-WW150 in der Fig. 6a vergleichend gegenübergestellt. Es zeigt sich, daß die Proteine löslich und nativ herstellbar sind. Die Veränderung des N-Terminus durch die Einführung der WW-Domänen hat keinen wesentlichen negativen Einfluß auf die Assemblierungskompetenz des Proteins zur Ausbildung von virusähnlichen Hüllstrukturen.

In analoger Weise wird die Herstellung und Reinigung einer GFP-Variante vorgenommen. GFP ist ein Protein, das im nativen Zustand eine grüne Fluoreszenz zeigt (Absorptionsmaximum bei 490 nm). Es eignet sich hervorragend zur Markierung von Komplexen und Assoziaten. Zur Herstellung einer GFP-Variante mit einer prolinreichen terminalen Sequenz erfolgt zunächst eine PCR-basierte Amplifikation des GFP-Gens, wobei als Templat das Plasmid pEGFP-N1 (Fa. Clontech) verwendet wird. Gleichzeitig werden geeignete Restriktionsschnittstellen in das PCR-Produkt eingeführt. Die PCR erfolgt mittels der Oligonukleotide 5'-TTA TTT ACA TAT GGT GAG CAA GGG CGA GGA G-3' (mit Nde I-Schnittstelle), und 5'-ATA TCT TAA GTA CAG CTC GTC CAT GCC G-3' (mit Afl II-Schnittstelle). Das so erhaltene PCR-Produkt wird über die Restriktionsschnittstellen in den Vektor pTIP kloniert und dort exprimiert. Dieser Vektor pTIP ist ein Derivat des in Beispiel 1 dokumentierten Intein-Reinigungsvektors auf Basis von pET21a, mit zusätzlich eingeführten prolinreichen Sequenzen. Der Vektor ist so konstruiert, daß wahlweise am 5'- oder 3'-Ende eines über eine multiple Klonierungsstelle eingefügten Gens eine prolinreiche Sequenz anfusioniert wird. Die prolinreiche Sequenz enthält dabei vorwiegend Pro-Pro-Pro-Pro-Pro-Pro-Pro-Pro-Leu-Pro. Die Herstellung und Reinigung des GFP-PLP-Proteins erfolgt mittels Chitinaffinitätschromatographie, in inhaltlicher Übereinstimmung mit dem Beispiel 1. Die erfolgreiche Herstellung und Reinigung von GFP-PLP ist in Fig. 6b dokumentiert. Das GFP-PLP-Protein, das am C-Terminus die prolinreiche Sequenz Pro-Pro-Pro-Pro- Pro-Pro-Pro-Pro-Leu-Pro trägt, kann löslich in großer Menge hergestellt werden. Die grün leuchtende Farbe der Proteinlösung zeigt gleichzeitig, daß das Protein zu seiner nativen Struktur falten kann.

Analog dazu erfolgte die Herstellung der Py-VP1-3C-PLP Variante. Zur Herstellung dieser PyVP1 Variante wurde PyVP1-3C-[N-14] in den Vektor pTIP so kloniert, dass die im Vektor enthaltene prolinreiche Sequenz N-terminal an das Py-VP1-3C-[N-14] fusioniert wurde.

Zur Überprüfung der funktionellen Eigenschaften der beiden PyVPI-Varianten mit WW-Domäne am jeweiligen N-Terminus der Proteine werden die beiden Varianten mit Proteinen inkubiert, die prolinreiche Sequenzen enthalten. Das PyVP1-3C-WW1-Protein wird mit dem zuvor hergestellten Protein GFP-PLP (molares Verhältnis 1 : 6) für 10 min inkubiert (10 mM HEPES, 1 mM EDTA, 150 mM NaCl, 5 % Glycerol, pH 7.2), und die Kapsidbildung der PyVPI-Varianten durch Dialyse gegen einen Puffer, der 0.5 mM CaCl₂ enthält, induziert (vgl. Beispiel 4). Der erfolgreiche Nachweis von Kapsiden (Fig. 7a) zeigt, daß die Variante PyVP1-3C-WW1 unter geeigneten Bedingungen assemblierungskompetent ist. Anhand von Gelfiltrationsuntersuchungen (Säule Säule TSKGel G6000PWXL, Fa. TosoHaas) kann gezeigt werden, daß ein geringer Teil des nativen GFP-PLP-Proteins (identifizierbar anhand der spezifischen Absorption bei 490 nm) in der Kapsidfraktion (bei Elutionsvolumina zwischen 9 und 10 ml) enthalten ist (Fig. 7a). Das bedeutet, daß während der Inkubation des GFP-PLP-Proteins mit der PyVPI-3C-WW[N-14]-Variante eine Bindung der beiden Proteine aneinander erfolgte, wodurch das GFP bei der nachfolgenden Kapsidassemblierung in das Innere des virusähnlichen Partikels dirigiert wurde.

### Beispiel 6

### Verpacken eines Peptides in das Innere einer virusähnlichen Proteinhülle

In einem zweiten Experiment analog zum Experiment 5 wird das PyVPl-3C-WW[N-14] in einem molaren Verhältnis von 1:10 mit einem prolinreichen Peptid inkubiert, das zuvor fluoreszenzmarkiert wird. Die Markierung des Peptids (Cys-Ser-Gly-Pro-Pro-Pro-Pro-Pro-Pro-Pro-Pro-Leu-Pro) erfolgt dabei mittels Fluorescein-Maleimid (Fa. Molecular Probes) gemäß den Herstellerangaben über eine Maleimidkopplung des Farbstoffes an das N-terminale Cystein. Wiederum zeigt sich wie im Beispiel 5 nach der Assemblierung der PyVP1-3C-WW[N-14]-Variante, daß PyVP1-3C-WW[N-14] unter geeigneten Bedingungen assemblierungskompetent ist. Darüber hinaus ist das Protein dazu in der Lage, das prolinreiche Peptid zu binden und bei der Assemblierung zu einer Proteinhülle das prolinreiche Peptid in das Innere der Kapside zu verbringen. Dies zeigt sich bei der Gelfiltration in Fig. 7b durch die spezifische Absorption des am Peptid kovalent gebundenen Fluoreszenzfarbstoffes bei 490 nm, die vorwiegend im Elutionsbereich der Kapside (9 bis 10 ml) zu finden ist.

Außerdem kann anhand der Varianten von PyVP1-3C-[N-14]-PLP (prolinreiche Sequenz am N-Terminus) und GFP-WW1 (WW-Domäne am N-Terminus) ein analoger Assemblierungsversuch durchgeführt werden. Dieser zeigt, daß auch eine umgekehrte Anordnung von WW-Domäne und prolinreichem Liganden an die zu verbindenden Substanzen, also die Plazierung der prolinreichen Sequenz an das Polyoma-Hüllprotein (Kapsid) und der WW-Domäne an das zu verpackende Protein, zu einer erfolgreichen Dirigierung des GFP in das Innere der virusähnlichen Hülle führt.

Zusammenfassend zeigen die Experimente in den Beispiele 5 und 6, daß Varianten von PyVP1 mit N-terminal anfusionierter WW-Domäne dazu in der Lage sind, prolinreiche Sequenzen zu binden und diese sowie die ggf. daran befindlichen molekularen Substanzen bei einer Assemblierung zu Kapsiden unter geeigneten Bedingungen in das Innere von virusähnlichen Hüllen zu dirigieren. Damit ist das beschriebene Verfahren dazu geeignet, eine gerichtete Verpackung von molekularen Substanzen in Viren bzw. in virusähnliche Kapside zu bewirken. Ebenfalls konnte gezeigt werden, daß Varianten von PyVP1 mit N-terminal anfusionierter prolinreicher Sequenz in der Lage sind, WW-Domänen und daran befindliche molekulare Substanzen zu binden.

### Beispiel 7

### Disulfidverbrückung zur kovalenten Verknüpfung auf der Basis von modifizierten WW-Domänen und prolinreichen Peptiden

Die in den vorstehenden Beispielen 1 bis 6 beschriebenen Untersuchungen zeigen, daß durch die Interaktion der WW-Domäne mit einer prolinreichen Peptidsequenz eine zeitlich befristete Zusammenlagerung dieser beiden Adaptersegmente erfolgen kann. Eine dauerhafte Verbrückung der Interaktionspartner kann dadurch erfolgen, daß die beiden Adaptersegmente mit spezifisch eingeführten Cystein-Aminosäuren ausgestattet werden, die bei geeigneter Positionierung eine Disulfidverbrückung nach der Assoziation der Bindungspartner erlauben.

Durch Punktmutationen, die gemäß nach dem Stand der Technik üblichen Verfahren durchgeführt werden, lassen sich sowohl in der WW-Domäne als auch in den prolinreichen Sequenzen des Liganden einzelne Aminosäuren, die nicht essentiell für die Anlagerung beider Adaptersegmente sind, in Cysteine überführen. Unter geeigneten Redoxbedingungen (oxidierend) kann dann spezifisch eine Disulfidbrücke zwischen dem angelagerten prolinreichen Liganden und der WW-Domäne, jeweils ein oder mehrere Cysteine enthaltend, ausgebildet werden. Diese Verbrückung wird dabei entscheidend durch die Anlagerung von WW-Domäne und dem Liganden begünstigt. Die zeitlich begrenzte Wechselwirkung zwischen unverknüpfter WW-Domäne und prolinreichem Liganden hält dabei lange genug vor, um die kovalente Verbrückung über die Disulfidbrücke auszubilden. Die Wechselwirkung der beiden Adaptersegmente wird dadurch zeitlich unbegrenzt, da Disulfidbrücken unter physiologischen Bedingungen, wie sie beispielsweise im extrazellulären Raum vorliegen, stabil sind. Falls erwünscht, kann *in vitro* unter reduzierenden Bedingungen (beispielsweise 50 mM DTT, DTE oder β-Mercaptoethanol) die Disulfidbrücke zwischen WW-Domäne und prolinreichem Liganden wieder aufgehoben werden; bei Entfernung des Reduktionsmittels ist auch eine Wiederverbrückung möglich.

Ausgehend von der Variante PyVP1-WW150 des Hüllproteins VP1 des murinen Polyomavirus wird durch Mutagenese eine Aspartat-Aminosäure (Position 8 in der WW-Domäne) in ein Cystein umgewandelt. Die resultierende Cystein-haltige Variante wird im folgenden PyVP1-WW150-D8C benannt. Über Bindungsstudien, basierend auf Oberflächenplasmonresonanz, läßt sich zeigen, daß diese Variante der WW-Domäne noch ohne Ausbildung der Disulfidbrücke den prolinreichen Liganden Cys-Ser-Gly-Pro-Pro-Pro-Pro-Pro-Pro-Pro-Pro-Leu-Pro bindet. Das Ausmaß der Wechselwirkung ist jedoch etwas geringer als bei PyVPI-WW150. Dies läßt sich offenbar auf das neu eingeführte Cystein zurückführen. Es kann jedoch gezeigt werden, daß diese Anlagerung durch Zusatz von 500 mM Ammoniumsulfat wiederum verstärkt werden kann. Dadurch werden vermutlich die hydrophoben Wechselwirkungen zwischen prolinreichem Liganden und WW-Domäne intensiviert. Die Stärke der Wechselwirkung ist somit durch die Ammoniumsulfat-Konzentration im Lösungsmittel modulierbar.

Die Ausbildung der Disulfidbrücke zwischen prolinreichem Liganden und WW-Domäne erfolgt anschließend unter leicht oxidierenden Bedingungen. Dazu wird ein Puffer verwendet, der sowohl Ammoniumsulfat enthält als auch definierte Redoxbedingungen aufrechterhält. Erreicht wird letztere Bedingungen durch Einsatz von 1 mM GSSG und 5 mM GSH im Redoxpuffer (50 mM Tris, pH 8.5, 1 mM EDTA, 500 mM Ammoniumsulfat); oxidiertes (GSSG) bzw. reduziertes Glutathion (GSH) fungiert dabei als Redox-Shuffling-System zur Ausbildung der Disulfidbrücken (vgl. R. Rudolph, In vitro folding of inclusion body proteins, *FASEB J.* 10, 49-56, 1996). Die Disulfidverbrückung wird bei 15°C für 24 h durchgeführt und durch Dialyse gegen 50 mM Tris, 1 mM EDTA, pH 7, beendet. Unter letztgenannten Bedingungen findet kein Disulfidaustausch mehr statt; die gebildeten Disulfidbrücken sind stabil.

Zur Verbrückung wurden zwei Varianten einer WW-Domäne verwendet, bei denen jeweils an einer Position eine Aminosäure gegen Cystein ausgetauscht wurde. Das ist zum einen die Variante D8C, bei der das Aspartat an Position 8 in der WW-Domäne gegen Cystein ausgetauscht wurde und zum anderen K19C, bei der Lysin19 durch Cystein ersetzt wurde. Die molekulare Substanz stellt dabei ein prolinreiches Peptid mit der Sequenz CSGP₈LP dar, das zum Zweck der Analytik an der Aminogruppe des N-Terminus mit einem Fluoreszenzfarbstoff (Oregon Green, OG, Firma Molecular Probes) markiert wurde. Die Disulfidverbrückung wurde wie in Beispiel 7 beschrieben durchgeführt. Um die Verbrückung zu analysieren, wurde die Probe einer Reversed-Phase-HPLC unterzogen (HPLC-Säule: YMC Protein-RP C₁₈: Laufpuffer A: 0.1% TFA in H2O, Laufpuffer B: 80% ACN, 0.1% TFA). Anhand dieser Chromatographie ließen sich WW-Domäne und freies, nichtverbrücktes Peptid voneinander trennen (Elutionszeiten: Peptid 12 min, WW-Domäne 25-27 min), während disulfidverbrücktes Peptid mit der WW-Domäne nahezu koeluiert (28 min). Anhand der Fluoreszenzmarkierung des Peptids läßt sich das Peptid zusammen mit der WW-Domäne detektieren. Figur 9 (a) zeigt, daß dies der Fall für die WW-Domänen-Variante K19C war, nicht jedoch für D8C. Die Ursache dafür kann in der sterischen Unzugänglichkeit des Cysteins der Variante D8C liegen. Zum Beweis der WW-Domänen-Spezifität dieser Verbrückung wurde in einem parallelen Ansatz die cysteinfreie Variante der WW-Domäne analysiert, die ebenfalls keine Verbrückung zeigte. Figur 9 (b) zeigt, daß sich die kovalente Wechselwirkung der WW-Domänen-Variante K19C und prolinreichem Peptid durch Zugabe eines Reduktionsmittels (50 mM DTT) wieder aufheben läßt. Das fluoreszierende Peptid liegt danach wieder vollständig in freier Form vor.

Zusammenfassend läßt sich sagen, daß die Einführung von Cystein-Aminosäureresten in die WW-Domäne die kovalente Verbrückung von polyprolinreichen Liganden, die mindestens ein Cystein tragen, mit der WW-Domäne ermöglichen und so zu einer stabilen kovalenten Verknüpfung von WW-Domäne und Ligand führen (siehe Figur 9).

### Beispiel 8

### Reinigung von Proteinen mittels Adaptersegmenten (Polyprolin/WW-Affinitätschromatographie)

Ein weiteres Anwendungsgebiet der vorliegenden Erfindung ist die Abtrennung von molekularen Substanzen aus Substanzgemischen, wie sie typischerweise bei der Reinigung von Proteinen aus Rohextrakten (Zellextrakten) auftritt. Dabei wird die Affinität der WW-Domäne zu prolinreichen Liganden ausgenutzt, um Proteine, die eine WW-Domäne enthalten, aus einem komplexen Gemisch (Rohextrakt) von Proteinen zu isolieren (Prinzip der Affinitätschromatographie). Dazu wird eine Säule gemäß dem Beispiel 3 verwendet; das SulfoLink-Material (Fa. Pierce, die Reaktivität der Matrix mit SH-Gruppen beruht auf der Iodacetamid-Gruppe am Ende eines Linkers, bestehend aus 10 CH₂-Gruppen) wird dabei über eine Thiolkopplung mit dem Peptid Cys-Ser-Gly-Pro-Pro-Pro-Pro-Pro-Pro-Pro-Pro-Leu-Pro gemäß den Herstellerangaben beladen.

Analog dazu ist auch die Kopplung des Peptids auf andere Matrices möglich, beispielsweise AffiGel 10 (Fa. Biorad, die Reaktivität der Matrix mit NH₂-Gruppen beruht auf der N-Hydroxysuccinimid-Gruppe am Ende eines Linkers, bestehend aus 10 CH₂-Gruppen) über den N-Terminus des Peptids. Ebenso kann die Peptidkopplung oder den N-Terminus des Peptids an eine Matrix, basierend auf CH-Sepharose 4B, erfolgen (Fa. Sigma, die reaktive Gruppe der Matrix ist ebenfalls ein N-Hydroxysuccinimidester). Auch hier resultiert eine kovalente Bindung des prolinreichen Liganden an ein Trägermaterial, das nachfolgend eine Reinigung von WW-Domänen-Proteinen erlaubt.

Die PyVPI-Variante PyVP1-3C-WW1 aus Beispiel 5 (WW-Domäne am N-Terminus des PyVP1-Proteins) wird analog den Angaben aus Beispiel 1 als Fusionsprotein mit einem Intein und einer chitin-bindenden Domäne hergestellt ([PyVP1-3C-WW1]-Intein-CBD). Sie wird jedoch nicht nach dem beschriebenen Standardverfahren mittels Chitin-Affinitätschromatographie gereinigt. Stattdessen wird der Zellextrakt nach Zellaufschluß und anschließender Zentrifugation auf die vorstehend beschriebene Säule aufgetragen. Als Laufpuffer dient dabei 10 mM HEPES, 150 mM NaCl, 1 mM EDTA, 5 % Glycerin, pH 8.0. Nach dem Auftragen des Extraktes wird mit 10 Säulenvolumina eines Puffers, der 10 mM HEPES (pH 8.0), 1 mM EDTA, 5 % Glycerin, und zusätzlich 2 M NaCl enthält, gewaschen. Bei diesem Waschvorgang werden alle unspezifisch adsorbierten Proteine und Zellbestandteile von der SulfoLink-Matrix entfernt. Anschließend erfolgt die Elution des gebundenen [PyVP1-3C-WW1]-Intein-CBD-Fusionsproteins mit einem Puffer, der 2 % SDS enthält. Wie anhand der Fig. 8 zu erkennen ist, erfolgt eine Bindung des Fusionsproteins [PyVP1-3C-WW1]-Intein-CBD über die Interaktion der WW-Domäne an das immobilisierte prolinreiche Peptid an der SulfoLink-Matrix. Dadurch wird das Fusionsprotein an die Matrix gebunden und somit die meisten anderen Proteine des Zellextrakts im Durchlauf oder während des Waschvorganges abgetrennt. Die Elution mit SDS liefert anschließend praktisch ausschließlich das vollständige WW-Domänen-haltige Fusionsprotein, sowie proteolytische Abbauprodukte hiervon (die im Falle des PyVP1 bei allen vergleichbaren Herstellungsverfahren nach dem Stand der Technik auftreten) und molekulare Chaperone, die bekanntermaßen direkt an PyVP 1 zu binden vermögen und üblicherweise nicht abgetrennt werden können. Anstatt der Elution des gebundenen WW-Domänenprotein mittels SDS ist auch eine Elution des nativen Proteins mit 300 mM Arginin im Laufpuffer möglich. Bei nachfolgender Dialyse des Eluats zur Entfernung des Arginins erhält man das gereinigte, native Protein.
Zusammenfassend zeigt dieses Beispiel, daß es möglich ist, mit dem beschriebenen System spezifisch Moleküle aus einem Substanzgemisch (Rohextrakt) abzutrennen und damit aufzureinigen.

### Beispiel 9

### Spezifische Dimerisierung von Molekülen über Adaptersegmente

Anhand der Wechselwirkung der Adaptersegmente (WW-Domäne und prolinreiches Peptid) kann auch die Herstellung von bifunktionellen oder bivalenten hybriden Molekülen *in vitro* erfolgen. Dazu werden zwei molekulare Substanzen hergestellt, die je nach Anwendungsfall gleiche oder verschiedene Eigenschaften haben können, und die jeweils eines der beiden Adaptersegmente kovalent verknüpft tragen. Im gewählten Beispiel erfolgt die Herstellung von einfach nachweisbaren Dimeren des GFP-Proteins.

Dazu wird eine Variante von GFP analog zur Herstellung von PyVP1 mit Hilfe des inteinbasierten Expressionssystems aus Beispiel 1 mit einer WW-Domäne am N-Terminus des GFP hergestellt (GFP-WW1). Zunächst wird eine PCR auf den Vektor pEGFP-N1 (Fa. Clontech, vgl. Beispiel 5) durchgeführt mit den Oligonukleotiden 5'-TAT AGC TAG CGT GAG CAA GGG CGA GGA GCT GTT C-3' und 5'-GGG AAT TAA GTA CAG CTC GTC CAT GCC G-3'. Das PCR-Produkt wird über die Schnittstellen Nhe I ― Sma I in den Vektor pET21a aus Beispiel 5 ligiert, der am 3'-Ende der Insertionsstelle das in Beispiel 1 beschriebene Fusionsprotein aus Intein und Chitin-Bindungsdomäne (CBD) enthält. Auf der 5'-Seite der Insertionsstelle befindet sich die WW-Domäne, die in Beispiel 5 beschrieben ist. Damit entsteht ein Fusionsprodukt in der Reihenfolge WW-Domäne - GFP - Intein - CBD. Das Plasmid, welches das Fusionsprotein codiert, kann in den Stamm *E*. *coli* BL21(DE3) transformiert werden. Analog zu den Beispielen 1 und 3 kann anschließend die Herstellung und Reinigung des Fusionsproteins erfolgen. Mit dem beschriebenen Verfahren kann das Protein GFP-WWI in gereinigter Form hergestellt werden.

Eine zweite Variante von GFP wird analog dazu mit einem prolinreichen Segment (Pro-Pro-Pro-Pro-Pro-Pro-Pro-Pro-Leu-Pro) am C-Terminus produziert. Die Herstellung und Reinigung des GFP-PLP-Proteins erfolgt dabei identisch zu der Beschreibung des Proteins im Beispiel 5.

Die beiden GFP-Varianten werden anschließend zusammen inkubiert. Dabei werden die beiden Proteine über die Adaptersegmente miteinander in Verbindung gebracht und es resultiert ein GFP-Dimer, das über Gelfiltration an einer TSK-PW2000XL-Gelfiltrationssäule (Fa. TosHaas) von dem GFP-Monomer unterschieden werden kann.

Das Beispiel zeigt, daß mit Hilfe des in der vorliegenden Erfindung beschriebenen Verfahrens eine Verbindung von beliebigen molekularen Substanzen erfolgen kann, die entsprechende Adaptersegmente auf der Basis von WW-Domänen bzw. prolinreichen Peptidsegmenten tragen. Dabei können homofunktionelle oder heterofunktionelle Assoziate entstehen.

## Patentansprüche

1. Verfahren zur Verbindung von zwei oder mehr molekularen Substanzen miteinander über Adaptersegmente,
**dadurch gekennzeichnet,**
**daß** eine der molekularen Substanzen so modifiziert wird, daß sie als Adaptersegment in zumindest einem Bereich eine WW-Domäne oder eine davon abgeleitete Struktur aufweist,
eine andere molekulare Substanz so modifiziert wird, daß sie als Adaptersegment in zumindest einem Bereich eine prolinreiche Sequenz aufweist, die an die WW-Domäne oder eine davon abgeleitete Struktur bindet,
und die molekularen Substanzen durch die Zusammenlagerung von WW-Domäne oder davon abgeleiteten Strukturen und prolinreicher Sequenz miteinander in Wechselwirkung treten, um eine Bindung aneinander zu erreichen.

2. Verfahren nach Anspruch 1,
**dadurch gekennzeichnet,**
**daß** die zu verbindenden molekularen Substanzen Proteine oder Peptide, Peptid- oder Proteinkonjugate mit Kohlenhydrat-, Nukleinsäure- oder Lipidanteilen, DNA, RNA, Ribozyme, synthetische Nukleinsäuren wie beispielsweise Peptide Nucleic Acids, oder Hybride hiervon oder von Peptiden und Proteinen abgeleitete oder weitere damit konjugierte Substanzen oder Moleküle umfassen, in oder an die zur Zusammenlagerung eine WW-Domäne und eine prolinreiche Sequenz einführbar sind.

3. Verfahren nach Anspruch 1 oder 2,
**dadurch gekennzeichnet,**
**daß** die zu verbindenden molekularen Substanzen Antikörper, antikörperanaloge Substanzen, Enzyme, Strukturproteine, Kapsomere von Viren oder Phagen, Peptidantibiotika, isolierte strukturbildende, katalytische oder regulatorische Proteindomänen, Teile von Proteinen, Peptide, Peptidanaloga, antigentragende Substanzen, Glykoproteine, Lipoproteine, Proteoglykane, oder Kombinationen aus genannten Substanzen umfassen, in oder an die zur Zusammenlagerung eine WW-Domäne und eine prolinreiche Sequenz einführbar sind.

4. Verfahren nach einem oder mehreren der vorhergehenden Ansprüche,.
**dadurch gekennzeichnet,**
**daß** eine der molekularen Substanzen ein Festphasenmatrixmolekül ist.

5. Verfahren nach einem oder mehreren der vorhergehenden Ansprüche,
**dadurch gekennzeichnet,**
**daß** sich die WW-Domäne in einer Loop-Region einer Proteinstruktur oder am C- oder N-Terminus einer Protein- oder Peptidstruktur befindet.

6. Verfahren nach einem oder mehreren der vorhergehenden Ansprüche,
**dadurch gekennzeichnet,**
**daß** sich die prolinreiche Sequenz in einer Loop-Region einer Proteinstruktur oder am C- oder N-Terminus einer Protein- oder Peptidstruktur befindet.

7. Verfahren nach einem oder mehreren der vorhergehenden Ansprüche,
**dadurch gekennzeichnet,**
**daß** als eine der molekularen Substanzen ein Virus-Kapsomer oder ein Phagen-Kapsomer eingesetzt wird, das eine WW-Domäne oder prolinreiche Sequenz in einem solchen Bereich des Kapsomers aufweist, daß sich die andere molekulare Substanz, nach Bindung oder Anlagerung an die erste molekulare Substanz und Assemblierung mit weiteren Kapsomeren zu einem Virus-Kapsid oder Phagen-Kapsid, im Innern des Virus-Kapsids oder Phagen-Kapsids befindet.

8. Verfahren nach einem oder mehreren der vorhergehenden Ansprüche,
**dadurch gekennzeichnet,**
**daß** als eine der molekularen Substanzen ein Virus-Kapsomer oder ein Phagen-Kapsomer eingesetzt wird, das eine WW-Domäne oder prolinreiche Sequenz in einem solchen Bereich des Kapsomers aufweist, daß sich diese nach Assemblierung mit weiteren Kapsomeren zu einem Virus-Kapsid oder Phagen-Kapsid auf der Außenseite des Kapsids befindet.

9. Verfahren nach einem oder mehreren der vorhergehenden Ansprüche,
**dadurch gekennzeichnet,**
**daß** zusätzlich zur Verbindung über die Adaptersegmente aufgrund der Wechselwirkung zwischen WW-Domäne und prolinreicher Sequenz eine kovalente Bindung der molekularen Substanzen miteinander erfolgt.

10. Verfahren nach einem oder mehreren der vorhergehenden Ansprüche,
**dadurch gekennzeichnet,**
**daß** durch die spezifische Einführung von einem oder mehreren Cysteinen im Bereich der WW-Domäne oder einer davon abgeleiteten Struktur, und die spezifische Einführung von einem oder mehreren Cysteinen im Bereich der prolinreichen Seqenz eine kovalente Verbindung der molekularen Substanzen erfolgt.

11. Verfahren nach einem oder mehreren der vorhergehenden Ansprüche,
**dadurch gekennzeichnet,**
**daß** die Verbindung der molekularen Substanzen irreversibel oder reversibel erfolgt.

12. Verfahren nach einem oder mehreren der vorhergehenden Ansprüche,
**dadurch gekennzeichnet,**
**daß** die WW-Domäne und die prolinreiche Sequenz kovalent oder nichtkovalent mit den molekularen Substanzen verbunden sind.

13. Verfahren nach einem oder mehreren der vorhergehenden Ansprüche,
**dadurch gekennzeichnet,**
**daß** zumindest eine der molekularen Substanzen und/oder die bindenden Bereiche synthetisch hergestellt werden.

14. Verfahren nach einem oder mehreren der vorhergehenden Ansprüche,
**dadurch gekennzeichnet,**
**daß** als von der WW-Domäne abgeleitete Struktur eine Variante eingesetzt wird, die gegenüber natürlichen WW-Domänen an einzelnen Aminosäurepositionen verändert, verkürzt oder verlängert ist, oder die ein Cystein an einer räumlich geeigneten Position enthält, oder die mehrere WW-Domänen aneinandergereiht enthält.

15. Verfahren nach einem oder mehreren der vorhergehenden Ansprüche,
**dadurch gekennzeichnet,**
**daß** als Virus-Kapsomer oder Phagen-Kapsomer rekombinant hergestellte modifizierte oder nicht modifizierte Monomer-, Dimer- oder Oligomereinheiten aus Virus- oder Phagenkapsiden eingesetzt werden.

## Claims

1. Procedure for linking of two or more molecular substances with each other through adapter segments,
**characterized by** the fact,
that one of the molecular substances is modified in such a way that it exhibits as an adapter segment, in at least one region, a WW domain or a structure derived therefrom, another molecular substance is modified in such a way that it exhibits as an adapter segment, in at least one region, a proline-rich sequence, which binds to the WW domain or a structure derived therefrom, and the molecular substances establish an interaction with each other through the connection of WW domain or structures derived therefrom with proline-rich sequence, in order to achieve binding to one another.

2. Procedure according to claim 1,
**characterized by** the fact,
that the molecular substances to be joined comprise proteins or peptides, peptide or protein conjugates with carbohydrate-, nucleic acid- or lipid-content, DNA, RNA, ribozymes, synthetic nucleic acids such as for example peptide nucleic acids, or hybrids thereof or of substances or molecules derived from peptides and proteins or conjugated with them, in or on which a WW domain and a proline-rich sequence are integratable for an assembly.

3. Procedure according to claim 1 or 2,
**characterized by** the fact,
that the molecular substances to be joined comprise antibodies, substances analogous to antibodies, enzymes, structural proteins, capsomeres of viruses or phages, peptide antibiotics, isolated structure-forming, catalytic or regulatory protein domains, fragments of proteins, peptides, peptide analogs, antigen-bearing substances, glycoproteins, lipoproteins, proteoglycans, or combinations of the named substances, in or on which a WW domain and a proline-rich sequence are integratable for an assembly.

4. Procedure according to one or more of the preceding claims,
**characterized by** the fact,
that one of the molecular substances is a fixed phase matrix molecule.

5. Procedure according to one or more of the preceding claims,
**characterized by** the fact,
that the WW domain is found in a loop region of a protein structure or at the C- or N-terminus of a protein- or peptide-structure.

6. Procedure according to one or more of the preceding claims,
**characterized by** the fact,
that the proline-rich sequence is found in a loop region of a protein structure or at the C- or N-terminus of a protein- or peptide-structure.

7. Procedure according to one or more of the preceding claims,
**characterized by** the fact,
that as one of the molecular substances a virus capsomer or phage capsomer is used which exhibits a WW domain or proline-rich sequence in such an area of the capsomer, that the other molecular substance, after binding or association to the first molecular substance and assembly with further capsomeres to a virus capsid or phage capsid, is found in the interior of the virus capsid or phage capsid.

8. Procedure according to one or more of the preceding claims,
**characterized by** the fact,
that as one of the molecular substances a virus capsomer or phage capsomer is used which exhibits a WW domain or proline-rich sequence in such an area of the capsomer, that after assembly with further capsomeres to a virus capsid or phage capsid, it is found on the outside of the capsid.

9. Procedure according to one or more of the preceding claims,
**characterized by** the fact,
that in addition to the connection between the adapter segments a covalent bond between the molecular substances is formed due to the interaction between WW domain and proline-rich sequence.

10. Procedure according to one or more of the preceding claims,
**characterized by** the fact,
that by the specific introduction of one or several cysteines in the region of the WW domain or of a structure derived therefrom, and the specific introduction of one or more cysteines in the region of the proline-rich sequence, a covalent link of the molecular substances results.

11. Procedure according to one or more of the preceding claims,
**characterized by** the fact,
that the connection of the molecular substances takes place irreversibly or reversibly.

12. Procedure according to one or more of the preceding claims,
**characterized by** the fact,
that the WW domain and the proline-rich sequence are joined covalently or non-covalently with the molecular substances.

13. Procedure according to one or more of the preceding claims,
**characterized by** the fact,
that at least one of the molecular substances and/or the binding areas are manufactured synthetically.

14. Procedure according to one or more of the preceding claims,
**characterized by** the fact,
that as the structure derived from the WW domain, a variant is utilized which, compared to natural WW domains, is shortened, extended or altered at individual amino acid positions, or which contains a cysteine at a spatially suitable position, or which contains several WW domains in tandem.

15. Procedure according to one or more of the preceding claims,
**characterized by** the fact,
that as virus capsomer or phage capsomer recombinantly produced, modified or non-modified monomer-, dimer- or oligomer-components from virus or phage capsids are utilized.

## Revendications

1. Procédé pour lier deux substances moléculaires ou plus, l'une avec l'autre, par des segments adaptateurs,
**caractérisé en ce qu'**une des substances moléculaires est modifiée de sorte qu'elle présente comme segment adaptateur, au moins dans une zone, un domaine WW ou une structure dérivée de celui-ci,
**en ce qu'**une autre substance moléculaire est modifiée de sorte qu'elle présente comme segment adaptateur, au moins dans une zone, une séquence riche en proline, qui se lie au domaine WW ou à une structure dérivée de celui-ci,
et **en ce que** les substances moléculaires entrent en interaction l'une avec l'autre par l'association du domaine WW ou de la structure dérivée de celui-ci et de la séquence riche en proline, pour former une liaison l'un avec l'autre.

2. Procédé selon la revendication 1,
**caractérisé en ce que**
les substances moléculaires à relier comprennent une protéine ou un peptide, un conjugué de peptide ou de protéine avec un radical glucidique, un radical acide nucléique ou un radical lipidique, un ADN, un ARN, un ribozyme, des acides nucléiques synthétiques comme par exemple des acides nucléiques peptidiques ou des hybrides de ceux-ci ou des substances ou molécules dérivées de peptides et de protéines ou d'autres conjuguées, dans ou sur lesquels un domaine WW ou une séquence riche en proline peuvent être introduits pour l'association.

3. Procédé selon la revendication 1 ou 2,
**caractérisé en ce que**
les substances moléculaires à relier comprennent des anticorps, des substances analogues à des anticorps, des enzymes, des protéines de structure, des capsomères de virus ou de phages, des antibiotiques peptidiques, des domaines protéiques isolés, formant structure, catalytiques ou régulateurs, des parties de protéines, de peptides, des analogues peptidiques, des substances portant un antigène, des glycoprotéines, des lipoprotéines, des protéoglycanes ou des combinaisons des substances citées, dans ou sur lesquels un domaine WW et une séquence riche en proline peuvent être introduits pour l'association.

4. Procédé selon une ou plusieurs des revendications précédentes,
**caractérisé en ce qu'**une des substances moléculaires est une molécule de matrice en phase solide.

5. Procédé selon une ou plusieurs des revendications précédentes,
**caractérisé en ce que**
le domaine WW se trouve dans une région de boucle d'une structure protéique ou sur l'extrémité C ou N d'une structure protéique ou peptidique.

6. Procédé selon une ou plusieurs des revendications précédentes,
**caractérisé en ce que**
la séquence riche en proline se trouve dans une région de boucle d'une structure protéique ou sur l'extrémité C ou N d'une structure protéique ou peptidique.

7. Procédé selon une ou plusieurs des revendications précédentes,
**caractérisé en ce que**
l'on met en oeuvre comme une des substances moléculaires, un capsomère de virus ou un capsomère de phage, qui présente un domaine WW ou une séquence riche en proline dans une zone du capsomère telle que l'autre substance moléculaire se trouve après liaison ou fixation sur la première substance moléculaire et assemblage avec d'autres capsomères en un capside de virus ou un capside de phage, à l'intérieur du capside de virus ou de capside de phage.

8. Procédé selon une ou plusieurs des revendications précédentes,
**caractérisé en ce que**
l'on met en oeuvre comme une des substances moléculaires, un capsomère de virus ou un capsomère de phage, qui présente un domaine WW ou une séquence riche en proline dans une zone du capsomère telle que celui-ci se trouve après assemblage avec d'autres capsomères en un capside de virus ou un capside de phage, sur la face extérieure du capside.

9. Procédé selon une ou plusieurs des revendications précédentes,
**caractérisé en ce qu'**en plus de la liaison par le segment adaptateur, sur base de l'interaction entre le domaine WW et la séquence riche en proline, une liaison covalente des substances moléculaires l'une avec l'autre est réalisée.

10. Procédé selon une ou plusieurs des revendications précédentes,
**caractérisé en ce que**
par l'introduction spécifique d'une ou de plusieurs cystéines dans la zone du domaine WW ou d'une structure dérivée de celui-ci, et par l'introduction spécifique d'une ou de plusieurs cystéines dans la zone de la séquence riche en proline, on réalise une liaison covalente des substances moléculaires.

11. Procédé selon une ou plusieurs des revendications précédentes,
**caractérisé en ce que**
la liaison des substances moléculaires est réalisée de manière irréversible ou réversible.

12. Procédé selon une ou plusieurs des revendications précédentes,
**caractérisé en ce que**
le domaine WW et la séquence riche en proline sont reliées de manière covalente ou non covalente avec les substances moléculaires.

13. Procédé selon une ou plusieurs des revendications précédentes,
**caractérisé en ce qu'**au moins une des substances moléculaires et/ou les domaines de liaison sont préparés de manière synthétique.

14. Procédé selon une ou plusieurs des revendications précédentes,
**caractérisé en ce que**
l'on met en oeuvre, comme structure dérivée du domaine WW, un variant, qui est modifié, raccourci ou allongé par rapport au domaine WW naturel, en certaines positions des acides aminés, ou qui contient une cystéine en une position spatialement appropriée, ou qui contient plusieurs domaines WW successivement.

15. Procédé selon une ou plusieurs des revendications précédentes,
**caractérisé en ce que**
comme capsomère de virus ou capsomère de phage, on met en oeuvre des unités monomère, dimères ou oligomères modifiées ou non modifiées, préparées par recombinaison de capsides de virus ou de phage.


### SEQUENZPROTOKOLL

<110> ACGT ProGenomics AG
<120> Verfahren zur Verbindung von molekularen Substanzen
<130> P12604
<140>
   <141>
<150> DE - 199 52 956.6
   <151> 1999-11-03
<160> 21
<170> PatentIn Ver. 2.1
<210> 1
   <211> 1266
   <212> DNA
   <213> Künstliche Sequenz
<220>
   <221> CDS
   <222> (1)..(1266)
<220>
   <221> misc_feature
   <222> (445)..(558)
   <223> inserierte WW-Domäne (WW150)
<220>
   <221> misc_feature
   <222> (481)..(483)
   <223> eingeführtes Cystein in der WW-Domäne
<220>
   <223> Beschreibung der künstlichen Sequenz: Cystein-Variante von VP1-WW150 mit Aminosäuresaustausch D8C an Position 8 der WW-Domäne (PyVP1-WW150-D8C)
<400> 1
   atg gcc ccc aaa aga aaa agc ggc gtc tct aaa agc gag aca aaa agc   48
   Met Ala Pro Lys Arg Lys Ser Gly Val Ser Lys Ser Glu Thr Lys Ser
     1               5                  10                  15
   aca aag gct agc cca aga ccc gca ccc gtt ccc aaa ctg ctt att aaa    96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca   144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct   192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att   240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt   288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac   336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc   384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa   432
   Glu Val Val Gly Ser Gly Ser Leu Leu Asp Val His Gly Phe Asn Lys
       130                 135                 140
   ccc aca gat aca ggc agc ggc agc ggc tgg aca gaa cat aaa tca cct   480
   Pro Thr Asp Thr Gly Ser Gly Ser Gly Trp Thr Glu His Lys Ser Pro
   145                 150                 155                 160
   tgt gga agg act tat tat tac aat act gaa aca aaa cag tct acc tgg   528
   Cys Gly Arg Thr Tyr Tyr Tyr Asn Thr Glu Thr Lys Gln Ser Thr Trp
                   165                 170                 175
   gaa aag cca gat gat ggt agt ggt agc ggc gta aac aca aaa gga att   576
   Glu Lys Pro Asp Asp Gly Ser Gly Ser Gly Val Asn Thr Lys Gly Ile
               180                 185                 190
   tcc act cca gtg gaa ggc agc caa tat cat gtg ttt gct gtg ggc ggg   624
   Ser Thr Pro Val Glu Gly Ser Gln Tyr His Val Phe Ala Val Gly Gly
           195                 200                 205
   gaa ccg ctt gac ctc cag gga ctt gtg aca gat gcc aga aca aaa tac      672
   Glu Pro Leu Asp Leu Gln Gly Leu Val Thr Asp Ala Arg Thr Lys Tyr
       210                 215                 220
   aag gaa gaa ggg gta gta aca atc aaa aca atc aca aag aag gac atg      720
   Lys Glu Glu Gly Val Val Thr Ile Lys Thr Ile Thr Lys Lys Asp Met
   225                 230                 235                 240
   gtc aac aaa gac caa gtc ctg aat cca att agc aag gcc aag ctg gat      768
   Val Asn Lys Asp Gln Val Leu Asn Pro Ile Ser Lys Ala Lys Leu Asp
                   245                 250                 255
   aag gac gga atg tat cca gtt gaa atc tgg cat cca gat cca gca aaa      816
   Lys Asp Gly Met Tyr Pro Val Glu Ile Trp His Pro Asp Pro Ala Lys
               260                 265                 270
   aat gag aac aca agg tac ttt ggc aat tac act gga ggc acg tgc acc      864
   Asn Glu Asn Thr Arg Tyr Phe Gly Asn Tyr Thr Gly Gly Thr Cys Thr
           275                 280                 285
   cca ccc gtc ctg cag ttc aca aac acc ctg aca act gtg ctc cta gat      912
   Pro Pro Val Leu Gln Phe Thr Asn Thr Leu Thr Thr Val Leu Leu Asp
       290                 295                 300
   gaa aat gga gtt ggg ccc ctc agc aaa gga gaa ggt cta tac ctc tcg      960
   Glu Asn Gly Val Gly Pro Leu Ser Lys Gly Glu Gly Leu Tyr Leu Ser
   305                 310                 315                 320
   agc gta gat ata atg ggc tgg aga gtt aca aga aac tat gat gtc cat     1008
   Ser Val Asp Ile Met Gly Trp Arg Val Thr Arg Asn Tyr Asp Val His
                   325                 330                 335
   cac tgg aga ggg ctt ccc aga tat ttc aaa atc acc ctg aga aaa aga     1056
   His Trp Arg Gly Leu Pro Arg Tyr Phe Lys Ile Thr Leu Arg Lys Arg
               340                 345                 350
   tgg gtc aaa aat ccc tat ccc atg gcc tcc ctc ata agt tcc ctt ttc     1104
   Trp Val Lys Asn Pro Tyr Pro Met Ala Ser Leu Ile Ser Ser Leu Phe
           355                 360                 365
   aac aac atg ctc ccc caa gtg cag ggc caa ccc atg gaa ggg gag aac     1152
   Asn Asn Met Leu Pro Gln Val Gln Gly Gln Pro Met Glu Gly Glu Asn
       370                 375                 380
   acc cag gta gag gag gtt aga gtg tat gat ggg act gaa cct gta ccg     1200
   Thr Gln Val Glu Glu Val Arg Val Tyr Asp Gly Thr Glu Pro Val Pro
   385                 390                 395                 400
   ggg gac cct gat atg acg cgc tat gtt gac cgc ttt gga aaa aca aag    1248
   Gly Asp Pro Asp Met Thr Arg Tyr Val Asp Arg Phe Gly Lys Thr Lys
                   405                 410                 415
   act gta ttt cct ccc ggg                                            1266
   Thr Val Phe Pro Pro Gly
               420
<210> 2
   <211> 422
   <212> PRT
   <213> Künstliche Sequenz
   <223> Beschreibung der künstlichen Sequenz: Cystein-Variante von VP1-WW150 mit Aminosäuresaustausch D8C an Position 8 der WW-Domäne (PyVP1-WW150-D8C)
<400> 2
   Met Ala Pro Lys Arg Lys Ser Gly Val Ser Lys Ser Glu Thr Lys Ser
   1               5                   10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Ser Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Gly Ser Gly Ser Gly Trp Thr Glu His Lys Ser Pro
   145                 150                 155                 160
   Cys Gly Arg Thr Tyr Tyr Tyr Asn Thr Glu Thr Lys Gln Ser Thr Trp
   165 170 175
   Glu Lys Pro Asp Asp Gly Ser Gly Ser Gly Val Asn Thr Lys Gly Ile
   180 185 190
   Ser Thr Pro Val Glu Gly Ser Gln Tyr His Val Phe Ala Val Gly Gly
   195 200 205
   Glu Pro Leu Asp Leu Gln Gly Leu Val Thr Asp Ala Arg Thr Lys Tyr
   210 215 220
   Lys Glu Glu Gly Val Val Thr Ile Lys Thr Ile Thr Lys Lys Asp Met
   225 230 235 240
   Val Asn Lys Asp Gln Val Leu Asn Pro Ile Ser Lys Ala Lys Leu Asp
   245 250 255
   Lys Asp Gly Met Tyr Pro Val Glu Ile Trp His Pro Asp Pro Ala Lys
   260 265 270
   Asn Glu Asn Thr Arg Tyr Phe Gly Asn Tyr Thr Gly Gly Thr Cys Thr
   275 280 285
   Pro Pro Val Leu Gln Phe Thr Asn Thr Leu Thr Thr Val Leu Leu Asp
   290 295 300
   Glu Asn Gly Val Gly Pro Leu Ser Lys Gly Glu Gly Leu Tyr Leu Ser
   305 310 315 320
   Ser Val Asp Ile Met Gly Trp Arg Val Thr Arg Asn Tyr Asp Val His
   325 330 335
   His Trp Arg Gly Leu Pro Arg Tyr Phe Lys Ile Thr Leu Arg Lys Arg
   340 345 350
   Trp Val Lys Asn Pro Tyr Pro Met Ala Ser Leu Ile Ser Ser Leu Phe
   355 360 365
   Asn Asn Met Leu Pro Gln Val Gln Gly Gln Pro Met Glu Gly Glu Asn
   370 375 380
   Thr Gln Val Glu Glu Val Arg Val Tyr Asp Gly Thr Glu Pro Val Pro
   385 390 395 400
   Gly Asp Pro Asp Met Thr Arg Tyr Val Asp Arg Phe Gly Lys Thr Lys
   405 410 415
   Thr Val Phe Pro Pro Gly
   420
<210> 3
   <211> 1266
   <212> DNA
   <213> Künstliche Sequenz
<220>
   <223> Beschreibung der künstlichen Sequenz: PyVP1-WW150, inserierte WW-Domäne aus FBP11
<220>
   <221> CDS
   <222> (1)..(1266)
<220>
   <221> misc feature
   <222> (445)..(558)
   <223> WW-Domaene
<400> 3
   atg gcc ccc aaa aga aaa agc ggc gtc tct aaa agc gag aca aaa agc     48
   Met Ala Pro Lys Arg Lys Ser Gly Val Ser Lys Ser Glu Thr Lys Ser
     1               5                  10                  15
   aca aag gct agc cca aga ccc gca ccc gtt ccc aaa ctg ctt att aaa     96
   Thr Lys Ala Ser Pro Arg Pro Ala Pro Val Pro Lys Leu Leu Ile Lys
                20                  25                  30
   ggg ggt atg gag gtg ctg gac ctt gtg aca ggg cca gac agt gtg aca    144
   Gly Gly Met Glu Val Leu Asp Leu Val Thr Gly Pro Asp Ser Val Thr
            35                  40                  45
   gaa ata gaa gct ttt ctg aac ccc aga atg ggg cag cca ccc acc cct    192
   Glu Ile Glu Ala Phe Leu Asn Pro Arg Met Gly Gln Pro Pro Thr Pro
        50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
    65                  70                  75                  80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                    85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc    384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca ggc agc ggc agc ggc tgg aca gaa cat aaa tca cct    480
   Pro Thr Asp Thr Gly Ser Gly Ser Gly Trp Thr Glu His Lys Ser Pro
   145                 150                 155                 160
   gat gga agg act tat tat tac aat act gaa aca aaa cag tct acc tgg    528
   Asp Gly Arg Thr Tyr Tyr Tyr Asn Thr Glu Thr Lys Gln Ser Thr Trp
                   165                 170                 175
   gaa aag cca gat gat ggt agt ggt agc ggc gta aac aca aaa gga att    576
   Glu Lys Pro Asp Asp Gly Ser Gly Ser Gly Val Asn Thr Lys Gly Ile
               180                 185                 190
   tcc act cca gtg gaa ggc agc caa tat cat gtg ttt gct gtg ggc ggg    624
   Ser Thr Pro Val Glu Gly Ser Gln Tyr His Val Phe Ala Val Gly Gly
           195                 200                 205
   gaa ccg ctt gac ctc cag gga ctt gtg aca gat gcc aga aca aaa tac    672
   Glu Pro Leu Asp Leu Gln Gly Leu Val Thr Asp Ala Arg Thr Lys Tyr
       210                 215                 220
   aag gaa gaa ggg gta gta aca atc aaa aca atc aca aag aag gac atg    720
   Lys Glu Glu Gly Val Val Thr Ile Lys Thr Ile Thr Lys Lys Asp Met
   225                 230                 235                 240
   gtc aac aaa gac caa gtc ctg aat cca att agc aag gcc aag ctg gat    768
   Val Asn Lys Asp Gln Val Leu Asn Pro Ile Ser Lys Ala Lys Leu Asp
                   245                 250                 255
   aag gac gga atg tat cca gtt gaa atc tgg cat cca gat cca gca aaa    816
   Lys Asp Gly Met Tyr Pro Val Glu Ile Trp His Pro Asp Pro Ala Lys
               260                 265                 270
   aat gag aac aca agg tac ttt ggc aat tac act gga ggc acg tgc acc    864
   Asn Glu Asn Thr Arg Tyr Phe Gly Asn Tyr Thr Gly Gly Thr Cys Thr
           275                 280                 285
   cca ccc gtc ctg cag ttc aca aac acc ctg aca act gtg ctc cta gat 912
   Pro Pro Val Leu Gln Phe Thr Asn Thr Leu Thr Thr Val Leu Leu Asp
   290 295 300
   gaa aat gga gtt ggg ccc ctc agc aaa gga gaa ggt cta tac ctc tcg 960
   Glu Asn Gly Val Gly Pro Leu Ser Lys Gly Glu Gly Leu Tyr Leu Ser
   305 310 315 320
   agc gta gat ata atg ggc tgg aga gtt aca aga aac tat gat gtc cat 1008
   Ser Val Asp Ile Met Gly Trp Arg Val Thr Arg Asn Tyr Asp Val His
   325 330 335
   cac tgg aga ggg ctt ccc aga tat ttc aaa atc acc ctg aga aaa aga 1056
   His Trp Arg Gly Leu Pro Arg Tyr Phe Lys Ile Thr Leu Arg Lys Arg
   340 345 350
   tgg gtc aaa aat ccc tat ccc atg gcc tcc ctc ata agt tcc ctt ttc 1104
   Trp Val Lys Asn Pro Tyr Pro Met Ala Ser Leu Ile Ser Ser Leu Phe
   355 360 365
   aac aac atg ctc ccc caa gtg cag ggc caa ccc atg gaa ggg gag aac 1152
   Asn Asn Met Leu Pro Gln Val Gln Gly Gln Pro Met Glu Gly Glu Asn
   370 375 380
   acc cag gta gag gag gtt aga gtg tat gat ggg act gaa cct gta ccg 1200
   Thr Gln Val Glu Glu Val Arg Val Tyr Asp Gly Thr Glu Pro Val Pro
   385 390 395 400
   ggg gac cct gat atg acg cgc tat gtt gac cgc ttt gga aaa aca aag 1248
   Gly Asp Pro Asp Met Thr Arg Tyr Val Asp Arg Phe Gly Lys Thr Lys
   405 410 415
   act gta ttt cct ccc ggg 1266
   Thr Val Phe Pro Pro Gly
   420
<210> 4
   <211> 422
   <212> PRT
   <213> Künstliche Sequenz
   <223> Beschreibung der künstlichen Sequenz: PyVP1-ww150, inserierte WW-Domäne aus FBP11
<400> 4
   Met Ala Pro Lys Arg Lys Ser Gly Val Ser Lys Ser Glu Thr Lys Ser
   1 5 10 15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Ser Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Gly Ser Gly Ser Gly Trp Thr Glu His Lys Ser Pro
   145                 150                 155                 160
   Asp Gly Arg Thr Tyr Tyr Tyr Asn Thr Glu Thr Lys Gln Ser Thr Trp
                   165                 170                 175
   Glu Lys Pro Asp Asp Gly Ser Gly Ser Gly Val Asn Thr Lys Gly Ile
               180                 185                 190
   Ser Thr Pro Val Glu Gly Ser Gln Tyr His Val Phe Ala Val Gly Gly
           195                 200                 205
   Glu Pro Leu Asp Leu Gln Gly Leu Val Thr Asp Ala Arg Thr Lys Tyr
       210                 215                 220
   Lys Glu Glu Gly Val Val Thr Ile Lys Thr Ile Thr Lys Lys Asp Met
   225                 230                 235                 240
   Val Asn Lys Asp Gln Val Leu Asn Pro Ile Ser Lys Ala Lys Leu Asp
                   245                 250                 255
   Lys Asp Gly Met Tyr Pro Val Glu Ile Trp His Pro Asp Pro Ala Lys
               260                 265                 270
   Asn Glu Asn Thr Arg Tyr Phe Gly Asn Tyr Thr Gly Gly Thr Cys Thr
   275 280 285
   Pro Pro Val Leu Gln Phe Thr Asn Thr Leu Thr Thr Val Leu Leu Asp
   290 295 300
   Glu Asn Gly Val Gly Pro Leu Ser Lys Gly Glu Gly Leu Tyr Leu Ser
   305 310 315 320
   Ser Val Asp Ile Met Gly Trp Arg Val Thr Arg Asn Tyr Asp Val His
   325 330 335
   His Trp Arg Gly Leu Pro Arg Tyr Phe Lys Ile Thr Leu Arg Lys Arg
   340 345 350
   Trp Val Lys Asn Pro Tyr Pro Met Ala Ser Leu Ile Ser Ser Leu Phe
   355 360 365
   Asn Asn Met Leu Pro Gln Val Gln Gly Gln Pro Met Glu Gly Glu Asn
   370 375 380
   Thr Gln Val Glu Glu Val Arg Val Tyr Asp Gly Thr Glu Pro Val Pro
   385 390 395 400
   Gly Asp Pro Asp Met Thr Arg Tyr Val Asp Arg Phe Gly Lys Thr Lys
   405 410 415
   Thr Val Phe Pro Pro Gly
   420
<210> 5
   <211> 1251
   <212> DNA
   <213> Künstliche Sequenz
<220>
   <223> Beschreibung der künstlichen Sequenz: PyVP1-3C-WW1, N-terminale WW-Domäne
<220>
   <221> CDS
   <222> (1)..(1251)
<220>
   <221> misc_feature
   <222> (9)..(93)
   <223> WW-Domaene
<400> 5
   atg agc ggc tgg aca gaa cat aaa tca cct gat gga agg act tat tat    48
   Met Ser Gly Trp Thr Glu His Lys Ser Pro Asp Gly Arg Thr Tyr Tyr
     1               5                  10                  15
   tac aat act gaa aca aaa cag tct acc tgg gaa aag cca gat gat gga    96
   Tyr Asn Thr Glu Thr Lys Gln Ser Thr Trp Glu Lys Pro Asp Asp Gly
                20                  25                  30
   cat atg gcc ccc aaa aga aaa agc ggc gtc tct aaa tct gag aca aaa   144
   His Met Ala Pro Lys Arg Lys Ser Gly Val Ser Lys Ser Glu Thr Lys
            35                  40                  45
   agc aca aag gcc tgt cca aga ccc gca ccc gtt ccc aaa ctg ctt att   192
   Ser Thr Lys Ala Cys Pro Arg Pro Ala Pro Val Pro Lys Leu Leu Ile
        50                  55                  60
   aaa ggg ggt atg gag gtg ctg gac ctt gtg aca ggg cca gac agt gtg   240
   Lys Gly Gly Met Glu Val Leu Asp Leu Val Thr Gly Pro Asp Ser Val
    65                  70                  75                  80
   aca gaa ata gaa gct ttt ctg aac ccc aga atg ggg cag cca ccc acc   288
   Thr Glu Ile Glu Ala Phe Leu Asn Pro Arg Met Gly Gln Pro Pro Thr
                    85                  90                  95
   cct gaa agc cta aca gag gga ggg caa tac tat ggt tgg agc aga ggg   336
   Pro Glu Ser Leu Thr Glu Gly Gly Gln Tyr Tyr Gly Trp Ser Arg Gly
               100                 105                 110
   att aat ttg gct aca tca gat aca gag gat tcc cca gga aat aat aca   384
   Ile Asn Leu Ala Thr Ser Asp Thr Glu Asp Ser Pro Gly Asn Asn Thr
           115                 120                 125
   ctt ccc aca tgg agt atg gca aag ctc cag ctt ccc atg ctc aat gag   432
   Leu Pro Thr Trp Ser Met Ala Lys Leu Gln Leu Pro Met Leu Asn Glu
       130                 135                 140
   gac ctc acc tgt gac acc cta caa atg tgg gag gca gtc tca gtg aaa   480
   Asp Leu Thr Cys Asp Thr Leu Gln Met Trp Glu Ala Val Ser Val Lys
   145                 150                 155                 160
   acc gag gtg gtg ggc tct ggc tca ctg tta gat gtg cat ggg ttc aac   528
   Thr Glu Val Val Gly Ser Gly Ser Leu Leu Asp Val His Gly Phe Asn
                   165                 170                 175
   aaa ccc aca gat aca gta aac aca aaa gga att tcc act cca gtg gaa    576
   Lys Pro Thr Asp Thr Val Asn Thr Lys Gly Ile Ser Thr Pro Val Glu
               180                 185                 190
   ggc agc caa tat cat gtg ttt gct gtg ggc ggg gaa ccg ctt gac ctc    624
   Gly Ser Gln Tyr His Val Phe Ala Val Gly Gly Glu Pro Leu Asp Leu
           195                 200                 205
   cag gga ctt gtg aca gat gcc aga aca aaa tac aag gaa gaa ggg gta    672
   Gln Gly Leu Val Thr Asp Ala Arg Thr Lys Tyr Lys Glu Glu Gly Val
       210                 215                 220
   gta aca atc aaa aca atc aca aag aag gac atg gtc aac aaa gac caa    720
   Val Thr Ile Lys Thr Ile Thr Lys Lys Asp Met Val Asn Lys Asp Gln
   225                 230                 235                 240
   gtc ctg aat cca att agc aag gcc aag ctg gat aag gac gga atg tat    768
   Val Leu Asn Pro Ile Ser Lys Ala Lys Leu Asp Lys Asp Gly Met Tyr
                   245                 250                 255
   cca gtt gaa atc tgg cat cca gat cca gca aaa aat gag aac aca agg    816
   Pro Val Glu Ile Trp His Pro Asp Pro Ala Lys Asn Glu Asn Thr Arg
               260                 265                 270
   tac ttt ggc aat tac act gga ggc acg tgc act cca ccc gtc ctg cag    864
   Tyr Phe Gly Asn Tyr Thr Gly Gly Thr Cys Thr Pro Pro Val Leu Gln
           275                 280                 285
   ttc aca aac acc ctg aca act gtg ctc cta gat gaa aat gga gtt ggg    912
   Phe Thr Asn Thr Leu Thr Thr Val Leu Leu Asp Glu Asn Gly Val Gly
       290                 295                 300
   ccc ctc agc aaa gga gag ggc cta tac ctc tcg agc gta gat ata atg    960
   Pro Leu Ser Lys Gly Glu Gly Leu Tyr Leu Ser Ser Val Asp Ile Met
   305                 310                 315                 320
   ggc tgg aga gtt aca aga aac tat gat gtc cat cac tgg aga ggg ctt    1008
   Gly Trp Arg Val Thr Arg Asn Tyr Asp Val His His Trp Arg Gly Leu
                   325                 330                 335
   ccc aga tat ttc aaa atc acc ctg aga aaa aga tgg gtc aaa aat ccc    1056
   Pro Arg Tyr Phe Lys Ile Thr Leu Arg Lys Arg Trp Val Lys Asn Pro
               340                 345                 350
   tat ccc atg gcc tcc ctc ata agt tcc ctt ttc aac aac atg ctc ccc    1104
   Tyr Pro Met Ala Ser Leu Ile Ser Ser Leu Phe Asn Asn Met Leu Pro
           355                 360                 365
   caa gtg cag ggc caa ccc atg gaa ggg gag aac acc cag gta gag gag 1152
   Gln Val Gln Gly Gln Pro Met Glu Gly Glu Asn Thr Gln Val Glu Glu
   370 375 380
   gtt aga gtg tat gat ggg act gaa cct gta ccg ggg gac cct gat atg 1200
   Val Arg Val Tyr Asp Gly Thr Glu Pro Val Pro Gly Asp Pro Asp Met
   385 390 395 400
   acg cgc tat gtt gac cgc ttt gga aaa aca aag act gta ttt cct ccc 1248
   Thr Arg Tyr Val Asp Arg Phe Gly Lys Thr Lys Thr Val Phe Pro Pro
   405 410 415
   ggg 1251
   Gly
<210> 6
   <211> 417
   <212> PRT
   <213> Künstliche Sequenz
   <223> Beschreibung der künstlichen Sequenz: PyVP1-3C-WW1, N-terminale WW-Domäne
<400> 6
   Met Ser Gly Trp Thr Glu His Lys Ser Pro Asp Gly Arg Thr Tyr Tyr
   1 5 10 15
   Tyr Asn Thr Glu Thr Lys Gln Ser Thr Trp Glu Lys Pro Asp Asp Gly
   20 25 30
   His Met Ala Pro Lys Arg Lys Ser Gly Val Ser Lys Ser Glu Thr Lys
   35 40 45
   Ser Thr Lys Ala Cys Pro Arg Pro Ala Pro Val Pro Lys Leu Leu Ile
   50 55 60
   Lys Gly Gly Met Glu Val Leu Asp Leu Val Thr Gly Pro Asp Ser Val
   65 70 75 80
   Thr Glu Ile Glu Ala Phe Leu Asn Pro Arg Met Gly Gln Pro Pro Thr
   85 90 95
   Pro Glu Ser Leu Thr Glu Gly Gly Gln Tyr Tyr Gly Trp Ser Arg Gly
   100 105 110
   Ile Asn Leu Ala Thr Ser Asp Thr Glu Asp Ser Pro Gly Asn Asn Thr
   115 120 125
   Leu Pro Thr Trp Ser Met Ala Lys Leu Gln Leu Pro Met Leu Asn Glu
       130                 135                 140
   Asp Leu Thr Cys Asp Thr Leu Gln Met Trp Glu Ala Val Ser Val Lys
   145                 150                 155                 160
   Thr Glu Val Val Gly Ser Gly Ser Leu Leu Asp Val His Gly Phe Asn
                   165                 170                 175
   Lys Pro Thr Asp Thr Val Asn Thr Lys Gly Ile Ser Thr Pro Val Glu
               180                 185                 190
   Gly Ser Gln Tyr His Val Phe Ala Val Gly Gly Glu Pro Leu Asp Leu
           195                 200                 205
   Gln Gly Leu Val Thr Asp Ala Arg Thr Lys Tyr Lys Glu Glu Gly Val
       210                 215                 220
   Val Thr Ile Lys Thr Ile Thr Lys Lys Asp Met Val Asn Lys Asp Gln
   225                 230                 235                 240
   Val Leu Asn Pro Ile Ser Lys Ala Lys Leu Asp Lys Asp Gly Met Tyr
                   245                 250                 255
   Pro Val Glu Ile Trp His Pro Asp Pro Ala Lys Asn Glu Asn Thr Arg
               260                 265                 270
   Tyr Phe Gly Asn Tyr Thr Gly Gly Thr Cys Thr Pro Pro Val Leu Gln
           275                 280                 285
   Phe Thr Asn Thr Leu Thr Thr Val Leu Leu Asp Glu Asn Gly Val Gly
       290                 295                 300
   Pro Leu Ser Lys Gly Glu Gly Leu Tyr Leu Ser Ser Val Asp Ile Met
   305                 310                 315                 320
   Gly Trp Arg Val Thr Arg Asn Tyr Asp Val His His Trp Arg Gly Leu
                   325                 330                 335
   Pro Arg Tyr Phe Lys Ile Thr Leu Arg Lys Arg Trp Val Lys Asn Pro
               340                 345                 350
   Tyr Pro Met Ala Ser Leu Ile Ser Ser Leu Phe Asn Asn Met Leu Pro
           355                 360                 365
   Gln Val Gln Gly Gln Pro Met Glu Gly Glu Asn Thr Gln Val Glu Glu
       370                 375                 380
   Val Arg Val Tyr Asp Gly Thr Glu Pro Val Pro Gly Asp Pro Asp Met
   385                 390                 395                 400
   Thr Arg Tyr Val Asp Arg Phe Gly Lys Thr Lys Thr Val Phe Pro Pro
                   405                 410                 415
   Gly
<210> 7
   <211> 1179
   <212> DNA
   <213> Künstliche Sequenz
<220>
   <223> Beschreibung der künstlichen Sequenz: PyVP1-3C-[N-14]-PLP, prolinreiche Sequenz am verkürzten N-Terminus
<220>
   <221> CDS
   <222> (1)..(1179)
<220>
   <221> misc_feature
   <222> (4)..(33)
   <223> prolinreiche Sequenz
<400> 7
   atg ccg cca cct cca ccg cca cct ccg tta cca ggc cta ggc cgg cgt      48
   Met Pro Pro Pro Pro Pro Pro Pro Pro Leu Pro Gly Leu Gly Arg Arg
     1               5                  10                  15
   ggg cta gcg acg tcc cat ggc tta agt aca aag gct tgt cca aga ccc      96
   Gly Leu Ala Thr Ser His Gly Leu Ser Thr Lys Ala Cys Pro Arg Pro
               20                  25                  30
   gca ccc gtt ccc aaa ctg ctt att aaa ggg ggt atg gag gtg ctg gac     144
   Ala Pro Val Pro Lys Leu Leu Ile Lys Gly Gly Met Glu Val Leu Asp
           35                  40                  45
   ctt gtg aca ggg cca gac tgt gtg aca gaa ata gaa gct ttt ctg aac     192
   Leu Val Thr Gly Pro Asp Cys Val Thr Glu Ile Glu Ala Phe Leu Asn
       50                  55                  60
   ccc aga atg ggg cag cca ccc acc cct gaa agc cta aca gag gga ggg     240
   Pro Arg Met Gly Gln Pro Pro Thr Pro Glu Ser Leu Thr Glu Gly Gly
    65                 70                  75                  80
   caa tac tat ggt tgg agc aga ggg att aat ttg gct aca tca gat aca    288
   Gln Tyr Tyr Gly Trp Ser Arg Gly Ile Asn Leu Ala Thr Ser Asp Thr
                    85                  90                  95
   gag gat tcc cca gga aat aat aca ctt ccc aca tgg agt atg gca aag    336
   Glu Asp Ser Pro Gly Asn Asn Thr Leu Pro Thr Trp Ser Met Ala Lys
               100                 105                 110
   ctc cag ctt ccc atg ctc aat gag gac ctc acg tgt gac acc cta caa    384
   Leu Gln Leu Pro Met Leu Asn Glu Asp Leu Thr Cys Asp Thr Leu Gln
           115                 120                 125
   atg tgg gag gca gtc tca gtg aaa acc gag gtg gtg ggc tct ggc tca    432
   Met Trp Glu Ala Val Ser Val Lys Thr Glu Val Val Gly Ser Gly Ser
       130                 135                 140
   ctg tta gat gtg cat ggg ttc aac aaa ccc aca gat aca gta aac aca    480
   Leu Leu Asp Val His Gly Phe Asn Lys Pro Thr Asp Thr Val Asn Thr
   145                 150                 155                 160
   aaa gga att tcc act cca gtg gaa ggc agc caa tat cat gtg ttt gct    528
   Lys Gly Ile Ser Thr Pro Val Glu Gly Ser Gln Tyr His Val Phe Ala
                   165                 170                 175
   gtg ggc ggg gaa ccg ctt gac ctc cag gga ctt gtg aca gat gcc aga    576
   Val Gly Gly Glu Pro Leu Asp Leu Gln Gly Leu Val Thr Asp Ala Arg
               180                 185                 190
   aca aaa tac aag gaa gaa ggg gta gta aca atc aaa aca atc aca aag    624
   Thr Lys Tyr Lys Glu Glu Gly Val Val Thr Ile Lys Thr Ile Thr Lys
           195                 200                 205
   aag gac atg gtc aac aaa gac caa gtc ctg aat cca att agc aag gcc    672
   Lys Asp Met Val Asn Lys Asp Gln Val Leu Asn Pro Ile Ser Lys Ala
       210                 215                 220
   aag ctg gat aag gac gga atg tat cca gtt gaa atc tgg cat cca gat    720
   Lys Leu Asp Lys Asp Gly Met Tyr Pro Val Glu Ile Trp His Pro Asp
   225                 230                 235                 240
   cca gca aaa aat gag aac aca agg tac ttt ggc aat tac act gga ggc    768
   Pro Ala Lys Asn Glu Asn Thr Arg Tyr Phe Gly Asn Tyr Thr Gly Gly
                   245                 250                 255
   acg tgc acc cca ccc gtc ctg cag ttc aca aac acc ctg aca act gtg    816
   Thr Cys Thr Pro Pro Val Leu Gln Phe Thr Asn Thr Leu Thr Thr Val
               260                 265                 270
   ctc cta gat gaa aat gga gtt ggg ccc ctc agc aaa gga gaa ggt cta     864
   Leu Leu Asp Glu Asn Gly Val Gly Pro Leu Ser Lys Gly Glu Gly Leu
           275                 280                 285
   tac ctc tcg agc gta gat ata atg ggc tgg aga gtt aca aga aac tat     912
   Tyr Leu Ser Ser Val Asp Ile Met Gly Trp Arg Val Thr Arg Asn Tyr
       290                 295                 300
   gat gtc cat cac tgg aga ggg ctt ccc aga tat ttc aaa atc acc ctg     960
   Asp Val His His Trp Arg Gly Leu Pro Arg Tyr Phe Lys Ile Thr Leu
   305                 310                 315                 320
   aga aaa aga tgg gtc aaa aat ccc tat ccc atg gcc tcc ctc ata agt     1008
   Arg Lys Arg Trp Val Lys Asn Pro Tyr Pro Met Ala Ser Leu Ile Ser
                   325                 330                 335
   tcc ctt ttc aac aac atg ctc ccc caa gtg cag ggc caa ccc atg gaa     1056
   Ser Leu Phe Asn Asn Met Leu Pro Gln Val Gln Gly Gln Pro Met Glu
               340                 345                 350
   ggg gag aac acc cag gta gag gag gtt aga gtg tat gat ggg act gaa     1104
   Gly Glu Asn Thr Gln Val Glu Glu Val Arg Val Tyr Asp Gly Thr Glu
           355                 360                 365
   cct gta ccg ggg gac cct gat atg acg cgc tat gtt gac cgc ttt gga     1152
   Pro Val Pro Gly Asp Pro Asp Met Thr Arg Tyr Val Asp Arg Phe Gly
       370                 375                 380
   aaa aca aag act gta ttt cct ccc ggg                                 1179
   Lys Thr Lys Thr Val Phe Pro Pro Gly
   385                 390
<210> 8
   <211> 393
   <212> PRT
   <213> Künstliche Sequenz
   <223> Beschreibung der künstlichen Sequenz: PyVP1-3C-[N-14]-PLP, prolinreiche Sequenz am verkürzten N-Terminus
<400> 8
   Met Pro Pro Pro Pro Pro Pro Pro Pro Leu Pro Gly Leu Gly Arg Arg
    1               5                  10                  15
   Gly Leu Ala Thr Ser His Gly Leu Ser Thr Lys Ala Cys Pro Arg Pro
   20 25 30
   Ala Pro Val Pro Lys Leu Leu Ile Lys Gly Gly Met Glu Val Leu Asp
   35 40 45
   Leu Val Thr Gly Pro Asp Cys Val Thr Glu Ile Glu Ala Phe Leu Asn
   50 55 60
   Pro Arg Met Gly Gln Pro Pro Thr Pro Glu Ser Leu Thr Glu Gly Gly
   65 70 75 80
   Gln Tyr Tyr Gly Trp Ser Arg Gly Ile Asn Leu Ala Thr Ser Asp Thr
   85 90 95
   Glu Asp Ser Pro Gly Asn Asn Thr Leu Pro Thr Trp Ser Met Ala Lys
   100 105 110
   Leu Gln Leu Pro Met Leu Asn Glu Asp Leu Thr Cys Asp Thr Leu Gln
   115 120 125
   Met Trp Glu Ala Val Ser Val Lys Thr Glu Val Val Gly Ser Gly Ser
   130 135 140
   Leu Leu Asp Val His Gly Phe Asn Lys Pro Thr Asp Thr Val Asn Thr
   145 150 155 160
   Lys Gly Ile Ser Thr Pro Val Glu Gly Ser Gln Tyr His Val Phe Ala
   165 170 175
   Val Gly Gly Glu Pro Leu Asp Leu Gln Gly Leu Val Thr Asp Ala Arg
   180 185 190
   Thr Lys Tyr Lys Glu Glu Gly Val Val Thr Ile Lys Thr Ile Thr Lys
   195 200 205
   Lys Asp Met Val Asn Lys Asp Gln Val Leu Asn Pro Ile Ser Lys Ala
   210 215 220
   Lys Leu Asp Lys Asp Gly Met Tyr Pro Val Glu Ile Trp His Pro Asp
   225 230 235 240
   Pro Ala Lys Asn Glu Asn Thr Arg Tyr Phe Gly Asn Tyr Thr Gly Gly
   245 250 255
   Thr Cys Thr Pro Pro Val Leu Gln Phe Thr Asn Thr Leu Thr Thr Val
   260 265 270
   Leu Leu Asp Glu Asn Gly Val Gly Pro Leu Ser Lys Gly Glu Gly Leu
           275                 280                 285
   Tyr Leu Ser Ser Val Asp Ile Met Gly Trp Arg Val Thr Arg Asn Tyr
       290                 295                 300
   Asp Val His His Trp Arg Gly Leu Pro Arg Tyr Phe Lys Ile Thr Leu
   305                 310                 315                 320
   Arg Lys Arg Trp Val Lys Asn Pro Tyr Pro Met Ala Ser Leu Ile Ser
                   325                 330                 335
   Ser Leu Phe Asn Asn Met Leu Pro Gln Val Gln Gly Gln Pro Met Glu
               340                 345                 350
   Gly Glu Asn Thr Gln Val Glu Glu Val Arg Val Tyr Asp Gly Thr Glu
           355                 360                 365
   Pro Val Pro Gly Asp Pro Asp Met Thr Arg Tyr Val Asp Arg Phe Gly
       370                 375                 380
   Lys Thr Lys Thr Val Phe Pro Pro Gly
   385                 390
<210> 9
   <211> 1209
   <212> DNA
   <213> Künstliche Sequenz
<220>
   <223> Beschreibung der künstlichen Sequenz: PyVP1-3C-WW[N-14] (verkuerzter N-Terminus, WW-Domaene am N-Terminus)
<220>
   <221> CDS
   <222> (1)..(1209)
<220>
   <221> misc feature
   <222> (9)..(93)
   <223> WW-Domaene
<400> 9
   atg agc ggc tgg aca gaa cat aaa tca cct gat gga agg act tat tat    48
   Met Ser Gly Trp Thr Glu His Lys Ser Pro Asp Gly Arg Thr Tyr Tyr
                   1               5                  10                  15
   tac aat act gaa aca aaa cag tct acc tgg gaa aag cca gat gat gga     96
   Tyr Asn Thr Glu Thr Lys Gln Ser Thr Trp Glu Lys Pro Asp Asp Gly
               20                  25                  30
   cat atg agc acc aag gct tgt cca aga ccc gca ccc gtt ccc aaa ctg    144
   His Met Ser Thr Lys Ala Cys Pro Arg Pro Ala Pro Val Pro Lys Leu
           35                  40                  45
   ctt att aaa ggg ggt atg gag gtg ctg gac ctt gtg aca ggg cca gac    192
   Leu Ile Lys Gly Gly Met Glu Val Leu Asp Leu Val Thr Gly Pro Asp
       50                  55                  60
   agt gtg aca gaa ata gaa gct ttt ctg aac ccc aga atg ggg cag cca    240
   Ser Val Thr Glu Ile Glu Ala Phe Leu Asn Pro Arg Met Gly Gln Pro
    65                  70                  75                  80
   ccc acc cct gaa agc cta aca gag gga ggg caa tac tat ggt tgg agc    288
   Pro Thr Pro Glu Ser Leu Thr Glu Gly Gly Gln Tyr Tyr Gly Trp Ser
                   85                  90                  95
   aga ggg att aat ttg gct aca tca gat aca gag gat tcc cca gga aat    336
   Arg Gly Ile Asn Leu Ala Thr Ser Asp Thr Glu Asp Ser Pro Gly Asn
               100                 105                 110
   aat aca ctt ccc aca tgg agt atg gca aag ctc cag ctt ccc atg ctc    384
   Asn Thr Leu Pro Thr Trp Ser Met Ala Lys Leu Gln Leu Pro Met Leu
           115                 120                 125
   aat gag gac ctc acg tgt gac acc cta caa atg tgg gag gca gtc tca    432
   Asn Glu Asp Leu Thr Cys Asp Thr Leu Gln Met Trp Glu Ala Val Ser
       130                 135                 140
   gtg aaa acc gag gtg gtg ggc tct ggc tca ctg tta gat gtg cat ggg    480
   Val Lys Thr Glu Val Val Gly Ser Gly Ser Leu Leu Asp Val His Gly
   145                 150                 155                 160
   ttc aac aaa ccc aca gat aca gta aac aca aaa gga att tcc act cca    528
   Phe Asn Lys Pro Thr Asp Thr Val Asn Thr Lys Gly Ile Ser Thr Pro
                   165                 170                 175
   gtg gaa ggc agc caa tat cat gtg ttt gct gtg ggc ggg gaa ccg ctt    576
   Val Glu Gly Ser Gln Tyr His Val Phe Ala Val Gly Gly Glu Pro Leu
               180                 185                 190
         gac ctc cag gga ctt gtg aca gat gcc aga aca aaa tac aag gaa gaa    624
         Asp Leu Gln Gly Leu Val Thr Asp Ala Arg Thr Lys Tyr Lys Glu Glu
   195                  200                  205
   ggg gta gta aca atc aaa aca atc aca aag aag gac atg gtc aac aaa    672
   Gly Val Val Thr Ile Lys Thr Ile Thr Lys Lys Asp Met Val Asn Lys
       210                 215                 220
   gac caa gtc ctg aat cca att agc aag gcc aag ctg gat aag gac gga    720
   Asp Gln Val Leu Asn Pro Ile Ser Lys Ala Lys Leu Asp Lys Asp Gly
   225                 230                 235                 240
   atg tat cca gtt gaa atc tgg cat cca gat cca gca aaa aat gag aac    768
   Met Tyr Pro Val Glu Ile Trp His Pro Asp Pro Ala Lys Asn Glu Asn
                   245                 250                 255
   aca agg tac ttt ggc aat tac act gga ggc acg tgc acc cca ccc gtc    816
   Thr Arg Tyr Phe Gly Asn Tyr Thr Gly Gly Thr Cys Thr Pro Pro Val
               260                 265                 270
   ctg cag ttc aca aac acc ctg aca act gtg ctc cta gat gaa aat gga    864
   Leu Gln Phe Thr Asn Thr Leu Thr Thr Val Leu Leu Asp Glu Asn Gly
           275                 280                 285
   gtt ggg ccc ctc agc aaa gga gaa ggt cta tac ctc tcg agc gta gat    912
   Val Gly Pro Leu Ser Lys Gly Glu Gly Leu Tyr Leu Ser Ser Val Asp
       290                 295                 300
   ata atg ggc tgg aga gtt aca aga aac tat gat gtc cat cac tgg aga    960
   Ile Met Gly Trp Arg Val Thr Arg Asn Tyr Asp Val His His Trp Arg
   305                 310                 315                 320
   ggg ctt ccc aga tat ttc aaa atc acc ctg aga aaa aga tgg gtc aaa    1008
   Gly Leu Pro Arg Tyr Phe Lys Ile Thr Leu Arg Lys Arg Trp Val Lys
                   325                 330                 335
   aat ccc tat ccc atg gcc tcc ctc ata agt tcc ctt ttc aac aac atg    1056
   Asn Pro Tyr Pro Met Ala Ser Leu Ile Ser Ser Leu Phe Asn Asn Met
               340                 345                 350
   ctc ccc caa gtg cag ggc caa ccc atg gaa ggg gag aac acc cag gta    1104
   Leu Pro Gln Val Gln Gly Gln Pro Met Glu Gly Glu Asn Thr Gln Val
           355                 360                 365
   gag gag gtt aga gtg tat gat ggg act gaa cct gta ccg ggg gac cct    1152
   Glu Glu Val Arg Val Tyr Asp Gly Thr Glu Pro Val Pro Gly Asp Pro
       370                 375                 380
   gat atg acg cgc tat gtt gac cgc ttt gga aaa aca aag act gta ttt    1200
   Asp Met Thr Arg Tyr Val Asp Arg Phe Gly Lys Thr Lys Thr Val Phe
   385 390 395 400
   cct ccc ggg 1209
   Pro Pro Gly
<210> 10
   <211> 403
   <212> PRT
   <213> Künstliche Sequenz
   <223> Beschreibung der künstlichen Sequenz: PyVPl-3C-WW[N-14] (verkuerzter N-Terminus, WW-Domaene am N-Terminus)
<400> 10
   Met Ser Gly Trp Thr Glu His Lys Ser Pro Asp Gly Arg Thr Tyr Tyr
   1 5 10 15
   Tyr Asn Thr Glu Thr Lys Gln Ser Thr Trp Glu Lys Pro Asp Asp Gly
   20 25 30
   His Met Ser Thr Lys Ala Cys Pro Arg Pro Ala Pro Val Pro Lys Leu
   35 40 45
   Leu Ile Lys Gly Gly Met Glu Val Leu Asp Leu Val Thr Gly Pro Asp
   50 55 60
   Ser Val Thr Glu Ile Glu Ala Phe Leu Asn Pro Arg Met Gly Gln Pro
   65 70 75 80
   Pro Thr Pro Glu Ser Leu Thr Glu Gly Gly Gln Tyr Tyr Gly Trp Ser
   85 90 95
   Arg Gly Ile Asn Leu Ala Thr Ser Asp Thr Glu Asp Ser Pro Gly Asn
   100 105 110
   Asn Thr Leu Pro Thr Trp Ser Met Ala Lys Leu Gln Leu Pro Met Leu
   115 120 125
   Asn Glu Asp Leu Thr Cys Asp Thr Leu Gln Met Trp Glu Ala Val Ser
   130 135 140
   Val Lys Thr Glu Val Val Gly Ser Gly Ser Leu Leu Asp Val His Gly
   145 150 155 160
   Phe Asn Lys Pro Thr Asp Thr Val Asn Thr Lys Gly Ile Ser Thr Pro
   165 170 175
   Val Glu Gly Ser Gln Tyr His Val Phe Ala Val Gly Gly Glu Pro Leu
   180 185 190
   Asp Leu Gln Gly Leu Val Thr Asp Ala Arg Thr Lys Tyr Lys Glu Glu
   195 200 205
   Gly Val Val Thr Ile Lys Thr Ile Thr Lys Lys Asp Met Val Asn Lys
   210 215 220
   Asp Gln Val Leu Asn Pro Ile Ser Lys Ala Lys Leu Asp Lys Asp Gly
   225 230 235 240
   Met Tyr Pro Val Glu Ile Trp His Pro Asp Pro Ala Lys Asn Glu Asn
   245 250 255
   Thr Arg Tyr Phe Gly Asn Tyr Thr Gly Gly Thr Cys Thr Pro Pro Val
   260 265 270
   Leu Gln Phe Thr Asn Thr Leu Thr Thr Val Leu Leu Asp Glu Asn Gly
   275 280 285
   Val Gly Pro Leu Ser Lys Gly Glu Gly Leu Tyr Leu Ser Ser Val Asp
   290 295 300
   Ile Met Gly Trp Arg Val Thr Arg Asn Tyr Asp Val His His Trp Arg
   305 310 315 320
   Gly Leu Pro Arg Tyr Phe Lys Ile Thr Leu Arg Lys Arg Trp Val Lys
   325 330 335
   Asn Pro Tyr Pro Met Ala Ser Leu Ile Ser Ser Leu Phe Asn Asn Met
   340 345 350
   Leu Pro Gln Val Gln Gly Gln Pro Met Glu Gly Glu Asn Thr Gln Val
   355 360 365
   Glu Glu Val Arg Val Tyr Asp Gly Thr Glu Pro Val Pro Gly Asp Pro
   370 375 380
   Asp Met Thr Arg Tyr Val Asp Arg Phe Gly Lys Thr Lys Thr Val Phe
   385 390 395 400
   Pro Pro Gly
<210> 11
   <211> 765
   <212> DNA
   <213> Künstliche Sequenz
<220>
   <223> Beschreibung der künstlichen Sequenz: GFP-PLP, prolinreiche Sequenz am C-Terminus
<220>
   <221> CDS
   <222> (1)..(765)
<220>
   <221> misc_feature
   <222> (736)..(765)
   <223> prolinreiche Sequenz
<400> 11
   atg gtg agc aag ggc gag gag ctg ttc acc ggg gtg gtg ccc atc ctg    48
   Met Val Ser Lys Gly Glu Glu Leu Phe Thr Gly Val Val Pro Ile Leu
    1               5                  10                  15
   gtc gag ctg gac ggc gac gta aac ggc cac aag ttc agc gtg tcc ggc    96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc   144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc   192
   Cys Thr Thr Gly Lys Leu Pro Val Pro Trp Pro Thr Leu Val Thr Thr
       50                  55                  60
   ctg acc tac ggc gtg cag tgc ttc agc cgc tac ccc gac cac atg aag   240
   Leu Thr Tyr Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag   288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag   336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc   384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag ggc ggc agc     528
   Gly Ile Lys Val Asn Phe Lys Ile Arg His Asn Ile Glu Gly Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg     624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac tta agc     720
   Val Thr Ala Ala Gly Ile Thr Leu Gly Met Asp Glu Leu Tyr Leu Ser
   225                 230                 235                 240
   cga cgt gcc tca ggt ccg ccg cct cca ccg cca ccg cct tta ccc         765
   Arg Arg Ala Ser Gly Pro Pro Pro Pro Pro Pro Pro Pro Leu Pro
                   245                 250                 255
<210> 12
   <211> 255
   <212> PRT
   <213> Künstliche Sequenz
   <223> Beschreibung der künstlichen Sequenz: GFP-PLP, prolinreiche Sequenz am C-Terminus
<400> 12
   Met Val Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Leu Thr Tyr Gly Val Gln Cys Phe Ser Arg Tyr Pro Asp His Met Lys
   65 70 75 80
   Gln His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Ile Arg His Asn Ile Glu Gly Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr Leu Gly Met Asp Glu Leu Tyr Leu Ser
   225 230 235 240
   Arg Arg Ala Ser Gly Pro Pro Pro Pro Pro Pro Pro Pro Leu Pro
   245 250 255
<210> 13
   <211> 837
   <212> DNA
   <213> Künstliche Sequenz
<220>
   <223> Beschreibung der künstlichen Sequenz: GFP-WW1, WW-Domaene am N-Terminus
<220>
   <221> CDS
   <222> (1)..(837)
<220>
   <221> misc_feature
   <222> (9)..(93)
   <223> WW-Domaene
<400> 13
   atg agc ggc tgg aca gaa cat aaa tca cct gat gga agg act tat tat     48
   Met Ser Gly Trp Thr Glu His Lys Ser Pro Asp Gly Arg Thr Tyr Tyr
     1               5                  10                  15
   tac aat act gaa aca aaa cag tct acc tgg gaa aag cca gat gat gga     96
   Tyr Asn Thr Glu Thr Lys Gln Ser Thr Trp Glu Lys Pro Asp Asp Gly
                20                  25                  30
   cat atg agc acc aag gct agc gtg agc aag ggc gag gag ctg ttc acc    144
   His Met Ser Thr Lys Ala Ser Val Ser Lys Gly Glu Glu Leu Phe Thr
            35                  40                  45
   ggg gtg gtg ccc atc ctg gtc gag ctg gac ggc gac gta aac ggc cac    192
   Gly Val Val Pro Ile Leu Val Glu Leu Asp Gly Asp Val Asn Gly His
        50                  55                  60
   aag ttc agc gtg tcc ggc gag ggc gag ggc gat gcc acc tac ggc aag    240
   Lys Phe Ser Val Ser Gly Glu Gly Glu Gly Asp Ala Thr Tyr Gly Lys
    65                  70                  75                  80
   ctg acc ctg aag ttc atc tgc acc acc ggc aag ctg ccc gtg ccc tgg    288
   Leu Thr Leu Lys Phe Ile Cys Thr Thr Gly Lys Leu Pro Val Pro Trp
                    85                  90                  95
   ccc acc ctc gtg acc acc ctg acc tac ggc gtg cag tgc ttc agc cgc    336
   Pro Thr Leu Val Thr Thr Leu Thr Tyr Gly Val Gln Cys Phe Ser Arg
               100                 105                 110
   tac ccc gac cac atg aag cag cac gac ttc ttc aag tcc gcc atg ccc    384
   Tyr Pro Asp His Met Lys Gln His Asp Phe Phe Lys Ser Ala Met Pro
           115                 120                 125
   gaa ggc tac gtc cag gag cgc acc atc ttc ttc aag gac gac ggc aac    432
   Glu Gly Tyr Val Gln Glu Arg Thr Ile Phe Phe Lys Asp Asp Gly Asn
       130                 135                 140
   tac aag acc cgc gcc gag gtg aag ttc gag ggc gac acc ctg gtg aac    480
   Tyr Lys Thr Arg Ala Glu Val Lys Phe Glu Gly Asp Thr Leu Val Asn
   145                 150                 155                 160
   cgc atc gag ctg aag ggc atc gac ttc aag gag gac ggc aac atc ctg    528
   Arg Ile Glu Leu Lys Gly Ile Asp Phe Lys Glu Asp Gly Asn Ile Leu
                   165                 170                 175
   ggg cac aag ctg gag tac aac tac aac agc cac aac gtc tat atc atg    576
   Gly His Lys Leu Glu Tyr Asn Tyr Asn Ser His Asn Val Tyr Ile Met
               180                 185                 190
   gcc gac aag cag aag aac ggc atc aag gtg aac ttc aag atc cgc cac    624
   Ala Asp Lys Gln Lys Asn Gly Ile Lys Val Asn Phe Lys Ile Arg His
           195                 200                 205
   aac atc gag gac ggc agc gtg cag ctc gcc gac cac tac cag cag aac    672
   Asn Ile Glu Asp Gly Ser Val Gln Leu Ala Asp His Tyr Gln Gln Asn
       210                 215                 220
   acc ccc atc ggc gac ggc ccc gtg ctg ctg ccc gac aac cac tac ctg    720
   Thr Pro Ile Gly Asp Gly Pro Val Leu Leu Pro Asp Asn His Tyr Leu
   225                 230                 235                 240
   agc acc cag tcc gcc ctg agc aaa gac ccc aac gag aag cgc gat cac    768
   Ser Thr Gln Ser Ala Leu Ser Lys Asp Pro Asn Glu Lys Arg Asp His
                   245                 250                 255
   atg gtc ctg ctg gag ttc gtg acc gcc gcc ggg atc act ctc ggc atg    816
   Met Val Leu Leu Glu Phe Val Thr Ala Ala Gly Ile Thr Leu Gly Met
               260                 265                 270
   gac gag ctg tac tta att ccc                                        837
   Asp Glu Leu Tyr Leu Ile Pro
           275
<210> 14
   <211> 279
   <212> PRT
   <213> Künstliche Sequenz
   <223> Beschreibung der künstlichen Sequenz: GFP-WW1, WW-Domaene am N-Terminus
<400> 14
   Met Ser Gly Trp Thr Glu His Lys Ser Pro Asp Gly Arg Thr Tyr Tyr
   1 5 10 15
   Tyr Asn Thr Glu Thr Lys Gln Ser Thr Trp Glu Lys Pro Asp Asp Gly
   20 25 30
   His Met Ser Thr Lys Ala Ser Val Ser Lys Gly Glu Glu Leu Phe Thr
   35 40 45
   Gly Val Val Pro Ile Leu Val Glu Leu Asp Gly Asp Val Asn Gly His
   50 55 60
   Lys Phe Ser Val Ser Gly Glu Gly Glu Gly Asp Ala Thr Tyr Gly Lys
   65 70 75 80
   Leu Thr Leu Lys Phe Ile Cys Thr Thr Gly Lys Leu Pro Val Pro Trp
   85 90 95
   Pro Thr Leu Val Thr Thr Leu Thr Tyr Gly Val Gln Cys Phe Ser Arg
   100 105 110
   Tyr Pro Asp His Met Lys Gln His Asp Phe Phe Lys Ser Ala Met Pro
   115 120 125
   Glu Gly Tyr Val Gln Glu Arg Thr Ile Phe Phe Lys Asp Asp Gly Asn
   130 135 140
   Tyr Lys Thr Arg Ala Glu Val Lys Phe Glu Gly Asp Thr Leu Val Asn
   145 150 155 160
   Arg Ile Glu Leu Lys Gly Ile Asp Phe Lys Glu Asp Gly Asn Ile Leu
   165 170 175
   Gly His Lys Leu Glu Tyr Asn Tyr Asn Ser His Asn Val Tyr Ile Met
   180 185 190
   Ala Asp Lys Gln Lys Asn Gly Ile Lys Val Asn Phe Lys Ile Arg His
   195 200 205
   Asn Ile Glu Asp Gly Ser Val Gln Leu Ala Asp His Tyr Gln Gln Asn
   210 215 220
   Thr Pro Ile Gly Asp Gly Pro Val Leu Leu Pro Asp Asn His Tyr Leu
   225 230 235 240
   Ser Thr Gln Ser Ala Leu Ser Lys Asp Pro Asn Glu Lys Arg Asp His
   245 250 255
   Met Val Leu Leu Glu Phe Val Thr Ala Ala Gly Ile Thr Leu Gly Met
   260 265 270
   Asp Glu Leu Tyr Leu Ile Pro
   275
<210> 15
   <211> 13
   <212> PRT
   <213> Künstliche Sequenz
<220>
   <223> Beschreibung der künstlichen Sequenz: prolinreiche Sequenz (PLP)
<400> 15
   Cys Ser Gly Pro Pro Pro Pro Pro Pro Pro Pro Leu Pro
   1 5 10
<210> 16
   <211> 1152
   <212> DNA
   <213> Künstliche Sequenz
<220>
   <223> Beschreibung der künstlichen Sequenz: PyVP1-CallS-T249C-Variante des Polyomavirus VP1-Proteins, mit allen Cysteinen gegen Serin ausgetauscht und Austausch von Thr 249 gegen Cys
<220>
   <221> CDS
   <222> ()..(1152)
<400> 16
   atg gcc ccc aaa aga aaa agc ggc gtc tct aaa agc gag aca aaa agc 48
   Met Ala Pro Lys Arg Lys Ser Gly Val Ser Lys Ser Glu Thr Lys Ser
   1 5 10 15
   aca aag gct agc cca aga ccc gca ccc gtt ccc aaa ctg ctt att aaa 96
   Thr Lys Ala Ser Pro Arg Pro Ala Pro Val Pro Lys Leu Leu Ile Lys
   20 25 30
   ggg ggt atg gag gtg ctg gac ctt gtg aca ggg cca gac agt gtg aca      144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct      192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att      240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt      288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac      336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc      384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa      432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc      480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag      528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta      576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc      624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca      672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc    768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                 375                 380
<210> 17
   <211> 384
   <212> PRT
   <213> Künstliche Sequenz
   <223> Beschreibung der künstlichen Sequenz: PyVP1-CallS-T249C-Variante des Polyomavirus VP1-Proteins, mit allen Cysteinen gegen Serin ausgetauscht und Austausch von Thr 249 gegen Cys
<400> 17
   Met Ala Pro Lys Arg Lys Ser Gly Val Ser Lys Ser Glu Thr Lys Ser
    1               5                  10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Ser Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145                 150                 155                 160
   Ser Gln Tyr His Val Phe Ala Val Gly Gly Glu Pro Leu Asp Leu Gln
                   165                 170                 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                 240
   Phe Gly Asn Tyr Thr Gly Gly Thr Cys Thr Pro Pro Val Leu Gln Phe
   245 250 255
   Thr Asn Thr Leu Thr Thr Val Leu Leu Asp Glu Asn Gly Val Gly Pro
   260 265 270
   Leu Ser Lys Gly Glu Gly Leu Tyr Leu Ser Ser Val Asp Ile Met Gly
   275 280 285
   Trp Arg Val Thr Arg Asn Tyr Asp Val His His Trp Arg Gly Leu Pro
   290 295 300
   Arg Tyr Phe Lys Ile Thr Leu Arg Lys Arg Trp Val Lys Asn Pro Tyr
   305 310 315 320
   Pro Met Ala Ser Leu Ile Ser Ser Leu Phe Asn Asn Met Leu Pro Gln
   325 330 335
   Val Gln Gly Gln Pro Met Glu Gly Glu Asn Thr Gln Val Glu Glu Val
   340 345 350
   Arg Val Tyr Asp Gly Thr Glu Pro Val Pro Gly Asp Pro Asp Met Thr
   355 360 365
   Arg Tyr Val Asp Arg Phe Gly Lys Thr Lys Thr Val Phe Pro Pro Gly
   370 375 380
<210> 18
   <211> 1110
   <212> DNA
   <213> Künstliche Sequenz
<220>
   <223> Beschreibung der künstlichen Sequenz:VP1-3C-[N-14]-Variante des Polyomavirus VP1-Proteins, am N-Terminus um 14 AS verkürzt, mit 4 der 6 Cysteine gegen Serin ausgetauscht und dem Austausch Thr 249 Cys
<220>
   <221> CDS
   <222> ()..(1110)
<400> 18
   atg agc acc aag gct tgt cca aga ccc gca ccc gtt ccc aaa ctg ctt 48
   Met Ser Thr Lys Ala Cys Pro Arg Pro Ala Pro Val Pro Lys Leu Leu
   1                   5                        10                        15
   att aaa ggg ggt atg gag gtg ctg gac ctt gtg aca ggg cca gac agt    96
   Ile Lys Gly Gly Met Glu Val Leu Asp Leu Val Thr Gly Pro Asp Ser
                   20                         25                        30
   gtg aca gaa ata gaa gct ttt ctg aac ccc aga atg ggg cag cca ccc   144
   Val Thr Glu Ile Glu Ala Phe Leu Asn Pro Arg Met Gly Gln Pro Pro
              35                         40                        45
   acc cct gaa agc cta aca gag gga ggg caa tac tat ggt tgg agc aga   192
   Thr Pro Glu Ser Leu Thr Glu Gly Gly Gln Tyr Tyr Gly Trp Ser Arg
         50                         55                        60
   ggg att aat ttg gct aca tca gat aca gag gat tcc cca gga aat aat   240
   Gly Ile Asn Leu Ala Thr Ser Asp Thr Glu Asp Ser Pro Gly Asn Asn
    65                       70                         75                         80
   aca ctt ccc aca tgg agt atg gca aag ctc cag ctt ccc atg ctc aat   288
   Thr Leu Pro Thr Trp Ser Met Ala Lys Leu Gln Leu Pro Met Leu Asn
                        85                         90                         95
   gag gac ctc acg tgt gac acc cta caa atg tgg gag gca gtc tca gtg   336
   Glu Asp Leu Thr Cys Asp Thr Leu Gln Met Trp Glu Ala Val Ser Val
                   100                        105                       110
   aaa acc gag gtg gtg ggc tct ggc tca ctg tta gat gtg cat ggg ttc   384
   Lys Thr Glu Val Val Gly Ser Gly Ser Leu Leu Asp Val His Gly Phe
              115                        120                       125
   aac aaa ccc aca gat aca gta aac aca aaa gga att tcc act cca gtg   432
   Asn Lys Pro Thr Asp Thr Val Asn Thr Lys Gly Ile Ser Thr Pro Val
         130                        135                       140
   gaa ggc agc caa tat cat gtg ttt gct gtg ggc ggg gaa ccg ctt gac   480
   Glu Gly Ser Gln Tyr His Val Phe Ala Val Gly Gly Glu Pro Leu Asp
   145                       150                        155                       160
   ctc cag gga ctt gtg aca gat gcc aga aca aaa tac aag gaa gaa ggg   528
   Leu Gln Gly Leu Val Thr Asp Ala Arg Thr Lys Tyr Lys Glu Glu Gly
                        165                        170                       175
   gta gta aca atc aaa aca atc aca aag aag gac atg gtc aac aaa gac   576
   Val Val Thr Ile Lys Thr Ile Thr Lys Lys Asp Met Val Asn Lys Asp
                   180                        185                       190
   caa gtc ctg aat cca att agc aag gcc aag ctg gat aag gac gga atg   624
   Gln Val Leu Asn Pro Ile Ser Lys Ala Lys Leu Asp Lys Asp Gly Met
   195 200 205
   tat cca gtt gaa atc tgg cat cca gat cca gca aaa aat gag aac aca 672
   Tyr Pro Val Glu Ile Trp His Pro Asp Pro Ala Lys Asn Glu Asn Thr
   210 215 220
   agg tac ttt ggc aat tac act gga ggc acg tgc acc cca ccc gtc ctg 720
   Arg Tyr Phe Gly Asn Tyr Thr Gly Gly Thr Cys Thr Pro Pro Val Leu
   225 230 235 240
   cag ttc aca aac acc ctg aca act gtg ctc cta gat gaa aat gga gtt 768
   Gln Phe Thr Asn Thr Leu Thr Thr Val Leu Leu Asp Glu Asn Gly Val
   245 250 255
   ggg ccc ctc agc aaa gga gaa ggt cta tac ctc tcg agc gta gat ata 816
   Gly Pro Leu Ser Lys Gly Glu Gly Leu Tyr Leu Ser Ser Val Asp Ile
   260 265 270
   atg ggc tgg aga gtt aca aga aac tat gat gtc cat cac tgg aga ggg 864
   Met Gly Trp Arg Val Thr Arg Asn Tyr Asp Val His His Trp Arg Gly
   275 280 285
   ctt ccc aga tat ttc aaa atc acc ctg aga aaa aga tgg gtc aaa aat 912
   Leu Pro Arg Tyr Phe Lys Ile Thr Leu Arg Lys Arg Trp Val Lys Asn
   290 295 300
   ccc tat ccc atg gcc tcc ctc ata agt tcc ctt ttc aac aac atg ctc 960
   Pro Tyr Pro Met Ala Ser Leu Ile Ser Ser Leu Phe Asn Asn Met Leu
   305 310 315 320
   ccc caa gtg cag ggc caa ccc atg gaa ggg gag aac acc cag gta gag 1008
   Pro Gln Val Gln Gly Gln Pro Met Glu Gly Glu Asn Thr Gln Val Glu
   325 330 335
   gag gtt aga gtg tat gat ggg act gaa cct gta ccg ggg gac cct gat 1056
   Glu Val Arg Val Tyr Asp Gly Thr Glu Pro Val Pro Gly Asp Pro Asp
   340 345 350
   atg acg cgc tat gtt gac cgc ttt gga aaa aca aag act gta ttt cct 1104
   Met Thr Arg Tyr Val Asp Arg Phe Gly Lys Thr Lys Thr Val Phe Pro
   355 360 365
   ccc ggg 1110
   Pro Gly
   370
<210> 19
   <211> 370
   <212> PRT
   <213> Künstliche Sequenz
   <223> Beschreibung der künstlichen Sequenz:VP1-3C-[N-14]-Variante des Polyomavirus VP1-Proteins, am N-Terminus um 14 AS verkürzt, mit 4 der 6 Cysteine gegen Serin ausgetauscht und dem Austausch Thr 249 Cys
<400> 19
   Met Ser Thr Lys Ala Cys Pro Arg Pro Ala Pro Val Pro Lys Leu Leu
   1 5 10 15
   Ile Lys Gly Gly Met Glu Val Leu Asp Leu Val Thr Gly Pro Asp Ser
   20 25 30
   Val Thr Glu Ile Glu Ala Phe Leu Asn Pro Arg Met Gly Gln Pro Pro
   35 40 45
   Thr Pro Glu Ser Leu Thr Glu Gly Gly Gln Tyr Tyr Gly Trp Ser Arg
   50 55 60
   Gly Ile Asn Leu Ala Thr Ser Asp Thr Glu Asp Ser Pro Gly Asn Asn
   65 70 75 80
   Thr Leu Pro Thr Trp Ser Met Ala Lys Leu Gln Leu Pro Met Leu Asn
   85 90 95
   Glu Asp Leu Thr Cys Asp Thr Leu Gln Met Trp Glu Ala Val Ser Val
   100 105 110
   Lys Thr Glu Val Val Gly Ser Gly Ser Leu Leu Asp Val His Gly Phe
   115 120 125
   Asn Lys Pro Thr Asp Thr Val Asn Thr Lys Gly Ile Ser Thr Pro Val
   130 135 140
   Glu Gly Ser Gln Tyr His Val Phe Ala Val Gly Gly Glu Pro Leu Asp
   145 150 155 160
   Leu Gln Gly Leu Val Thr Asp Ala Arg Thr Lys Tyr Lys Glu Glu Gly
   165 170 175
   Val Val Thr Ile Lys Thr Ile Thr Lys Lys Asp Met Val Asn Lys Asp
   180 185 190
   Gln Val Leu Asn Pro Ile Ser Lys Ala Lys Leu Asp Lys Asp Gly Met
   195 200 205
   Tyr Pro Val Glu Ile Trp His Pro Asp Pro Ala Lys Asn Glu Asn Thr
       210                 215                 220
   Arg Tyr Phe Gly Asn Tyr Thr Gly Gly Thr Cys Thr Pro Pro Val Leu
   225                 230                 235                 240
   Gln Phe Thr Asn Thr Leu Thr Thr Val Leu Leu Asp Glu Asn Gly Val
                   245                 250                 255
   Gly Pro Leu Ser Lys Gly Glu Gly Leu Tyr Leu Ser Ser Val Asp Ile
               260                 265                 270
   Met Gly Trp Arg Val Thr Arg Asn Tyr Asp Val His His Trp Arg Gly
           275                 280                 285
   Leu Pro Arg Tyr Phe Lys Ile Thr Leu Arg Lys Arg Trp Val Lys Asn
       290                 295                 300
   Pro Tyr Pro Met Ala Ser Leu Ile Ser Ser Leu Phe Asn Asn Met Leu
   305                 310                 315                 320
   Pro Gln Val Gln Gly Gln Pro Met Glu Gly Glu Asn Thr Gln Val Glu
                   325                 330                 335
   Glu Val Arg Val Tyr Asp Gly Thr Glu Pro Val Pro Gly Asp Pro Asp
               340                 345                 350
   Met Thr Arg Tyr Val Asp Arg Phe Gly Lys Thr Lys Thr Val Phe Pro
           355                 360                 365
   Pro Gly
       370
<210> 20
   <211> 1152
   <212> DNA
   <213> Künstliche Sequenz
<220>
   <223> Beschreibung der künstlichen Sequenz:PyVP1-3C-Variante des Polyomavirus VP1, mit Austausch von 4 der 6 Cysteine gegen Serin und Austausch von Thr 249 gegen Cys
<220>
   <221> CDS
   <222> (1)..(1152)
<400> 20
   atg gcc ccc aaa aga aaa agc ggc gtc tct aaa agc gag aca aaa agc    48
   Met Ala Pro Lys Arg Lys Ser Gly Val Ser Lys Ser Glu Thr Lys Ser
     1               5                  10                  15
   aca aag gcc tgt cca aga ccc gca ccc gtt ccc aaa ctg ctt att aaa    96
   Thr Lys Ala Cys Pro Arg Pro Ala Pro Val Pro Lys Leu Leu Ile Lys
                20                  25                  30
   ggg ggt atg gag gtg ctg gac ctt gtg aca ggg cca gac agt gtg aca    144
   Gly Gly Met Glu Val Leu Asp Leu Val Thr Gly Pro Asp Ser Val Thr
            35                  40                  45
   gaa ata gaa gct ttt ctg aac ccc aga atg ggg cag cca ccc acc cct    192
   Glu Ile Glu Ala Phe Leu Asn Pro Arg Met Gly Gln Pro Pro Thr Pro
        50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
    65                  70                  75                  80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                    85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acc tgt gac acc cta caa atg tgg gag gca gtc tca gtg aaa acc    384
   Leu Thr Cys Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc    480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc    768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg 1152
   Arg Tyr Val Asp Arg Phe Gly Lys Thr Lys Thr Val Phe Pro Pro Gly
   370 375 380
<210> 21
   <211> 384
   <212> PRT
   <213> Künstliche Sequenz
   <223> Beschreibung der künstlichen Sequenz:PyVP1-3C-Variante des Polyomavirus VP1, mit Austausch von 4 der 6 Cysteine gegen Serin und Austausch von Thr 249 gegen Cys
<400> 21
   Met Ala Pro Lys Arg Lys Ser Gly Val Ser Lys Ser Glu Thr Lys Ser
   1 5 10 15
   Thr Lys Ala Cys Pro Arg Pro Ala Pro Val Pro Lys Leu Leu Ile Lys
   20 25 30
   Gly Gly Met Glu Val Leu Asp Leu Val Thr Gly Pro Asp Ser Val Thr
   35 40 45
   Glu Ile Glu Ala Phe Leu Asn Pro Arg Met Gly Gln Pro Pro Thr Pro
   50 55 60
   Glu Ser Leu Thr Glu Gly Gly Gln Tyr Tyr Gly Trp Ser Arg Gly Ile
   65 70 75 80
   Asn Leu Ala Thr Ser Asp Thr Glu Asp Ser Pro Gly Asn Asn Thr Leu
   85 90 95
   Pro Thr Trp Ser Met Ala Lys Leu Gln Leu Pro Met Leu Asn Glu Asp
   100 105 110
   Leu Thr Cys Asp Thr Leu Gln Met Trp Glu Ala Val Ser Val Lys Thr
   115 120 125
   Glu Val Val Gly Ser Gly Ser Leu Leu Asp Val His Gly Phe Asn Lys
   130 135 140
   Pro Thr Asp Thr Val Asn Thr Lys Gly Ile Ser Thr Pro Val Glu Gly
   145 150 155 160
   Ser Gln Tyr His Val Phe Ala Val Gly Gly Glu Pro Leu Asp Leu Gln
   165 170 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                     240
   Phe Gly Asn Tyr Thr Gly Gly Thr Cys Thr Pro Pro Val Leu Gln Phe
                   245                 250                 255
   Thr Asn Thr Leu Thr Thr Val Leu Leu Asp Glu Asn Gly Val Gly Pro
               260                 265                 270
   Leu Ser Lys Gly Glu Gly Leu Tyr Leu Ser Ser Val Asp Ile Met Gly
           275                 280                 285
   Trp Arg Val Thr Arg Asn Tyr Asp Val His His Trp Arg Gly Leu Pro
       290                 295                 300
   Arg Tyr Phe Lys Ile Thr Leu Arg Lys Arg Trp Val Lys Asn Pro Tyr
   305                 310                 315                     320
   Pro Met Ala Ser Leu Ile Ser Ser Leu Phe Asn Asn Met Leu Pro Gln
                   325                 330                 335
   Val Gln Gly Gln Pro Met Glu Gly Glu Asn Thr Gln Val Glu Glu Val
               340                 345                 350
   Arg Val Tyr Asp Gly Thr Glu Pro Val Pro Gly Asp Pro Asp Met Thr
           355                 360                 365
   Arg Tyr Val Asp Arg Phe Gly Lys Thr Lys Thr Val Phe Pro Pro Gly
       370                 375                 380